(19) Europäisches Patentamt
European Patent Office
Office européen des brevets

(11) EP 3 821 911 A1

(12) EUROPEAN PATENT APPLICATION
published in accordance with Art. 153(4) EPC

(43) Date of publication:
**19.05.2021 Bulletin 2021/20**

(21) Application number: **19833361.9**

(22) Date of filing: **12.07.2019**

(51) Int Cl.:
***A61K 47/54*** (2017.01) ***C12N 15/10*** (2006.01)
***C12N 15/11*** (2006.01) ***A61K 31/713*** (2006.01)

(86) International application number:
**PCT/CN2019/095766**

(87) International publication number:
**WO 2020/011248 (16.01.2020 Gazette 2020/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **12.07.2018 CN 201810766003**
**12.07.2018 CN 201810765976**

(71) Applicant: **Bai Yao Zhi Da (Beijing) Nanobio Technology Co., Ltd.**
**Beijing 100080 (CN)**

(72) Inventors:
- **WANG, Liyuan**
  **Beijing 100080 (CN)**
- **WANG, Meng**
  **Beijing 100080 (CN)**

(74) Representative: **Biggi, Cristina**
**Bugnion S.p.A.**
**Viale Lancetti 17**
**20158 Milano (IT)**

(54) **NUCLEIC ACID NANOPARTICLES, PHARMACEUTICAL COMPOSITION COMPRISING SAME, DRUG COMPRISING DOXORUBICIN AND PREPARATION METHOD THEREFOR**

(57) Disclosed are nucleic acid nanoparticles, a pharmaceutical composition comprising the same, a drug comprising doxorubicin and a preparation method thereof. The nucleic acid nanoparticles have a nucleic acid structural domain, the nucleic acid structural domain includes a sequence a, a sequence b and a sequence c; the sequence a includes a sequence a1 or a sequence obtained by insertion, deletion or substitution of at least one base in the sequence a1, the sequence b includes a sequence b1 or a sequence obtained by insertion, deletion or substitution of at least one base in the sequence b1, and the sequence c includes a sequence d or a sequence obtained by insertion, deletion or substitution of at least one base in the sequence d. The nucleic acid nanoparticles are capable of, by means of including the three sequences described above or variant sequences thereof, not only self-assembling to form the nucleic acid structural domain, but also serving as a carrier. While served as the carrier, the nucleic acid nanoparticles are not only capable of loading and delivering nucleic acid drugs, but are also suitable for loading and delivering other biologically active substances such as chemical drugs.

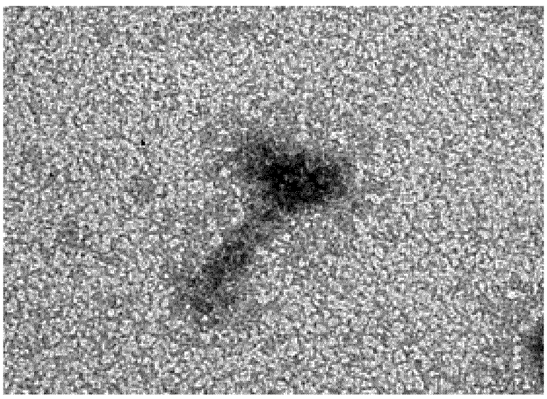

Fig. 9

EP 3 821 911 A1

SEQUENCE LISTING

<110> BAI YAO ZHI DA (Beijing) NanoBio Technology Co., Ltd

<120> Nucleic Acid Nanoparticles, Pharmaceutical Composition thereof, Medicine comprising Doxorubicin and Preparation Method thereof

<130> PN114615BYZD

<150> 201810766003.4
<151> 2018-07-12

<150> 201810765976.6
<151> 2018-07-12

<160> 171

<170> PatentIn version 3.5

<210> 1
<211> 10
<212> DNA
<213> Artificial Sequence

<220>
<223> Artificial Sequence

<220>
<221> misc_feature
<222> (1)..(10)
<223> a1 sequence

<400> 1
ccagcguucc 10

<210> 2
<211> 10
<212> DNA
<213> Artificial Sequence

<220>
<223> Artificial Sequence

<220>
<221> mRNA
<222> (1)..(10)
<223> a1 sequcence

<400> 2
ccagcgttcc 10

<210> 3
<211> 10
<212> DNA
<213> Artificial Sequence

<220>
<223> y is not exist,just for completing the sequence lising.

<220>
<221>  misc_feature
<222>  (1)..(9)
<223>  b1 sequcence

<400>  3
gguucgccgy                                                              10

<210>  4
<211>  10
<212>  DNA
<213>  Artificial Sequence

<220>
<223>  y is not exist,just for completing the sequence listing.

<220>
<221>  misc_feature
<222>  (1)..(9)
<223>  b1 sequcence

<400>  4
ggttcgccgy                                                              10

<210>  5
<211>  12
<212>  DNA
<213>  Artificial Sequence

<220>
<223>  Artificial Sequence

<220>
<221>  misc_feature
<222>  (1)..(12)
<223>  c1 sequcence

<400>  5
cggccauagc gg                                                           12

<210>  6
<211>  12
<212>  DNA
<213>  Artificial Sequence

<220>
<223>  Artificial Sequence

<220>
<221>  misc_feature
<222>  (1)..(12)
<223>  c1 sequcence

<400>  6
cggccatagc gg                                                           12

<210> 7
<211> 10
<212> DNA
<213> Artificial Sequence

<220>
<223> Artificial Sequence

<220>
<221> misc_feature
<222> (1)..(10)
<223> a sequcence

<400> 7
ggagcguugg 10

<210> 8
<211> 10
<212> DNA
<213> Artificial Sequence

<220>
<223> y is not exist,just for completing the sequence listing.

<220>
<221> misc_feature
<222> (1)..(9)
<223> b sequcence

<400> 8
ccuucgccgy 10

<210> 9
<211> 12
<212> DNA
<213> Artificial Sequence

<220>
<223> Artificial Sequence

<220>
<221> misc_feature
<222> (1)..(12)
<223> c sequcence

<400> 9
cggccauagc cc 12

<210> 10
<211> 10
<212> DNA
<213> Artificial Sequence

<220>
<223> Artificial Sequence

<220>
<221> misc_feature
<222> (1)..(10)
<223> a sequcence

<400> 10
gcagcguucg 10

<210> 11
<211> 10
<212> DNA
<213> Artificial Sequence

<220>
<223> y is not exist,just for completing the sequence listing.

<220>
<221> misc_feature
<222> (1)..(9)
<223> b sequcence

<400> 11
cguucgccgy 10

<210> 12
<211> 12
<212> DNA
<213> Artificial Sequence

<220>
<223> Artificial Sequence

<220>
<221> misc_feature
<222> (1)..(12)
<223> c sequcence

<400> 12
cggccauagc gc 12

<210> 13
<211> 10
<212> DNA
<213> Artificial Sequence

<220>
<223> Artificial Sequence

<220>
<221> misc_feature
<222> (1)..(10)
<223> a sequcence

<400> 13
cgagcguugc 10

<210> 14
<211> 10
<212> DNA
<213> Artificial Sequence

<220>
<223> y is not exist,just for completing the sequence listing.

<220>
<221> misc_feature
<222> (1)..(9)
<223> b sequcence

<400> 14
gcuucgccgy 10

<210> 15
<211> 12
<212> DNA
<213> Artificial Sequence

<220>
<223> Artificial Sequence

<220>
<221> misc_feature
<222> (1)..(12)
<223> c sequcence

<400> 15
cggccauagc cg 12

<210> 16
<211> 10
<212> DNA
<213> Artificial Sequence

<220>
<223> Artificial Sequence

<220>
<221> misc_feature
<222> (1)..(10)
<223> a sequcence

<400> 16
ggagcguugg 10

<210> 17
<211> 10
<212> DNA
<213> Artificial Sequence

<220>
<223> y is not exist,just for completing the sequence listing.

<220>
<221> misc_feature
<222> (1)..(9)
<223> b sequcence

<400> 17
ccuucggggy 10

<210> 18
<211> 12
<212> DNA
<213> Artificial Sequence

<220>
<223> Artificial Sequence

<220>
<221> misc_feature
<222> (1)..(12)
<223> c sequcence

<400> 18
cccccauagc cc 12

<210> 19
<211> 10
<212> DNA
<213> Artificial Sequence

<220>
<223> Artificial Sequence

<220>
<221> misc_feature
<222> (1)..(10)
<223> a sequcence

<400> 19
gcagcguucg 10

<210> 20
<211> 10
<212> DNA
<213> Artificial Sequence

<220>
<223> y is not exist,just for completing the sequence listing.

<220>
<221> misc_feature
<222> (1)..(9)
<223> b sequcence

<400> 20
cguucggcgy 10

<210> 21
<211> 12
<212> DNA
<213> Artificial Sequence

<220>
<223> Artificial Sequence

<220>
<221> misc_feature
<222> (1)..(12)
<223> c sequcence

<400> 21
cgcccauagc gc 12

<210> 22
<211> 10
<212> DNA
<213> Artificial Sequence

<220>
<223> Artificial Sequence

<220>
<221> misc_feature
<222> (1)..(10)
<223> a sequcence

<400> 22
gcagcguucg 10

<210> 23
<211> 10
<212> DNA
<213> Artificial Sequence

<220>
<223> y is not exist,just for completing the sequence listing.

<220>
<221> misc_feature
<222> (1)..(9)
<223> b sequcence

<400> 23
cguucggccy 10

<210> 24
<211> 12
<212> DNA
<213> Artificial Sequence

<220>
<223> Artificial Sequence

<220>
<221> misc_feature
<222> (1)..(12)
<223> c sequcence

<400> 24
ggcccauagc gc 12

<210> 25
<211> 10
<212> DNA
<213> Artificial Sequence

<220>
<223> Artificial Sequence

<220>
<221> misc_feature
<222> (1)..(10)
<223> a sequcence

<400> 25
cgagcguugc 10

<210> 26
<211> 10
<212> DNA
<213> Artificial Sequence

<220>
<223> y is not exist,just for completing the sequence listing.

<220>
<221> misc_feature
<222> (1)..(9)
<223> b sequcence

<400> 26
gcuucggcgy 10

<210> 27
<211> 12
<212> DNA
<213> Artificial Sequence

<220>
<223> Artificial Sequence

<220>
<221> misc_feature
<222> (1)..(12)
<223> c sequcence

<400> 27
cgcccauagc cg 12

<210> 28
<211> 10
<212> DNA
<213> Artificial Sequence

<220>
<223> Artificial Sequence

<220>
<221> misc_feature
<222> (1)..(10)
<223> a sequcence

<400> 28
ggagcgttgg 10

<210> 29
<211> 10
<212> DNA
<213> Artificial Sequence

<220>
<223> y is not exist,just for completing the sequence listing.

<220>
<221> misc_feature
<222> (1)..(9)
<223> b sequcence

<400> 29
ccttcgccgy 10

<210> 30
<211> 12
<212> DNA
<213> Artificial Sequence

<220>
<223> Artificial Sequence

<220>
<221> misc_feature
<222> (1)..(12)
<223> c sequcence

<400> 30
cggccatagc cc 12

<210> 31
<211> 10
<212> DNA
<213> Artificial Sequence

<220>
<223> Artificial Sequence

<220>
<221> misc_feature
<222> (1)..(10)
<223> a sequcence

<400> 31
gcagcgttcg 10

<210> 32
<211> 10
<212> DNA
<213> Artificial Sequence

<220>
<223> y is not exist,just for completing the sequence listing.

<220>
<221> misc_feature
<222> (1)..(9)
<223> b sequcence

<400> 32
cgttcgccgy 10

<210> 33
<211> 12
<212> DNA
<213> Artificial Sequence

<220>
<223> Artificial Sequence

<220>
<221> misc_feature
<222> (1)..(12)
<223> c sequcence

<400> 33
cggccatagc gc 12

<210> 34
<211> 10
<212> DNA
<213> Artificial Sequence

<220>
<223> Artificial Sequence

<220>
<221> misc_feature
<222> (1)..(10)
<223> a sequcence

<400> 34
cgagcgttgc 10

<210> 35
<211> 10
<212> DNA
<213> Artificial Sequence

<220>
<223> y is not exist,just for completing the sequence listing.

<220>
<221> misc_feature
<222> (1)..(9)
<223> b sequcence

<400> 35
gcttcgccgy 10

<210> 36
<211> 12
<212> DNA
<213> Artificial Sequence

<220>
<223> Artificial Sequence

<220>
<221> misc_feature
<222> (1)..(12)
<223> c sequcence

<400> 36
cggccatagc cg 12

<210> 37
<211> 10
<212> DNA
<213> Artificial Sequence

<220>
<223> Artificial Sequence

<220>
<221> misc_feature
<222> (1)..(10)
<223> a sequcence

<400> 37
ggagcgttgg 10

<210> 38
<211> 10
<212> DNA
<213> Artificial Sequence

<220>
<223> y is not exist,just for completing the sequence listing.

<220>
<221> misc_feature
<222> (1)..(9)
<223> b sequcence

<400> 38
ccttcggggy 10

<210> 39
<211> 12
<212> DNA
<213> Artificial Sequence

<220>
<223> Artificial Sequence

<220>
<221> misc_feature
<222> (1)..(12)
<223> c sequcence

<400> 39
cccccatagc cc 12

<210> 40
<211> 10
<212> DNA
<213> Artificial Sequence

<220>
<223> Artificial Sequence

<220>
<221> misc_feature
<222> (1)..(10)
<223> a sequcence

<400> 40
gcagcgttcg 10

<210> 41
<211> 10
<212> DNA
<213> Artificial Sequence

<220>
<223> y is not exist,just for completing the sequence listing.

<220>
<221> misc_feature
<222> (1)..(9)
<223> b sequcence

<400> 41
cgttcggcgy 10

<210> 42
<211> 12
<212> DNA
<213> Artificial Sequence

<220>
<223> Artificial Sequence

<220>
<221> misc_feature
<222> (1)..(12)
<223> c sequcence

<400> 42
cgcccatagc gc 12

<210> 43
<211> 10
<212> DNA
<213> Artificial Sequence

<220>
<223> Artificial Sequence

<220>
<221> misc_feature
<222> (1)..(10)
<223> a sequcence

<400> 43
gcagcgttcg 10

<210> 44
<211> 10
<212> DNA
<213> Artificial Sequence

<220>
<223> y is not exist,just for completing the sequence listing.

<220>
<221> misc_feature
<222> (1)..(9)
<223> b sequcence

<400> 44
cgttcggccy 10

<210> 45
<211> 12
<212> DNA
<213> Artificial Sequence

<220>
<223> Artificial Sequence

<220>
<221> misc_feature
<222> (1)..(12)
<223> c sequcence

<400> 45
ggcccatagc gc 12

<210> 46
<211> 10
<212> DNA
<213> Artificial Sequence

<220>
<223> Artificial Sequence

<220>
<221> misc_feature
<222> (1)..(10)
<223> a sequcence

<400> 46
cgagcgttgc 10

<210> 47
<211> 10
<212> DNA
<213> Artificial Sequence

<220>
<223> y is not exist,just for completing the sequence listing.

<220>
<221> misc_feature
<222> (1)..(9)
<223> b sequcence

<400> 47
gcttcggcgy 10

<210> 48
<211> 12
<212> DNA
<213> Artificial Sequence

<220>
<223> Artificial Sequence

<220>
<221> misc_feature
<222> (1)..(12)
<223> c sequcence

<400> 48
cgcccatagc cg 12

<210> 49
<211> 77
<212> DNA
<213> Artificial Sequence

<220>
<223> Artificial Sequence

<220>
<221> misc_feature
<222> (1)..(77)
<223> a sequcence

<220>
<221> misc_feature
<222> (1)..(77)
<223> M is U or T

<400> 49
cgcgcgaaaa aacgcgcgaa aaaacgcgcg cccaccagcg mmccgggcgc gcgaaaaaac 60

gcgcgaaaaa acgcgcg 77

<210> 50
<211> 75
<212> DNA
<213> Artificial Sequence

<220>
<223> Artificial Sequence

<220>
<221> misc_feature
<222> (1)..(75)
<223> b sequcence

<220>
<221> misc_feature
<222> (1)..(75)
<223> M is U or T

<400> 50
cgcgcgmmmm mmcgcgcgmm mmmmcgcgcg cccggmmcgc cgccagccgc cmmmmmmgcc 60

gccmmmmmmg ccgcc 75

<210> 51
<211> 78
<212> DNA
<213> Artificial Sequence

<220>
<223> Artificial Sequence

<220>
<221> misc_feature
<222> (1)..(78)

<223> c sequcence

<220>
<221> misc_feature
<222> (1)..(78)
<223> M is U or T

<400> 51
ggcggcaaaa aaggcggcaa aaaaggcggc aggcggcama gcggmgggcg cgcgmmmmmm 60

cgcgcgmmmm mmcgcgcg 78

<210> 52
<211> 29
<212> RNA
<213> Artificial Sequence

<220>
<223> Artificial Sequence

<220>
<221> misc_feature
<222> (1)..(29)
<223> a strand

<220>
<221> modified_base
<222> (1)..(1)
<223> 5'Biotin

<400> 52
cgcgcgccca ccagcguucc gggcgccgc 29

<210> 53
<211> 27
<212> RNA
<213> Artificial Sequence

<220>
<223> Artificial Sequence

<220>
<221> misc_feature
<222> (1)..(27)
<223> b strand

<220>
<221> modified_base
<222> (1)..(1)
<223> 5'Biotin

<400> 53
gcggcgcccg guucgccgcc aggcggc 27

<210> 54
<211> 31
<212> RNA
<213> Artificial Sequence

<220>
<223> Artificial Sequence

<220>
<221> misc_feature
<222> (1)..(31)
<223> c strand

<220>
<221> modified_base
<222> (1)..(1)
<223> 5'CY3

<400> 54
gccgccaggc ggccauagcg gugggcgcgc g 31

<210> 55
<211> 10
<212> RNA
<213> Artificial Sequence

<220>
<223> Artificial Sequence

<220>
<221> misc_feature
<222> (1)..(10)
<223> a strand

<400> 55
ggagcguugg 10

<210> 56
<211> 10
<212> RNA
<213> Artificial Sequence

<220>
<223> y is not exist,just for completing the sequence listing.

<220>
<221> misc_feature
<222> (1)..(9)
<223> b strand

<400> 56
ccuucgccgy 10

<210> 57
<211> 12
<212> RNA
<213> Artificial Sequence

<220>
<223> Artificial Sequence

<220>
<221> misc_feature
<222> (1)..(12)
<223> c strand

<400> 57
cggccauagc cc 12

<210> 58
<211> 10
<212> RNA
<213> Artificial Sequence

<220>
<223> Artificial Sequence

<220>
<221> misc_feature
<222> (1)..(10)
<223> a strand

<400> 58
gcagcguucg 10

<210> 59
<211> 10
<212> RNA
<213> Artificial Sequence

<220>
<223> y is not exist,just for completing the sequence listing.

<220>
<221> misc_feature
<222> (1)..(9)
<223> b strand

<400> 59
cguucgccgy 10

<210> 60
<211> 12
<212> RNA
<213> Artificial Sequence

<220>
<223> Artificial Sequence

<220>
<221> misc_feature
<222> (1)..(12)
<223> c strand

<400> 60
cggccauagc gc 12

<210> 61
<211> 10
<212> RNA
<213> Artificial Sequence

<220>
<223> Artificial Sequence

<220>
<221> misc_feature
<222> (1)..(10)
<223> a strand

<400> 61
cgagcguugc 10

<210> 62
<211> 10
<212> RNA
<213> Artificial Sequence

<220>
<223> y is not exist,just for completing the sequence listing.

<220>
<221> misc_feature
<222> (1)..(9)
<223> b strand

<400> 62
gcuucgccgy 10

<210> 63
<211> 12
<212> RNA
<213> Artificial Sequence

<220>
<223> Artificial Sequence

<220>
<221> misc_feature
<222> (1)..(12)
<223> c strand

<400> 63
cggccauagc cg 12

<210> 64
<211> 10
<212> RNA
<213> Artificial Sequence

<220>
<223> Artificial Sequence

<220>
<221> misc_feature
<222> (1)..(10)
<223> a strand

<400> 64
ggagcguugg 10

<210> 65
<211> 10
<212> RNA
<213> Artificial Sequence

<220>
<223> y is not exist,just for completing the sequence listing.

<220>
<221> misc_feature
<222> (1)..(9)
<223> b strand

<400> 65
ccuucggggy 10

<210> 66
<211> 12
<212> RNA
<213> Artificial Sequence

<220>
<223> Artificial Sequence

<220>
<221> misc_feature
<222> (1)..(12)
<223> c strand

<400> 66
cccccauagc cc 12

<210> 67
<211> 10
<212> RNA
<213> Artificial Sequence

<220>
<223> Artificial Sequence

<220>
<221> misc_feature
<222> (1)..(10)
<223> a strand

<400> 67
gcagcguucg 10

<210> 68
<211> 10
<212> RNA
<213> Artificial Sequence

<220>
<223> y is not exist,just for completing the sequence listing.

<220>
<221> misc_feature
<222> (1)..(9)
<223> b strand

<400> 68
cguucggcgy 10

<210> 69
<211> 12
<212> RNA
<213> Artificial Sequence

<220>
<223> Artificial Sequence

<220>
<221> misc_feature
<222> (1)..(12)
<223> c strand

<400> 69
cgcccauagc gc 12

<210> 70
<211> 10
<212> RNA
<213> Artificial Sequence

<220>
<223> Artificial Sequence

<220>
<221> misc_feature
<222> (1)..(10)
<223> a strand

<400> 70
gcagcguucg 10

<210> 71
<211> 10
<212> RNA
<213> Artificial Sequence

<220>
<223> y is not exist,just for completing the sequence listing.

<220>
<221> misc_feature
<222> (1)..(9)
<223> b strand

<400> 71
cguucggccy 10

<210> 72
<211> 12
<212> RNA
<213> Artificial Sequence

<220>
<223> Artificial Sequence

<220>
<221> misc_feature
<222> (1)..(12)
<223> c strand

<400> 72
ggcccauagc gc 12

<210> 73
<211> 10
<212> RNA
<213> Artificial Sequence

<220>
<223> Artificial Sequence

<220>
<221> misc_feature
<222> (1)..(10)
<223> a strand

<400> 73
cgagcguugc 10

<210> 74
<211> 10
<212> RNA
<213> Artificial Sequence

<220>
<223> y is not exist,just for completing the sequence listing.

<220>
<221> misc_feature
<222> (1)..(9)
<223> b strand

<400> 74
gcuucggcgy 10

<210> 75
<211> 12
<212> RNA
<213> Artificial Sequence

<220>
<223> Artificial Sequence

<220>
<221> misc_feature
<222> (1)..(12)
<223> c strand

<400> 75
cgcccauagc cg 12

<210> 76
<211> 29
<212> RNA
<213> Artificial Sequence

<220>
<223> Artificial Sequence

<220>
<221> misc_feature
<222> (1)..(29)
<223> a strand

<400> 76
cgcgcgccca ggagcguugg cgggcggcg 29

<210> 77
<211> 27
<212> RNA
<213> Artificial Sequence

<220>
<223> Artificial Sequence

<220>
<221> misc_feature
<222> (1)..(27)
<223> b strand

<400> 77
cgccgcccgc cuucgccgcc agccgcc 27

<210> 78
<211> 31
<212> RNA
<213> Artificial Sequence

<220>
<223> Artificial Sequence

<220>
<221> misc_feature
<222> (1)..(31)
<223> c strand

<400> 78
ggcggcaggc ggccauagcc cugggcgcgc g 31

<210> 79
<211> 29
<212> RNA
<213> Artificial Sequence

<220>
<223> Artificial Sequence

<220>
<221> misc_feature
<222> (1)..(29)
<223> a strand

<400> 79
cgcgcgccca gcagcguucg cgggcggcg 29

<210> 80
<211> 27
<212> RNA
<213> Artificial Sequence

<220>
<223> Artificial Sequence

<220>
<221> misc_feature
<222> (1)..(27)
<223> b strand

<400> 80
cgccgcccgc guucgccgcc agccgcc 27

<210> 81
<211> 31
<212> RNA
<213> Artificial Sequence

<220>
<223> Artificial Sequence

<220>
<221> misc_feature
<222> (1)..(31)
<223> c strand

<400> 81
ggcggcaggc ggccauagcg cugggcgcgc g 31

<210> 82
<211> 29
<212> RNA
<213> Artificial Sequence

<220>
<223> Artificial Sequence

<220>
<221> misc_feature
<222> (1)..(29)
<223> a strand

<400> 82
cgcgcgccca cgagcguugc ggggcggcg 29

<210> 83
<211> 27
<212> RNA
<213> Artificial Sequence

<220>
<223> Artificial Sequence

<220>
<221> misc_feature
<222> (1)..(27)
<223> b strand

<400> 83
cgccgccccg cuucgccgcc agccgcc 27

<210> 84
<211> 31
<212> RNA
<213> Artificial Sequence

<220>
<223> Artificial Sequence

<220>
<221> misc_feature
<222> (1)..(31)
<223> c strand

<400> 84
ggcggcaggc ggccauagcc gugggcgcgc g 31

<210> 85
<211> 29
<212> RNA
<213> Artificial Sequence

<220>
<223> Artificial Sequence

<220>
<221> misc_feature
<222> (1)..(29)
<223> a strand

<400> 85
cgcgcgccca ggagcguugg cccgcggcg 29

<210> 86
<211> 27
<212> RNA
<213> Artificial Sequence

<220>
<223> Artificial Sequence

<220>
<221> misc_feature
<222> (1)..(27)
<223> b strand

<400> 86
cgccgcgggc cuucggggcc agccgcc 27

<210> 87
<211> 31
<212> RNA
<213> Artificial Sequence

<220>
<223> Artificial Sequence

<220>
<221> misc_feature
<222> (1)..(31)
<223> c strand

<400> 87
ggcggcaggc ccccauagcc cugggcgcgc g 31

<210> 88
<211> 29
<212> RNA
<213> Artificial Sequence

<220>
<223> Artificial Sequence

<220>
<221> misc_feature
<222> (1)..(29)
<223> a strand

<400> 88
cgcgcgccca gcagcguucg ccccgccgc 29

<210> 89
<211> 27
<212> RNA
<213> Artificial Sequence

<220>
<223> Artificial Sequence

<220>
<221> misc_feature
<222> (1)..(27)
<223> b strand

<400> 89
gcggcggggc guucggcggc aggcggc 27

<210> 90
<211> 31
<212> RNA
<213> Artificial Sequence

<220>
<223> Artificial Sequence

<220>
<221> misc_feature
<222> (1)..(31)
<223> c strand

<400> 90
gccgccagcc gcccauagcg cugggcgcgc g 31

<210> 91
<211> 29
<212> RNA
<213> Artificial Sequence

<220>
<223> Artificial Sequence

<220>
<221> misc_feature
<222> (1)..(29)
<223> a strand

<400> 91
cgcgcgccca gcagcguucg gggcgccgc 29

<210> 92
<211> 28
<212> RNA
<213> Artificial Sequence

<220>
<223> Artificial Sequence

<220>
<221> misc_feature
<222> (1)..(28)
<223> b strand

<400> 92
gcggcgcccc guucggccgg caggcggc 28

<210> 93
<211> 32
<212> RNA
<213> Artificial Sequence

<220>
<223> Artificial Sequence

<220>
<221> misc_feature
<222> (1)..(32)
<223> c strand

<400> 93
gccgccagcc ggcccauagc gcugggcgcg cg 32

<210> 94
<211> 40
<212> DNA
<213> Artificial Sequence

<220>
<223> Artificial Sequence

<220>
<221> misc_feature
<222> (1)..(40)
<223> a strand

<400> 94
cgcgcgcgag cguugcaaug acagauaagg aaccugcutt 40

<210> 95
<211> 36
<212> RNA
<213> Artificial Sequence

<220>
<223> Artificial Sequence

<220>
<221> misc_feature
<222> (1)..(36)
<223> b strand

<400> 95
ggcagguucc uuaucuguca aagcuucggc ggcagc 36

<210> 96
<211> 23
<212> RNA
<213> Artificial Sequence

<220>
<223> Artificial Sequence

<220>
<221> misc_feature
<222> (1)..(23)
<223> c strand

<400> 96
gcagccgccc auagccgcgc gcg 23

<210> 97
<211> 39
<212> DNA
<213> Artificial Sequence

<220>
<223> Artificial Sequence

<220>
<221> misc_feature
<222> (1)..(39)
<223> EGFRapt

<400> 97
gccttagtaa cgtgctttga tgtcgattcg acaggaggc 39

<210> 98
<211> 41
<212> DNA
<213> Artificial Sequence

<220>
<223> Artificial Sequence

<220>
<221> misc_feature
<222> (1)..(41)
<223> PSMAapt

<400> 98
gggccgaaaa agacctgact tctatactaa gtctacgtcc c 41

<210> 99
<211> 68
<212> DNA
<213> Artificial Sequence

<220>
<223> Artificial Sequence

<220>
<221> misc_feature
<222> (1)..(68)
<223> a strand

<400> 99
cgcgcgccca ggagcgttgg cgggcggcgg ccttagtaac gtgctttgat gtcgattcga 60

caggaggc 68

<210> 100
<211> 27
<212> DNA
<213> Artificial Sequence

<220>
<223> Artificial Sequence

<220>
<221> misc_feature
<222> (1)..(27)
<223> b strand

<400> 100
cgccgcccgc cttcgccgcc agccgcc 27

<210> 101
<211> 31
<212> DNA
<213> Artificial Sequence

<220>
<223> Artificial Sequence

<220>
<221> misc_feature
<222> (1)..(31)
<223> c strand

<400> 101
ggcggcaggc ggccatagcc ctgggcgcgc g 31

<210> 102
<211> 68
<212> DNA
<213> Artificial Sequence

<220>
<223> Artificial Sequence

<220>
<221> misc_feature
<222> (1)..(68)
<223> a strand

<400> 102

cgcgcgccca gcagcgttcg cgggcggcgg ccttagtaac gtgctttgat gtcgattcga 60

caggaggc 68

<210> 103
<211> 27
<212> DNA
<213> Artificial Sequence

<220>
<223> Artificial Sequence

<220>
<221> misc_feature
<222> (1)..(27)
<223> b strand

<400> 103
cgccgcccgc gttcgccgcc agccgcc 27

<210> 104
<211> 31
<212> DNA
<213> Artificial Sequence

<220>
<223> Artificial Sequence

<220>
<221> misc_feature
<222> (1)..(31)
<223> c strand

<400> 104
ggcggcaggc ggccatagcg ctgggcgcgc g 31

<210> 105
<211> 68
<212> DNA
<213> Artificial Sequence

<220>
<223> Artificial Sequence

<220>
<221> misc_feature
<222> (1)..(68)
<223> a strand

<400> 105
cgcgcgccca cgagcgttgc ggggcggcgg ccttagtaac gtgctttgat gtcgattcga 60

caggaggc 68

<210> 106
<211> 27

<212> DNA
<213> Artificial Sequence

<220>
<223> Artificial Sequence

<220>
<221> misc_feature
<222> (1)..(27)
<223> b strand

<400> 106
cgccgccccg cttcgccgcc agccgcc 27

<210> 107
<211> 31
<212> DNA
<213> Artificial Sequence

<220>
<223> Artificial Sequence

<220>
<221> misc_feature
<222> (1)..(31)
<223> c strand

<400> 107
ggcggcaggc ggccatagcc gtgggcgcgc g 31

<210> 108
<211> 71
<212> DNA
<213> Artificial Sequence

<220>
<223> Artificial Sequence

<220>
<221> misc_feature
<222> (1)..(71)
<223> a strand

<400> 108
cgcgcgccca ggagcgttgg cccgcggcgt gggccgaaaa agacctgact tctatactaa 60

gtctacgtcc c 71

<210> 109
<211> 27
<212> DNA
<213> Artificial Sequence

<220>
<223> Artificial Sequence

<220>
<221> misc_feature
<222> (1)..(27)
<223> b strand

<400> 109
cgccgcgggc cttcggggcc agccgcc 27

<210> 110
<211> 31
<212> DNA
<213> Artificial Sequence

<220>
<223> Artificial Sequence

<220>
<221> misc_feature
<222> (1)..(31)
<223> c strand

<400> 110
ggcggcaggc ccccatagcc ctgggcgcgc g 31

<210> 111
<211> 71
<212> DNA
<213> Artificial Sequence

<220>
<223> Artificial Sequence

<220>
<221> misc_feature
<222> (1)..(71)
<223> a strand

<400> 111
cgcgcgccca gcagcgttcg ccccgccgct gggccgaaaa agacctgact tctatactaa 60

gtctacgtcc c 71

<210> 112
<211> 27
<212> DNA
<213> Artificial Sequence

<220>
<223> Artificial Sequence

<220>
<221> misc_feature
<222> (1)..(27)
<223> b strand

<400> 112
gcggcggggc gttcggcggc aggcggc 27

<210> 113
<211> 31
<212> DNA
<213> Artificial Sequence

<220>
<223> Artificial Sequence

<220>
<221> misc_feature
<222> (1)..(31)
<223> c strand

<400> 113
gccgccagcc gcccatagcg ctgggcgcgc g 31

<210> 114
<211> 29
<212> DNA
<213> Artificial Sequence

<220>
<223> Artificial Sequence

<220>
<221> misc_feature
<222> (1)..(29)
<223> a strand

<400> 114
cgcgcgccca gcagcgttcg gggcgccgc 29

<210> 115
<211> 28
<212> DNA
<213> Artificial Sequence

<220>
<223> Artificial Sequence

<220>
<221> misc_feature
<222> (1)..(20)
<223> b strand

<400> 115
gcggcgcccc gttcggccgg caggcggc 28

<210> 116
<211> 32
<212> DNA
<213> Artificial Sequence

<220>
<223> Artificial Sequence

<220>
<221> misc_feature
<222> (1)..(32)
<223> c strand

<400> 116
gccgccagcc ggcccatagc gctgggcgcg cg 32

<210> 117
<211> 29
<212> DNA
<213> Artificial Sequence

<220>
<223> Artificial Sequence

<220>
<221> misc_feature
<222> (1)..(29)
<223> a strand

<400> 117
cgcgcgccca cgagcgttgc gggcgccgc 29

<210> 118
<211> 27
<212> DNA
<213> Artificial Sequence

<220>
<223> Artificial Sequence

<220>
<221> misc_feature
<222> (1)..(27)
<223> b strand

<400> 118
gcggcgcccg cttcggcggc aggcggc 27

<210> 119
<211> 31
<212> DNA
<213> Artificial Sequence

<220>
<223> Artificial Sequence

<220>
<221> misc_feature
<222> (1)..(31)
<223> c strand

<400> 119
gccgccagcc gcccatagcc gtgggcgcgc g 31

<210> 120
<211> 37
<212> RNA
<213> Artificial Sequence

<220>
<223> Artificial Sequence

<220>
<221> misc_feature
<222> (1)..(37)
<223> a strand

<400> 120
gcggcgagcg gcgaggagcg uuggggccgg aggccgg 37

<210> 121
<211> 31
<212> RNA
<213> Artificial Sequence

<220>
<223> Artificial Sequence

<220>
<221> misc_feature
<222> (1)..(37)
<223> b strand

<400> 121
ccggccuccg gccccuucgg ggccagccgc c 31

<210> 122
<211> 35
<212> RNA
<213> Artificial Sequence

<220>
<223> Artificial Sequence

<220>
<221> misc_feature
<222> (1)..(35)
<223> c strand

<400> 122
ggcggcaggc ccccauagcc cucgccgcuc gccgc 35

<210> 123
<211> 37
<212> RNA
<213> Artificial Sequence

<220>
<223> Artificial Sequence

<220>
<221> misc_feature
<222> (1)..(37)
<223> a strand

<400> 123
gcggcgagcg gcgagcagcg uucgggccgg aggccgg 37

<210> 124
<211> 31
<212> RNA
<213> Artificial Sequence

<220>
<223> Artificial Sequence

<220>
<221> misc_feature
<222> (1)..(31)
<223> b strand

<400> 124
ccggccuccg gcccguucgc cgccagccgc c 31

<210> 125
<211> 35
<212> RNA
<213> Artificial Sequence

<220>
<223> Artificial Sequence

<220>
<221> misc_feature
<222> (1)..(35)
<223> c strand

<400> 125
ggcggcaggc ggccauagcg cucgccgcuc gccgc 35

<210> 126
<211> 37
<212> RNA
<213> Artificial Sequence

<220>
<223> Artificial Sequence

<220>
<221> misc_feature
<222> (1)..(37)
<223> a strand

<400> 126
gcggcgagcg gcgaggagcg uuggggccgg aggccgg 37

<210> 127
<211> 31
<212> RNA
<213> Artificial Sequence

<220>
<223> Artificial Sequence

<220>
<221> misc_feature
<222> (1)..(31)
<223> b strand

<400> 127
ccggccuccg gccccuucgc cgccagccgc c 31

<210> 128
<211> 35
<212> RNA
<213> Artificial Sequence

<220>
<223> Artificial Sequence

<220>
<221> misc_feature
<222> (1)..(35)
<223> c strand

<400> 128
ggcggcaggc ggccauagcc cucgccgcuc gccgc 35

<210> 129
<211> 37
<212> RNA
<213> Artificial Sequence

<220>
<223> Artificial Sequence

<220>
<221> misc_feature
<222> (1)..(37)
<223> a strand

<400> 129
gcggcgagcg gcgagcagcg uucgggccgg aggccgg 37

<210> 130
<211> 31
<212> RNA
<213> Artificial Sequence

<220>
<223> Artificial Sequence

<220>
<221> misc_feature
<222> (1)..(31)
<223> b strand

<400> 130
ccggccuccg gcccguucgg cgccagccgc c 31

<210> 131
<211> 35
<212> RNA
<213> Artificial Sequence

<220>
<223> Artificial Sequence

<220>
<221> misc_feature
<222> (1)..(35)
<223> c strand

<400> 131
ggcggcaggc gcccauagcg cucgccgcuc gccgc 35

<210> 132
<211> 37
<212> RNA
<213> Artificial Sequence

<220>
<223> Artificial Sequence

<220>
<221> misc_feature
<222> (1)..(37)
<223> a strand

<400> 132
gcggcgagcg gcgagcagcg uucgggccgg aggccgg 37

<210> 133
<211> 31
<212> RNA
<213> Artificial Sequence

<220>
<223> Artificial Sequence

<220>
<221> misc_feature
<222> (1)..(31)
<223> b strand

<400> 133
ccggccuccg gcccguucgg ccccagccgc c 31

<210> 134
<211> 35
<212> RNA
<213> Artificial Sequence

<220>
<223> Artificial Sequence

<220>
<221> misc_feature
<222> (1)..(35)
<223> c strand

<400> 134
ggcggcaggg gcccauagcg cucgccgcuc gccgc 35

<210> 135
<211> 37
<212> RNA
<213> Artificial Sequence

<220>
<223> Artificial Sequence

<220>
<221> misc_feature
<222> (1)..(37)
<223> a strand

<400> 135
gcggcgagcg gcgacgagcg uugcggccgg aggccgg 37

<210> 136
<211> 31
<212> RNA
<213> Artificial Sequence

<220>
<223> Artificial Sequence

<220>
<221> misc_feature
<222> (1)..(31)
<223> b strand

<400> 136
ccggccuccg gccgcuucgc cgccagccgc c 31

<210> 137
<211> 35
<212> RNA
<213> Artificial Sequence

<220>
<223> Artificial Sequence

<220>
<221> misc_feature
<222> (1)..(35)
<223> c strand

<400> 137
ggcggcaggc ggccauagcc gucgccgcuc gccgc 35

<210> 138
<211> 37
<212> RNA
<213> Artificial Sequence

<220>
<223> Artificial Sequence

<220>
<221> misc_feature
<222> (1)..(37)
<223> a strand

<400> 138
gcggcgagcg gcgacgagcg uugcggccgg aggccgg 37

<210> 139
<211> 31
<212> RNA
<213> Artificial Sequence

<220>
<223> Artificial Sequence

<220>
<221> misc_feature
<222> (1)..(31)
<223> b strand

<400> 139
ccggccuccg gccgcuucgg cgccagccgc c 31

<210> 140
<211> 35
<212> RNA
<213> Artificial Sequence

<220>
<223> Artificial Sequence

<220>
<221> misc_feature
<222> (1)..(35)
<223> c strand

<400> 140
ggcggcaggc gcccauagcc gucgccgcuc gccgc 35

<210> 141
<211> 37
<212> DNA
<213> Artificial Sequence

<220>
<223> Artificial Sequence

<220>
<221> misc_feature
<222> (1)..(37)
<223> a strand

<400> 141
gcggcgagcg gcgaggagcg ttggggccgg aggccgg 37

<210> 142
<211> 31
<212> DNA
<213> Artificial Sequence

<220>
<223> Artificial Sequence

<220>
<221> misc_feature
<222> (1)..(31)
<223> b strand

<400> 142
ccggcctccg gccccttcgg ggccagccgc c 31

<210> 143
<211> 35
<212> DNA
<213> Artificial Sequence

<220>
<223> Artificial Sequence

<220>
<221> misc_feature
<222> (1)..(35)
<223> c strand

<400> 143
ggcggcaggc ccccatagcc ctcgccgctc gccgc 35

<210> 144
<211> 37
<212> DNA
<213> Artificial Sequence

<220>
<223> Artificial Sequence

<220>
<221> misc_feature
<222> (1)..(37)
<223> a strand

<400> 144
gcggcgagcg gcgagcagcg ttcgggccgg aggccgg 37

<210> 145
<211> 31
<212> DNA
<213> Artificial Sequence

<220>
<223> Artificial Sequence

<220>
<221> misc_feature
<222> (1)..(31)
<223> b strand

<400> 145
ccggcctccg gcccgttcgc cgccagccgc c 31

<210> 146
<211> 35
<212> DNA
<213> Artificial Sequence

<220>
<223> Artificial Sequence

<220>
<221> misc_feature
<222> (1)..(35)
<223> c strand

<400> 146
ggcggcaggc ggccatagcg ctcgccgctc gccgc 35

<210> 147
<211> 37
<212> DNA
<213> Artificial Sequence

<220>
<223> Artificial Sequence

<220>
<221> misc_feature
<222> (1)..(37)
<223> a strand

<400> 147
gcggcgagcg gcgaggagcg ttggggccgg aggccgg 37

<210> 148
<211> 31
<212> DNA
<213> Artificial Sequence

<220>
<223> Artificial Sequence

<220>
<221> misc_feature
<222> (1)..(31)
<223> b strand

<400> 148
ccggcctccg gccccttcgc cgccagccgc c 31

<210> 149
<211> 35
<212> DNA
<213> Artificial Sequence

<220>
<223> Artificial Sequence

<220>
<221> misc_feature
<222> (1)..(35)
<223> c strand

<400> 149
ggcggcaggc ggccatagcc ctcgccgctc gccgc 35

<210> 150
<211> 37
<212> DNA
<213> Artificial Sequence

<220>
<223> Artificial Sequence

<220>
<221> misc_feature
<222> (1)..(37)
<223> a strand

<400> 150
gcggcgagcg gcgagcagcg ttcgggccgg aggccgg 37

<210> 151
<211> 31
<212> DNA
<213> Artificial Sequence

<220>
<223> Artificial Sequence

<220>
<221> misc_feature
<222> (1)..(31)
<223> b strand

<400> 151
ccggcctccg gcccgttcgg cgccagccgc c 31

<210> 152
<211> 35
<212> DNA
<213> Artificial Sequence

<220>
<223> Artificial Sequence

<220>
<221> misc_feature
<222> (1)..(35)
<223> c strand

<400> 152
ggcggcaggc gcccatagcg ctcgccgctc gccgc 35

<210> 153
<211> 37
<212> DNA
<213> Artificial Sequence

<220>
<223> Artificial Sequence

<220>
<221> misc_feature
<222> (1)..(37)
<223> a strand

<400> 153
gcggcgagcg gcgagcagcg ttcgggccgg aggccgg 37

<210> 154
<211> 31
<212> DNA
<213> Artificial Sequence

<220>
<223> Artificial Sequence

<220>
<221> misc_feature
<222> (1)..(31)
<223> b strand

<400> 154
ccggcctccg gcccgttcgg ccccagccgc c 31

<210> 155
<211> 35
<212> DNA
<213> Artificial Sequence

<220>
<223> Artificial Sequence

<220>
<221> misc_feature
<222> (1)..(35)
<223> c strand

<400> 155
ggcggcaggg gcccatagcg ctcgccgctc gccgc 35

<210> 156
<211> 37
<212> DNA
<213> Artificial Sequence

<220>
<223> Artificial Sequence

<220>
<221> misc_feature
<222> (1)..(37)
<223> a strand

<400> 156
gcggcgagcg gcgacgagcg ttgcggccgg aggccgg 37

<210> 157
<211> 31
<212> DNA
<213> Artificial Sequence

<220>
<223> Artificial Sequence

<220>
<221> misc_feature
<222> (1)..(31)
<223> b strand

<400> 157
ccggcctccg gccgcttcgc cgccagccgc c 31

<210> 158
<211> 35
<212> DNA
<213> Artificial Sequence

<220>
<223> Artificial Sequence

<220>
<221> misc_feature
<222> (1)..(35)
<223> c strand

<400> 158
ggcggcaggc ggccatagcc gtcgccgctc gccgc 35

<210> 159
<211> 37
<212> DNA
<213> Artificial Sequence

<220>
<223> Artificial Sequence

<220>
<221> misc_feature
<222> (1)..(37)
<223> a strand

<400> 159
gcggcgagcg gcgacgagcg ttgcggccgg aggccgg 37

<210> 160
<211> 31
<212> DNA
<213> Artificial Sequence

<220>
<223> Artificial Sequence

<220>
<221> misc_feature
<222> (1)..(31)
<223> b strand

<400> 160
ccggcctccg gccgcttcgg cgccagccgc c 31

<210> 161
<211> 35
<212> DNA
<213> Artificial Sequence

<220>
<223> Artificial Sequence

<220>
<221> misc_feature
<222> (1)..(35)
<223> c strand

<400> 161
ggcggcaggc gcccatagcc gtcgccgctc gccgc 35

<210> 162
<211> 14
<212> DNA
<213> Artificial Sequence

<220>
<223> Artificial Sequence

<220>
<221> misc_feature
<222> (1)..(14)
<223> first elongating fragment

<400> 162
gcggcgagcg gcga 14

<210> 163
<211> 14
<212> DNA
<213> Artificial Sequence

<220>
<223> Artificial Sequence

<220>
<221> misc_feature
<222> (1)..(14)
<223> first elongating fragment

<400> 163
ucgccgcucg ccgc 14

<210> 164
<211> 13
<212> DNA
<213> Artificial Sequence

<220>
<223> Artificial Sequence

<220>
<221> misc_feature
<222> (1)..(13)
<223> first elongating fragment

<400> 164
ggccggaggc cgg 13

<210> 165
<211> 13
<212> DNA
<213> Artificial Sequence

<220>
<223> Artificial Sequence

<220>
<221> misc_feature
<222> (1)..(13)
<223> first elongating fragment

<400> 165
ccggccuccg gcc 13

<210> 166
<211> 10
<212> DNA
<213> Artificial Sequence

<220>
<223> y is not exist,just for completing the sequence listing.

<220>
<221> misc_feature
<222> (1)..(9)
<223> first elongating fragment

<400> 166
ccagccgccy 10

<210> 167
<211> 10
<212> DNA
<213> Artificial Sequence

<220>
<223> y is not exist,just for completing the sequence listing.

<220>
<221> misc_feature
<222> (1)..(9)
<223> first elongating fragment

<400> 167
ggcggcaggy 10

<210> 168
<211> 14
<212> DNA
<213> Artificial Sequence

<220>
<223> Artificial Sequence

<220>
<221> misc_feature
<222> (1)..(14)
<223> first elongating fragment

<400> 168
gcggcgagcg gcga 14

<210> 169
<211> 14
<212> DNA
<213> Artificial Sequence

<220>
<223> Artificial Sequence

<220>
<221> misc_feature
<222> (1)..(14)
<223> first elongating fragment

<400> 169
tcgccgctcg ccgc 14

<210> 170
<211> 13
<212> DNA
<213> Artificial Sequence

<220>
<223> Artificial Sequence

<220>
<221> misc_feature
<222> (1)..(13)
<223> first elongating fragment

<400> 170
ggccggaggc cgg 13

<210> 171
<211> 13
<212> DNA
<213> Artificial Sequence

<220>
<223> first elongating fragment

<220>
<221> misc_feature
<222> (1)..(13)
<223> first elongating fragment

<400> 171
ccggcctccg gcc 13

## Description

### Technical Field

The disclosure relates to the field of delivery carriers, and in particular to nucleic acid nanoparticles, a pharmaceutical composition comprising the same, a drug comprising doxorubicin and a preparation method thereof.

### Background

Cancer has become a most important disease to endanger human health. Drug therapy, including cell-targeted drug administration, gene therapy, RNAi and the like, is all frontier research fields of conquering the cancer. However, many anti-cancer drugs have defects such as indissolubility in water or poor stability. For example, the doxorubicin and the like are difficult to be used by an organism due to poor solubility, and it is the key to clinical applications of such drug preparations that a problem of water solubility thereof is solved. In addition, the effects of oncotherapy and diagnostic drugs are mostly non-selective, and usually have large toxic side effects on normal tissues and organs at therapeutic doses. As the delivery of many protein substances, the degradation and lower bioavailability of nucleic acid substances in a gastrointestinal tract are main obstacles to the oral delivery of siRNA. Even for the intravenous delivery, conventional siRNA is rapidly degraded by serum factors, and thereby a target position thereof may not be reached.

Active ingredients of the drugs often may not achieve therapy purposes or generate the toxic side effects due to failing to reach a lesion site effectively or acting excessively on normal cells and tissues. For example, platinum drugs are more typical, and are widely used in chemotherapy of ovarian cancer, small cell lung cancer, testicular cancer, head and neck squamous cancer, cervical cancer, non-small cell lung cancer, bladder cancer, pleural mesothelioma, melanoma, and endometrial cancer. However, the platinum drugs are poor in targeting, and the therapeutic effect may not be achieved by general drug doses, but the excessive doses may cause the drugs to act on the normal cells and tissues in large amounts, adverse effects, such as a human bone marrow suppression effect, a gastrointestinal tract effect, nephrotoxicity, and neurotoxicity, after the chemotherapy are caused, thus the use of the platinum chemotherapy drugs is limited.

In order to alleviate the side effects generated by the poor targeting of the active ingredients of the drugs, a drug delivery carrier is generated at the right moment, and a function thereof is mainly to carry the active ingredients of the drugs and deliver the active ingredients into blood or tissue cells to treat diseases. For example, in the chemotherapy of the cancer, a chemotherapeutic drug is delivered into cancer cells by the delivery carrier, so that the active ingredients of the drug interact with a DNA in the cancer cell, and an inhibitory effect is generated on the tumor. At present, the commonly used platinum chemotherapeutic drug delivery carriers include a liposome, a micelle, a nanocapsule, a polymer-platinum conjugate and a carbon nanotube and the like.

In addition, there are already a variety of methods to achieve targeted delivery of the different drugs at present. It may be achieved by using an instrument or an apparatus, such as a gene gun, and an electroporation apparatus. These methods do not need to use gene carriers, but the transfection efficiency is very low generally, steps are complicated, and the damage to the tissues is relatively large. It may also be mediated by using viral carriers, such as an adenovirus, and a lentivirus. Although the viral carriers have higher transfection activity in vitro, immunogenicity thereof and a disadvantage that it is easy to cause mutations have huge safety hidden hazards to the delivery in vivo. There are also non-viral carriers, especially biodegradable high molecular materials, to achieve the targeted delivery of the drugs. A main advantage of the non-viral carriers is that under a condition of guaranteeing the expected transfection activity, the immunogenicity and many inflammatory responses caused by the viral carriers may be greatly reduced.

In the above multiple targeted delivery modes, more researches are focused on the field of the non-viral carriers at present, and it is generally a plurality of the following carrier designs: (a) a cationic liposome; and (b) a polycationic gene carrier. At present, the more researches focus on modification of the polycationic gene carrier and the cationic liposome, so that it is suitable for the targeted delivery of the genetic substances. The cationic liposome has the higher transfection activity in vivo and in vitro. However, because normal distribution thereof in vivo is affected by positive charge on a surface, at the same time, the cationic liposome may cause the immunogenicity and the inflammatory responses in animal experiments. The development of the polycationic gene carrier is relatively mature at present, but it is difficult to guarantee that a targeting group is on a surface of a structure in a structural design, and there is an own design contradiction between the toxicity and the transfection activity, at the same time, connection thereof is difficult to achieve non-toxic degradation in vivo. However, it may be seen from the above that the more existing non-viral carrier researches focus on nucleic acid drugs, and there is no valuable report on the delivery effect of non-nucleic acid drugs.

Therefore, how to provide a reliable drug carrier is the key to solve the existing limited clinical applications of the drugs.

## Summary

A main purpose of the disclosure is to provide nucleic acid nanoparticles, a pharmaceutical composition comprising the same, a drug comprising doxorubicin and a preparation method thereof, as to provide a reliable drug carrier to solve a problem of currently limited clinical applications of drugs.

In order to achieve the above purpose, according to one aspect of the disclosure, a nucleic acid nanoparticle is provided, it has a nucleic acid structural domain, the nucleic acid structural domain includes a sequence a, a sequence b and a sequence c. The sequence a includes a sequence a1 or a sequence obtained by insertion, deletion or substitution of at least one base in the sequence a1, the sequence b includes a sequence b1 or a sequence obtained by insertion, deletion or substitution of at least one base in the sequence b1, and the sequence c includes a sequence c1 or a sequence obtained by insertion, deletion or substitution of at least one base in the sequence c1, herein the sequence a1 is SEQ ID NO:1:5'-CCAGCGUUCC-3' or SEQ ID NO:2:5'-CCAGCGTTCC-3'; the sequence b1 is SEQ ID NO:3:5'-GGUUCGCCG-3' or SEQ ID NO:4:5'-GGTTCGCCG-3'; and the sequence c1 is SEQ ID NO:5:5'-CGGCCAUAGCGG-3' or SEQ ID NO:6:5'-CGGCCATAGCGG-3'.

Further, when the sequence a1 is the SEQ ID NO:1, the sequence b1 is the SEQ ID NO:3, and the sequence c1 is the SEQ ID NO:5, at least one sequence of the sequence a, the second b and the sequence c includes a sequence obtained by insertion, deletion or substitution of at least one base in the sequence a, b and/or c.

Further, the insertion, deletion or substitution of at least one base is occurred:

(1) at 1, 2, 4 or 5-th base starting from a 5 '-end of the sequence shown in the SEQ ID NO:1 or the SEQ ID NO:2; and/or
(2) between 8-th and 10-th bases starting from the 5 '-end of the sequence shown in the SEQ ID NO:1 or the SEQ ID NO:2; and/or
(3) between 1-th and 3-th bases starting from a 5 '-end of the sequence shown in the SEQ ID NO:3 or the SEQ ID NO:4; and/or
(4) between 6-th and 9-th bases starting from the 5 '-end of the sequence shown in the SEQ ID NO:3 or the SEQ ID NO:4; and/or
(5) between 1-th and 4-th bases starting from a 5 '-end of the sequence shown in the SEQ ID NO:5 or the SEQ ID NO:6; and/or
(6) between 9-th and 12-th bases starting from the 5 '-end of the sequence shown in the SEQ ID NO:5 or the SEQ ID NO:6.

Further, the sequence a, the sequence b and the sequence c are self-assembled into a structure shown in Formula (1):

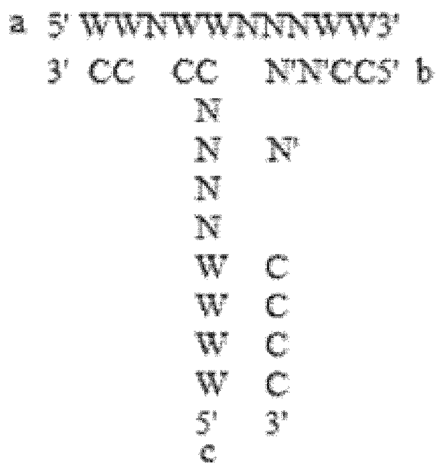


Formula (1)

Herein, W-C represents Watson-Crick pairing, N and N' represent non-Watson-Crick pairing, the W-C in any one position is independently selected from C-G or G-C; in the sequence a, the first N from the 5'-end is A, the second N is G, the third N is U or T, and the fourth N is any one of U, T, A, C or G; in the sequence b, the first N' from the 5'-end is any one of U, T, A, C or G, the second N' is U or T, and the third N' is C; and in the sequence c, a sequence NNNN along a direction from the 5'-end to the 3'-end is CAUA or CATA.

Further, the sequence a, the sequence b and the sequence c are any one of the following groups:

(1)

sequence a: 5'-GGAGCGUUGG-3',
sequence b: 5'-CCUUCGCCG-3',
sequence c: 5'-CGGCCAUAGCCC-3';

(2)

sequence a: 5'-GCAGCGUUCG-3',
sequence b: 5'-CGUUCGCCG-3',
sequence c: 5'-CGGCCAUAGCGC-3';

(3)

sequence a: 5'-CGAGCGUUGC-3',
sequence b: 5'-GCUUCGCCG-3',
sequence c: 5'-CGGCCAUAGCCG-3';

(4)

sequence a: 5'-GGAGCGUUGG-3',
sequence b: 5'-CCUUCGGGG-3',
sequence c: 5'-CCCCCAUAGCCC-3';

(5)

sequence a: 5'-GCAGCGUUCG-3',
sequence b: 5'-CGUUCGGCG-3',
sequence c: 5'-CGCCCAUAGCGC-3';

(6)

sequence a: 5'-GCAGCGUUCG-3',
sequence b: 5'-CGUUCGGCC-3',
sequence c: 5'-GGCCCAUAGCGC-3';

(7)

sequence a: 5'-CGAGCGUUGC-3',
sequence b: 5'-GCUUCGGCG-3',
sequence c: 5'-CGCCCAUAGCCG-3';

(8)

sequence a: 5'-GGAGCGTTGG-3',
sequence b: 5'-CCTTCGCCG-3',
sequence c: 5'-CGGCCATAGCCC-3';

(9)

sequence a: 5'-GCAGCGTTCG-3',
sequence b: 5'-CGTTCGCCG-3',
sequence c: 5'-CGGCCATAGCGC-3';

(10)

sequence a: 5'-CGAGCGTTGC-3',
sequence b: 5'-GCTTCGCCG-3',

sequence c: 5'-CGGCCATAGCCG-3';

(11)

sequence a: 5'-GGAGCGTTGG-3',
sequence b: 5'-CCTTCGGGG-3',
sequence c: 5'-CCCCCATAGCCC-3';

(12)

sequence a: 5'-GCAGCGTTCG-3',
sequence b: 5'-CGTTCGGCG-3',
sequence c: 5'-CGCCCATAGCGC-3';

(13)

sequence a: 5'-GCAGCGTTCG-3',
sequence b: 5'-CGTTCGGCC-3',
sequence c: 5'-GGCCCATAGCGC-3'; and

(14)

sequence a: 5'-CGAGCGTTGC-3',
sequence b: 5'-GCTTCGGCG-3',
sequence c: 5'-CGCCCATAGCCG-3'.

Further, in the nucleic acid structural domain, a first extension fragment is further included, the first extension fragment is an extension fragment of Watson-Crick pairing, and the first extension fragment is positioned at the 5'-end and/or the 3'-end of any one sequence of the sequence a, the sequence b or the sequence c; preferably, the first extension fragment is at least selected from any one of the following groups: (1): a-strand 5'-end: 5'-CCCA-3', c-strand 3'-end: 5'-UGGG-3'; (2): a-strand 3'-end: 5'-GGG-3', b-strand 5'-end: 5'-CCC-3'; (3): b-strand 3'-end: 5'-CCA-3', c-strand 5'-end: 5'-UGG-3'; (4): a-strand 5'-end: 5'-CCCG-3', c-strand 3'-end: 5'-CGGG-3'; (5): a-strand 5'-end: 5'-CCCC-3', c-strand 3'-end: 5'-GGGG-3'; (6): b-strand 3'-end: 5'-CCC-3', c-strand 5'-end: 5'-GGG-3'; (7): b-strand 3'-end: 5'-CCG-3', c-strand 5'-end: 5'-CGG-3'; (8): a-strand 5'-end: 5'-CCCA-3', c-strand 3'-end: 5'-TGGG-3'; and (9): b-strand 3'-end: 5'-CCA-3', c-strand 5'-end: 5'-TGG-3'.

Further, the nucleic acid structural domain further includes a second extension fragment, the second extension fragment is positioned at the 5'-end and/or the 3'-end of any one sequence of the sequence a, the sequence b, or the sequence c, and the second extension fragment is an extension fragment of Watson-Crick pairing; preferably, the second extension fragment is an extension sequence of a CG base pair; and more preferably, the second extension fragment is an extension sequence of 1-10 CG base pairs.

Further, the nucleic acid structural domain further includes at least one group of the following second extension fragments: a first group: a-strand 5'-end: 5'-CGCGCG-3', c-strand 3'-end: 5'-CGCGCG-3'; a second group: a-strand 3'-end: 5'-CGCCGC-3', b-strand 5'-end: 5'-GCGGCG-3'; and a third group: b-strand 3'-end: 5'-GGCGGC-3', c-strand 5'-end: 5'-GCCGCC-3'.

Further the second extension fragment is an extension sequence containing both CG base pair and AT/AU base pair, and preferably the second extension fragment is an extension sequence of 2-50 base pairs.

Further, the second extension fragment is an extension sequence in which sequences of 2-8 continuous CG base pairs and sequences of 2-8 continuous AT/AU base pairs are alternately arranged; or the second extension fragment is an extension sequence in which a sequence of 1 CG base pair and a sequence of 1 AT/AU base pair are alternately arranged.

Further, a base, a ribose and a phosphate in the sequence a, the sequence b and the sequence c have at least one modifiable site, and any one of the modifiable sites is modified by any one of the following modificationadaptors: -F, a methyl, an amino, a disulfide, a carbonyl, a carboxyl, a sulfhydryl and a formyl; and preferably, the base C or U in the sequence a, the sequence b and the sequence c has 2'-F modification.

Further, the nucleic acid nanoparticle further includes a bioactive substance, and the bioactive substance is linked with the nucleic acid structural domain.

Further, a ratio of a relative molecular weight of the nucleic acid structural domain and a total relative molecular weight of the bioactive substance is ≥1:1; and preferably, the bioactive substance is one or more of a target head, a

fluorescein, an interfering nucleic acid siRNA, a miRNA, a ribozyme, a riboswitch, an aptamer, a RNA antibody, a drug, a protein, a polypeptide, a flavonoid, a glucose, a natural salicylic acid, a monoclonal antibody, a vitamin, an phenol and a lecithin.

Further, the bioactive substance is the target head, the fluorescein and the miRNA, herein, the target head is positioned on any one sequence of the sequences a, b and c, preferably the 5'-end or the 3'-end of any one sequence of the sequences a, b and c, or inserted between GC bonds of the nucleic acid structural domain, the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c; and preferably, the target head is a folic acid or a biotin, the fluorescein is any one or more of FAM, CY5 and CY3, and the anti-miRNA is anti-miR-21.

Further, the drug is a drug for treating liver cancer, gastric cancer, lung cancer, breast cancer, head and neck cancer, uterine cancer, ovarian cancer, melanoma, leukemia, Alzheimer's disease, ankylosing spondylitis, malignant lymphoma, bronchial cancer, rheumatoid arthritis, HBV hepatitis B, multiple myeloma, pancreatic cancer, non-small cell lung cancer, prostate cancer, nasopharyngeal cancer, esophageal cancer, oral cancer, lupus erythematosus; and preferably, the head and neck cancer is brain cancer, neuroblastoma or glioblastoma.

Further, the drug is a drug containing any one or more of the following groups: an amino group, a hydroxyl group, a carboxyl group, a mercapto group, a benzene ring group and an acetamido group.

Further, the protein is one or more of antibodies or aptamers of SOD, survivin, hTERT, EGFR and PSMA; the vitamin is L-Vc and/or esterified Vc; and the phenol is a tea polyphenol and/or a grape polyphenol.

Further, the bioactive substance is linked with the nucleic acid structural domain in any one of the following modes: mode I: physical insertion; and mode II: covalent linkage.

Further, when the bioactive substance is linked with the nucleic acid structural domain in the physical insertion mode, the physical insertion is performed on the bioactive substance and the nucleic acid structural domain according to a molar ratio of 1 to 200:1.

Further, when the bioactive substance is linked with the nucleic acid structural domain in the physical insertion mode and the covalent linkage mode, a molar ratio of the bioactive substance linked in the physical insertion mode and the drug linked in the covalent linkage mode is 1 to 200:1.

Further, the bioactive substance linked in the covalent linkage mode is covalently linked through a solvent, covalently linked through a linker or click-linked; preferably, the solvent is selected from paraformaldehyde, DCM, DCC, DMAP, Py, DMSO, PBS or glacial acetic acid; preferably, the linker is selected from a disulfide bond, a p-phenylazide, bromopropyne or a PEG; and preferably, the click-linkage is that a bioactive substance precursor and the nucleic acid structural domain are modified by a alkynyl or azide simultaneously, and then linked through a click reaction.

Further, when the bioactive substance is linked with the nucleic acid structural domain in the click-linkage mode, a site, for performing the alkynyl or azide modification, of the bioactive substance precursor is selected from a 2'-hydroxyl, a carboxyl or an amino, and a site, for performing the alkynyl or azide modification, of the nucleic acid structural domain is selected from a G-exocyclic amino, a 2'-hydroxyl, an $\alpha$-amino or a 2'-hydroxyl.

Further, a particle size of the nucleic acid nanoparticles is 1-100 nm, preferably 5-50 nm; more preferably 10-30 nm; and further preferably 10-15 nm.

According to another aspect of the disclosure, a pharmaceutical composition is further provided, and the pharmaceutical composition includes the above nucleic acid nanoparticles.

According to a third aspect of the disclosure, a drug comprising doxorubicin is further provided, and the drug including the doxorubicin includes the doxorubicin and the nucleic acid nanoparticle (without loading a bioactive substance).

Further, the doxorubicin is loaded on the nucleic acid nanoparticle in the physical linkage mode and/or the covalent linkage mode, and a molar ratio between the doxorubicin and the nucleic acid nanoparticle is 2 to 300:1, preferably 10 to 50:1, and more preferably 15 to 25:1.

Further, the nucleic acid nanoparticle further includes a bioactive substance, the bioactive substance is linked with the nucleic acid structural domain, and the bioactive substance is one or more of a target head, a fluorescein, an interfering nucleic acid siRNA, a miRNA, a ribozyme, a riboswitch, an aptamer, a RNA antibody, a protein, a polypeptide, a flavonoid, a glucose, a natural salicylic acid, a monoclonal antibody, a vitamin, a phenol, a lecithin and a small molecular drug except the doxorubicin.

Further, a relative molecular weight of the nucleic acid structural domain is marked as $N_1$, and a total relative molecular weight of the doxorubicin and the bioactive substance is marked as $N_2$, $N_1/N_{2\geq}1$ :1.

Further, the bioactive substance is one or more of the target head, the fluorescein and the miRNA, herein, the target head is positioned on any one sequence of the sequences a, b and c, preferably the 5'-end or the 3'-end of any one sequence of the sequences a, b and c, or inserted between GC bonds of the nucleic acid structural domain, the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c; and

preferably, the target head is a folic acid or a biotin, the fluorescein is any one or more of FAM, CY5 and CY3, and the anti-miRNA is anti-miR-21.

Further, the small molecular drug except the doxorubicin is a drug containing any one or more of the following groups: an amino group, a hydroxyl group, a carboxyl group, a mercapto group, a benzene ring group and an acetamido group.

Further, the protein is one or more of SOD, survivin, hTERT, EGFR and PSMA; the vitamin is L-Vc and/or esterified Vc; and the phenol is a tea polyphenol and/or a grape polyphenol.

According to a fourth aspect of the disclosure, a preparation method for a drug comprising doxorubicin is further provided, and the preparation method includes the following steps: providing any nucleic acid nanoparticle (without loading a bioactive substance) mentioned above; enabling the doxorubicin to be loaded on the nucleic acid nanoparticle in a physical linkage mode of and/or a covalent linkage mode, to obtain the drug comprising the doxorubicin.

Further, the step of loading the doxorubicin in the physical linkage mode includes: enabling the doxorubicin, the nucleic acid nanoparticle and a first solvent to be mixed and stirring, to obtain a premixed system; and removing a free substance in the premixed system, to obtain the drug comprising the doxorubicin; preferably, the first solvent is selected from one or more of DCM, DCC, DMAP, Py, DMSO, PBS and glacial acetic acid; preferably, the step of removing the free substance in the premixed system includes: mixing the premixed system with absolute ethyl alcohol, and precipitating the drug comprising the doxorubicin at a temperature condition lower than 10 DEG C; and more preferably, precipitating the drug comprisng the doxorubicin at a temperature condition of 0-5 DEG C.

Further, the step of loading the doxorubicin in the covalent linkage mode includes: preparing doxorubicin solution; enabling the doxorubicin solution to react with the G-exocyclic amino of the nucleic acid nanoparticle under a mediating effect of the formaldehyde, to obtain a reaction system; and purifying the reaction system, to obtain the drug comprising the doxorubicin; preferably, the reaction step includes: mixing the doxorubicin solution with a paraformaldehyde solution and the nucleic acid nanoparticle, and reacting in a dark condition, to obtain the reaction system; herein a concentration of the paraformaldehyde solution is preferably 3.7-4wt%, and the paraformaldehyde solution is preferably a solution formed by mixing paraformaldehyde and a second solvent, and the second solvent is one or more of DCM, DCC, DMAP, Py, DMSO, PBS and glacial acetic acid.

Further, the preparation method further includes a step of preparing the nucleic acid nanoparticle, the step includes: self-assembling single strands corresponding to the nucleic acid structural domain in any nucleic acid nanoparticle (without loading a bioactive substance) mentioned above, to obtain the nucleic acid structural domain; preferably, after the nucleic acid structural domain is obtained, the preparation method further includes: enabling the bioactive substance to be loaded on the nucleic acid structural domain in the physical linkage mode and/or a covalent linkage mode, to obtain the nucleic acid nanoparticle, herein the drug in the bioactive substance is a small molecular drug except the doxorubicin.

Further, in a process of loading the bioactive substance in the covalent linkage mode, the loading is performed through a solvent covalent linkage, a linker covalent linkage or a click-linkage; preferably, a third solvent used in the solvent covalent linkage is served as a linkage medium, and the third solvent is selected from one or more of paraformaldehyde, DCM, DCC, DMAP, Py, DMSO, PBS and glacial acetic acid; preferably, the linker is selected from a disulfide bond, a p-phenylazide, bromopropyne or PEG; and preferably, the click-linkage is that a bioactive substance precursor and the nucleic acid structural domain is modified by a alkynyl or a azide simultaneously, and then t linked through a click reaction.

Further, when the bioactive substance is linked with the nucleic acid structural domain in the click-linkage mode, a site, for performing the alkynyl or azide modification, of the bioactive substance precursor is selected from a 2'-hydroxyl, a carboxyl or an amino, and a site, for performing the alkynyl or azide modification, of the nucleic acid structural domain is selected from a G-exocyclic amino, a 2'-hydroxyl, an A-amino or a 2'-hydroxyl.

The nucleic acid nanoparticles provided by the disclosure are capable of, through including the above three sequences or the variant sequence thereof, not only self-assembling to form the nucleic acid structural domain, but also serving as the carrier, and linking the siRNA drug or the miRNA drug at the arbitrary 5'-end and/or 3'-end of the three strands, and in the process of forming the above nanoparticles, because of the presence of the nucleic acid structural domain, a degradation effect of the nuclease to the loaded nucleic acid drug is reduced, and delivery reliability and stability of the drug are improved.

## Brief Description of the Drawings

Drawings of the description for constituting a part of the present application are used to provide further understanding of the disclosure, exemplary embodiments of the disclosure and descriptions thereof are used to explain the disclosure, and do not constitute improper limitation to the disclosure. In the drawings:

Fig. 1 shows an electrophoresis detection result of RNA nanoparticles formed by self-assembly in Embodiment 1

of the disclosure.

Fig. 2 shows an electrophoresis detection result of DNA nanoparticles formed by self-assembly in Embodiment 1 of the disclosure.

Fig. 3 shows a 2% agarose gel electrophoresis detection result of 7 groups of short-sequence RNA nanoparticles formed by self-assembly in Embodiment 2 of the disclosure.

Fig. 4 shows a 4% agarose gel electrophoresis detection result of 7 groups of short-sequence RNA nanoparticles formed by self-assembly in Embodiment 2 of the disclosure.

Fig. 5 shows a 2% agarose gel electrophoresis detection result of 7 groups of conventional sequence RNA nanoparticles formed by self-assembly in Embodiment 3 of the disclosure.

Fig. 6 shows a 4% agarose gel electrophoresis detection result of 7 groups of conventional sequence RNA nanoparticles formed by self-assembly in Embodiment 3 of the disclosure.

Fig. 7 shows a 2% agarose gel electrophoresis detection result of 7 groups of conventional sequence DNA nanoparticles formed by self-assembly in Embodiment 4 of the disclosure.

Fig. 8 shows a 4% agarose gel electrophoresis detection result of 7 groups of conventional sequence DNA nanoparticles formed by self-assembly in Embodiment 4 of the disclosure.

Fig. 9 shows a transmission electron microscope picture of conventional sequence DNA nanoparticles D-7 formed by self-assembly in Embodiment 4 of the disclosure.

Fig. 10 shows an electrophoresis detection result of a doxorubicin loading product in Embodiment 5 of the disclosure.

Fig. 11 shows a standard curve of a doxorubicin absorbance used in a loading rate detection process in Embodiment 5 of the disclosure.

Fig. 12 shows a FACS fluorescence signal strength detection result of the different nanoparticles in Embodiment 7 of the disclosure.

Fig. 13 shows results of binding and internalization of the different nanoparticles and HepG2 cells in Embodiment 7 of the disclosure.

Fig. 14 shows an electrophoresis detection result of RNA nanoparticles, after being incubated in serum for different times, under a Coomassie Blue program in Embodiment 9 of the disclosure.

Fig. 15 shows an electrophoresis detection result of RNA nanoparticles, after being incubated in serum for different times, under a Stain Free Gel program in Embodiment 9 of the disclosure.

Fig. 16 shows a detection result of HepG2 cell proliferation of the different nanoparticles in Embodiment 10 of the disclosure.

Fig. 17 shows non-denaturing PAGE gel electrophoresis detection results of 7 groups of extension fragment deformation + core short-sequence RNA self-assembly products in Embodiment 11 of the disclosure.

Fig. 18 shows a solubility curve of RNA nanoparticles R-15 in Embodiment 11 of the disclosure.

Fig. 19 shows a solubility curve of RNA nanoparticles R-16 in Embodiment 11 of the disclosure.

Fig. 20 shows a solubility curve of RNA nanoparticles R-17 in Embodiment 11 of the disclosure.

Fig. 21 shows a solubility curve of RNA nanoparticles R-18 in Embodiment 11 of the disclosure.

Fig. 22 shows a solubility curve of RNA nanoparticles R-19 in Embodiment 11 of the disclosure.

Fig. 23 shows a solubility curve of RNA nanoparticles R-20 in Embodiment 11 of the disclosure.

Fig. 24 shows a solubility curve of RNA nanoparticles R-21 in Embodiment 11 of the disclosure.

Fig. 25 shows non-denaturing PAGE gel electrophoresis detection results of 7 groups of extension fragment deformation + core short-sequence DNA self-assembly products in Embodiment 12 of the disclosure.

Fig. 26 shows a solubility curve of DNA nanoparticles D-8 in Embodiment 12 of the disclosure.

Fig. 27 shows a solubility curve of DNA nanoparticles D-9 in Embodiment 12 of the disclosure.

Fig. 28 shows a solubility curve of DNA nanoparticles D-10 in Embodiment 12 of the disclosure.

Fig. 29 shows a solubility curve of DNA nanoparticles D-11 in Embodiment 12 of the disclosure.

Fig. 30 shows a solubility curve of DNA nanoparticles D-12 in Embodiment 12 of the disclosure.

Fig. 31 shows a solubility curve of DNA nanoparticles D-13 in Embodiment 12 of the disclosure.

Fig. 32 shows a solubility curve of DNA nanoparticles D-14 in Embodiment 12 of the disclosure.

Fig. 33 shows an electrophoresis detection result of RNA nanoparticles R-15 after being incubated in serum for different times in Embodiment 13 of the disclosure.

Fig. 34 shows an electrophoresis detection result of RNA nanoparticles R-16 after being incubated in serum for different times in Embodiment 13 of the disclosure.

Fig. 35 shows an electrophoresis detection result of RNA nanoparticles R-17 after being incubated in serum for different times in Embodiment 13 of the disclosure.

Fig. 36 shows an electrophoresis detection result of RNA nanoparticles R-18 after being incubated in serum for different times in Embodiment 13 of the disclosure.

Fig. 37 shows an electrophoresis detection result of RNA nanoparticles R-19 after being incubated in serum for different times in Embodiment 13 of the disclosure.

Fig. 38 shows an electrophoresis detection result of RNA nanoparticles R-20 after being incubated in serum for different times in Embodiment 13 of the disclosure.

Fig. 39 shows an electrophoresis detection result of RNA nanoparticles R-21 after being incubated in serum for different times in Embodiment 13 of the disclosure.

Fig. 40 shows an electrophoresis detection result of DNA nanoparticles D-8 after being incubated in serum for different times in Embodiment 14 of the disclosure.

Fig. 41 shows an electrophoresis detection result of DNA nanoparticles D-9 after being incubated in serum for different times in Embodiment 14 of the disclosure.

Fig. 42 shows an electrophoresis detection result of DNA nanoparticles D-10 after being incubated in serum for different times in Embodiment 14 of the disclosure.

Fig. 43 shows an electrophoresis detection result of DNA nanoparticles D-11 after being incubated in serum for different times in Embodiment 14 of the disclosure.

Fig. 44 shows an electrophoresis detection result of DNA nanoparticles D-12 after being incubated in serum for different times in Embodiment 14 of the disclosure.

Fig. 45 shows an electrophoresis detection result of DNA nanoparticles D-13 after being incubated in serum for different times in Embodiment 14 of the disclosure.

Fig. 46 shows an electrophoresis detection result of DNA nanoparticles D-14 after being incubated in serum for different times in Embodiment 14 of the disclosure.

Fig. 47a, Fig. 47b, Fig. 47c, Fig. 47d, Fig. 47e, Fig. 47f, Fig. 47g, and Fig. 47h respectively show cell survival rate curves corresponding to DMSO and original drug doxorubicin, D-8 and D-8-doxorubicin, D-9 and D-9-doxorubicin, D-10 and D-10-doxorubicin, D-11 and D-11-doxorubicin, D-12 and D-12-doxorubicin, D-13 and D-13-doxorubicin, and D-14 and D-14-doxorubicin.

Fig. 48 shows a standard curve of a daunorubicin absorbance used in a loading rate detection process of Embodiment 18.

## Detailed Description of the Embodiments

It is to be noted that embodiments in the present application and features in the embodiments may be combined with each other under a condition without conflicting. The disclosure is described in detail below with reference to the embodiments.

As mentioned in the background, although there are a variety of drug carriers for improving drug delivery efficiency in the prior art, it is still difficult to solve the problem of the limited clinical applications of the drugs. In order to improve this situation, the inventor of the present application researches on all existing materials which may be used as the drug carriers, and deeply investigates and analyzes the various carriers in aspects, such as cell/tissue targeting of the carrier, stability in a delivery process, activity and efficiency of entering target cells, drug release ability after reaching the target cells and toxicity to cells, it is discovered that an emerging nanostructure formed by self-assembly of DNA and/or RNA molecules, such as a self-assembled DNA dendrimersmay significantly prevent DNA from degradation by a nuclease, and have a very important application value in the fields of gene therapy and biomedicine.

Through analyzing the existing reported nanoparticles formed by the self-assembly of DNA and RNA, it is discovered that, compared with the relatively rigid DNA nanoparticles, the RNA nanoparticles have larger flexibility and stronger tension because there are a large number of stem-loop structures within or between molecules, so it has more advantages in an aspect as a candidate drug carrier. However, the RNA nanoparticles in a natural state are relatively poor in stability, and the existing improvements based on application aspects of the RNA nanocarriers are mostly focused on improving the stability and reliability thereof. Although research results at present provide the possibility of loading the drug to a certain extent, they are more focused on researching the possibility and effectiveness of loading nucleic acid drugs, especially a siRNA drug or a miRNA drug and the like. There are few reports at present on whether non-nucleic acid drugs are equally effective. In addition, the existing self-assembled nanoparticles, especially the self-assembled nanoparticles used as carriers, are self-assembled with a RNA strand, and a very few is self-assembled in a mode of a RNA strand and DNA strand combination, but no self-assembly is achieved with a DNA strand only.

In order to provide a new RNA nanoparticle carrier with good reliability and self-assembly, the existing RNA nanoparticles are compared and improved by the applicant, a series of new RNA nanoparticles are developed, and in view of improving applicability and reducing cost, the self-assembly is further tried to be performed with the DNA strand only, it is unexpectedly discovered that after being changed, these DNA single strands may not only self-assemble into the DNA nanoparticles, but also have the same excellent performance as the RNA nanoparticles. In addition, the self-assembly of the DNA nanoparticles also has advantages of low price and easy operation. After being verified by experiments, both the RNA nanoparticles and the DNA nanoparticles improved by the inventor may be loaded with various drugs, and may exist stably in serum; and further verified by the experiments, it may carry the drugs into the cells, and the carrier alone is non-toxic to the cells. However, the drug-carried carrier may have alleviating and therapeutic effects

to corresponding diseases.

On the basis of the above research result, the applicant provides a technical scheme of the present application. In a typical implementation mode, a nucleic acid nanoparticle is provided, the nucleic acid nanoparticle has a nucleic acid structural domain, the nucleic acid structural domain includes a sequence a, a sequence b and a sequence c. The sequence a includes a sequence a1 or a sequence obtained by insertion, deletion or substitution of at least one base in the sequence a1, the sequence b includes a sequence b1 or a sequence obtained by insertion, deletion or substitution of at least one base in the sequence b1, and the sequence c includes a sequence c1 or a sequence obtained by insertion, deletion or substitution of at least one base in the sequence c1, herein the sequence a1 is SEQ ID NO:1:5'-CCAGCGUUCC-3'or SEQ ID NO:2:5'-CCAGCGTTCC-3'; the sequence b1 is SEQ ID NO:3:5'-GGUUCGCCG-3'or SEQ ID NO:4:5'-GGTTCGCCG-3'; and the sequence c1 is SEQ ID NO:5:5'-CGGCCAUAGCGG-3'or SEQ ID NO:6:5'-CGGCCATAGCGG-3'.

The above nucleic acid nanoparticles are capable of, through including the above three sequences or the variant sequence thereof, not only self-assembling to form the nucleic acid structural domain, but also serving as the carrier, and linking the siRNA drug or the miRNA drug at the arbitrary 5'-end and/or 3'-end of the three strands, and in the process of forming the above nanoparticles, because of the presence of the nucleic acid structural domain, a degradation effect of the nuclease to the loaded nucleic acid drug is reduced, and delivery reliability and stability of the drug are improved.

The above self-assembly refers to a technology that basic structural units spontaneously form an ordered structure. In a process of the self-assembly, the basic structural units spontaneously organize or aggregate into a stable structure with a certain regular geometric appearance under an interaction based on a non-covalent bond. The self-assembly process is not a simple superposition of weak interaction forces (herein the "weak interaction forces" refer to a hydrogen bond, a Van der Waals force, an electrostatic force, a hydrophobic action force and the like) between a large number of atoms, ions or molecules, but a tight and orderly whole formed by simultaneously spontaneously parallel connection and aggregation between a plurality of individuals, and is an overall complicated synergistic effect.

The production of the self-assembly requires two conditions: self-assembly power and guiding effect. The self-assembly power refers to the synergistic effect of the weak interaction forces between the molecules, and it provides energy for molecular self-assembly. The guiding effect of the self-assembly refers to complementary of the molecules in space, namely the production of the self-assembly needs to meet requirements of molecular rearrangement in direction and size of the space.

A DNA nanotechnology is a bottom-up molecular self-assembly mode, a stable structure is spontaneously formed using a molecular structure as a starting point on the basis of physical and chemical properties of the nucleic acid nanoparticles, and a strict nucleic acid base pairing principle is followed. Multiple DNA fragments are linked together in vitro in a correct sequence, a sub-assembly structure is established through the base complementary pairing principle, and finally a complicated multi-level structure is formed. Unlike the DNA, the structure of the RNA may exceed limitation of double-helix. The RNA may form a series of different base pairs, and at least two hydrogen bonds are formed between the base pairs. The different bases may be divided into two types, including a standard Waston-Crick base pair type and a non-Waston-Crick base pair type, so that the RNA forms a large number and multiple types of cyclic structure modules, and these modules are basic units for forming a folded RNA three-level structure. The RNA nanotechnology may make use of these natural existing 3D modules and predictable interactions thereof, herein, many RNA structures with biological activity may have an atomic-level resolution, such as a ribosome, various ribozymes and a natural RNA aptamer existing in a riboswitch. A superior feature of the RNA nanotechnology is that a structure which is comparable in size and complexity to a natural RNA substance may be designed. A unique assembly property of the RNA in a natural RNA complex may also be utilized.

The above nucleic acid nanoparticles of the present application include three sequences shown in sequences SEQ ID NO:1, SEQ ID NO:3 and SEQ ID NO:5 or variant sequences thereof, or include three sequences shown in sequences SEQ ID NO:2, SEQ ID NO:4 and SEQ ID NO:6 or variant sequences thereof, and all of the sequences are subject to an ability to form the nucleic acid nanoparticles through the self-assembly, the specific variant sequence may be obtained on the basis of the sequences of the SEQ ID NO:1, the SEQ ID NO:2, the SEQ ID NO:3, the SEQ ID NO:4, the SEQ ID NO:5 and the SEQ ID NO:6 by rationally selecting a variant site and a variant type thereof, or obtained by extending a suitable fragment.

The nanoparticles formed by the self-assembly of the SEQ ID NO:1, the SEQ ID NO:3 and the SEQ ID NO:5 are RNA nanoparticles, and the nanoparticles formed by the self-assembly of the SEQ ID NO:2, the SEQ ID NO:4 and the SEQ ID NO:6 are DNA nanoparticles. In a preferred embodiment, the above nucleic acid nanoparticles are the RNA nanoparticles, and at least one of the sequence a, the sequence b and the sequence c includes a sequence obtained by insertion, deletion or substitution of at least one base, a specific position and a base type of the variant sequence in the RNA nanoparticles may be modified into the nanoparticles of improving a drug loading amount or improving stability according to the needs under a precondition of achieving the self-assembly.

In order to make the prepared nucleic acid nanoparticles have the relatively higher stability, when the base

insertion, deletion or substitution is performed on the sequences shown in the above SEQ ID NO:1/2, SEQ ID NO:3/4 and SEQ ID NO:5/6, it may be performed on bases in some specific positions of the above sequences. On one hand, the variant sequence is the same as the original sequence, and may be self-assembled into the nanoparticles, and on the other hand, the variation retains at least 50%, 55%, 60%, 65%, 70%, 75%, 80%, 85% 90% or 95% of identity with the original sequence, so that it has the same drug-loading features and similar stability as the nanoparticles formed by the self-assembly of the above sequences, it may not only load and deliver the nucleic acid drug, but also apply to the loading and delivery of other bioactive substances, such as a chemical drug, thus it has relative universality.

In a preferred embodiment, the above base insertion, deletion or substitution is occurred: (1) at 1, 2, 4 or 5-th base starting from a 5 '-end of the sequence a shown in the SEQ ID NO:1 or 2; and/or (2) between 8-th and 10-th bases starting from the 5 '-end of the sequence a shown in the SEQ ID NO:1 or 2; and/or (3) between 1-th and 3-th bases starting from a 5 '-end of the sequence b shown in the SEQ ID NO:3 or 4; and/or (4) between 6-th and 9-th bases starting from the 5 '-end of the sequence b shown in the SEQ ID NO:3 or 4; and/or (5) between 1-th and 4-th bases starting from a 5 '-end of the sequence c shown in the SEQ ID NO:5 or 6; and/or (6) between 9-th and 12-th bases starting from the 5 '-end of the sequence c shown in the SEQ ID NO:5 or 6.

In the above preferred embodiment, the defined base position in which the variation happens is a non-classical Watson-Crick paired base position or a bulged unpaired base position in the nanostructure formed by the sequences shown in the SEQ ID NO:1, SEQ ID NO:2, SEQ ID NO:3, SEQ ID NO:4, SEQ ID NO:5 and SEQ ID NO:6, thus the formation of these bulges or loop structures is not affected, thereby the flexibility and tension of the nanostructure formed by the above sequences are maintained, and it is helpful to maintain the stability thereof as a carrier.

In order to further improve the stability of the above nucleic acid nanoparticles, in a preferred embodiment, the sequence a, the sequence b and the sequence c are self-assembled into a structure shown in Formula (1):

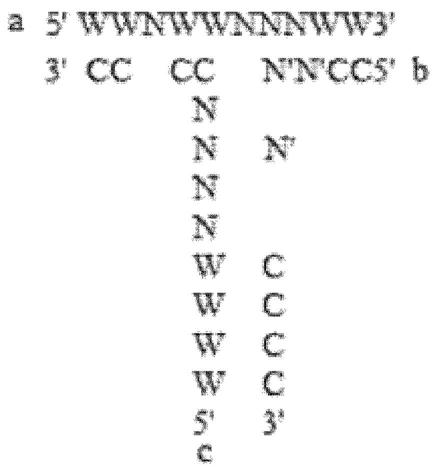


Formula (1)

Herein, W-C represents a Watson-Crick pairing, N and N' represent a non-Watson-Crick pairing, the W-C in any one position is independently selected from C-G or G-C; in the sequence a, the first N from the 5'-end is A, the second N is G, the third N is U or T, and the fourth N is any one of U, T, A, C or G; in the sequence b, the first N' from the 5'-end is any one of U, T, A, C or G, the second N' is U or T, and the third N' is C; and in the sequence c, a sequence NNNN along a direction from the 5'-end to the 3'-end is CAUA or CATA.

In the above preferred embodiment, the sequences a, b and c are self-assembled to form the nucleic acid domain shown in Formula (1), herein, except the non-Watson-Crick paired bases defined by N and N', the bases in the rest positions all form classical Watson-Crick pairing, and the bases of the above Watson-Crick pairing all choose G-C or C-G base pairs. Because an action force of hydrogen bonds between the G-C or C-G base pairs is greater than an action force of hydrogen bonds between the A-U/T or U/T-A base pairs, the nucleic acid nanostructure is more stable. Rather than the bulges or loop structures formed by the non-Watson-Crick paired bases, the greater tension is brought to the nucleic acid nanocarrier, so that it has the stronger adaptation to a micro-environmental change, thus the stability of the nucleic acid nanoparticles is higher.

In the nanoparticles in the above structure of Formula (1), the specific sequence formation of the sequence a, the sequence b and the sequence c is not specially limited, as long as the above structure may be formed. In view of the self-assembly of the nucleic acid sequence, in order to further improve the efficiency of the self-assembly of the above three sequences into the nanoparticles in the above structure of Formula (1), when the Watson-Crick paired bases are selected, the selection of the bases in the different positions is best to follow the following principles: (1) the sequence a, the sequence b and the sequence c, when as a single sequence, it is not complementary-paired by itself

to form a secondary structure; and (2) the sequence a, the sequence b and the sequence c, one end of arbitrary two sequences is complementary-paired to form a double-strand, and the other end is not complementary-paired, to form a Y-type or T-type structure. The above principle of the base selection is to most efficiently enable two ends of any one strand to be respectively complementary-paired with two ends of the other two strands, thereby the self-assembly efficiency is improved. Certainly, in addition to the Y-type or T-type structure, it may also be other deformation modes such as a quadrangle rather than a trigeminal shape, as long as it meets the principle that one end of arbitrary two sequences is complementary-paired to form the double-strand, and the other end is not complementary-paired.

In the nanoparticles in the above structure of Formula (1), in the non-Watson-Crick paired bases, the fourth N starting from the 5'-end in the sequence a and the first N' starting from the 5'-end paired in the sequence b may be non-Watson-Crick paired U-U, or may be T, A, C or G improved for following the Watson-Crick pairing principle. A binding force between the strands is relatively improved by the Watson-Crick pairing, the stability is improved, and the nanoparticles are endowed with the larger softness and flexibility by the non-Watson-Crick pairing, in the face of the micro-environmental change, it is also helpful to improve the stability of the nanoparticles.

In a preferred embodiment, the sequence a, the sequence b and the sequence c are any one of the following groups: (1) sequence a (SEQ ID NO:7): 5'-GGAGCGUUGG-3', sequence b (SEQ ID NO:8): 5'-CCUUCGCCG-3', sequence c (SEQ ID NO:9): 5'-CGGCCAUAGCCC-3'; (2) sequence a (SEQ ID NO:10): 5'-GCAGCGUUCG-3', sequence b (SEQ ID NO:11): 5'-CGUUCGCCG-3', sequence c (SEQ ID NO:12): 5'-CGGCCAUAGCGC-3'; (3) sequence a (SEQ ID NO:13): 5'-CGAGCGUUGC-3', sequence b (SEQ ID NO:14): 5'-GCUUCGCCG-3', sequence c (SEQ ID NO:15): 5'-CGGCCAUAGCCG-3'; (4) sequence a (SEQ ID NO:16): 5'-GGAGCGUUGG-3', sequence b (SEQ ID NO:17): 5'-CCUUCGGGG-3', sequence c (SEQ ID NO:18): 5'-CCCCCAUAGCCC-3'; (5) sequence a (SEQ ID NO:19): 5'-GCAGCGUUCG-3', sequence b (SEQ ID NO:20): 5'-CGUUCGGCG-3', sequence c (SEQ ID NO:21): 5'-CGCCCAUAGCGC-3'; (6) sequence a (SEQ ID NO:22): 5'-GCAGCGUUCG-3', sequence b (SEQ ID NO:23): 5'-CGUUCGGCC-3', sequence c (SEQ ID NO:24): 5'-GGCCCAUAGCGC-3'; (7) sequence a (SEQ ID NO:25): 5'-CGAGCGUUGC-3', sequence b (SEQ ID NO:26): 5'-GCUUCGGCG-3', sequence c (SEQ ID NO:27): 5'-CGCCCAUAGCCG-3'; (8) sequence a (SEQ ID NO:28): 5'-GGAGCGTTGG-3', sequence b (SEQ ID NO:29): 5'-CCTTCGCCG-3', sequence c (SEQ ID NO:30): 5'-CGGCCATAGCCC-3'; (9) sequence a (SEQ ID NO:31): 5'-GCAGCGTTCG-3', sequence b (SEQ ID NO:32): 5'-CGTTCGCCG-3', sequence c (SEQ ID NO:33): 5'-CGGCCATAGCGC-3'; (10) sequence a (SEQ ID NO:34): 5'-CGAGCGTTGC-3', sequence b (SEQ ID NO:35): 5'-GCTTCGCCG-3', sequence c (SEQ ID NO:36): 5'-CGGCCATAGCCG-3'; (11) sequence a (SEQ ID NO:37): 5'-GGAGCGTTGG-3', sequence b (SEQ ID NO:38): 5'-CCTTCGGGG-3', sequence c (SEQ ID NO:39): 5'-CCCCCATAGCCC-3'; (12) sequence a (SEQ ID NO:40): 5'-GCAGCGTTCG-3', sequence b (SEQ ID NO:41): 5'-CGTTCGGCG-3', sequence c (SEQ ID NO:42): 5'-CGCCCATAGCGC-3'; (13) sequence a (SEQ ID NO:43): 5'-GCAGCGTTCG-3', sequence b (SEQ ID NO:44): 5'-CGTTCGGCC-3', sequence c (SEQ ID NO:45): 5'-GGCCCATAGCGC-3'; and (14) sequence a (SEQ ID NO:46): 5'-CGAGCGTTGC-3', sequence b (SEQ ID NO:46): 5'-GCTTCGGCG-3', sequence c (SEQ ID NO:48): 5'-CGCCCATAGCCG-3'.

The nucleic acid nanoparticles formed by the self-assembly of fourteen groups of the above sequences not only have the higher stability, but also have the higher self-assembly efficiency.

The nucleic acid nanoparticles mentioned above may not only be self-assembled for forming, but also have the ability to carry or load the drugs. According to the different positions of G-C or C-G base pairs in the above nucleic acid nanoparticles and differences of types or natures of the drugs to be loaded, the amounts of the loaded drugs are also different. Similarly, the nucleic acid drugs may be loaded at the 5'-end and/or 3'-end of any one sequence in the sequence a, the sequence b and the sequence c by stand extension.

According to a size of a molecular weight of the loaded drug, or a difference of a steric hindrance in the structure during loading, in order to enable the above nucleic acid drug to be loaded on the bioactive substance with a relatively large molecular weight, in a preferred embodiment, in the above nucleic acid structural domain, a first extension fragment is further included, the first extension fragment is an extension fragment of Watson-Crick pairing, and the first extension fragment is positioned at the 5'-end and/or the 3'-end of any one sequence of the sequence a, the sequence b or the sequence c. a certain matching relation is required between the carrier and the loaded substance, when a molecular weight of the carrier is too small and a molecular weight of the loaded substance is too large, in view of mechanics, carrying or delivering capacity of the carrier to the loaded substance is relatively reduced. Therefore, based on the above nucleic acid nanostructure, through adding the first extension fragment at the 5'-end and/or 3'-end of any one sequence of the sequence a, the sequence b and the sequence c, the carrier matched with the size of the loaded substance may be acquired.

A specific length of the above first extension fragment may be determined according to the size of the substance to be loaded. In a preferred embodiment, the first extension fragment is selected from any one of the following groups: (1): a-strand 5'-end: 5'-CCCA-3', c-strand 3'-end: 5'-UGGG-3'; (2): a-strand 3'-end: 5'-GGG-3', b-strand 5'-end: 5'-CCC-3'; (3): b-strand 3'-end: 5'-CCA-3', c-strand 5'-end: 5'-UGG-3'; (4): a-strand 5'-end: 5'-CCCG-3', c-strand 3'-end: 5'-CGGG-3'; (5): a-strand 5'-end: 5'-CCCC-3', c-strand 3'-end: 5'-GGGG-3'; (6): b-strand 3'-end: 5'-CCC-3', c-strand 5'-end: 5'-GGG-3'; (7): b-strand 3'-end: 5'-CCG-3', c-strand 5'-end: 5'-CGG-3'; (8): a-strand 5'-end: 5'-CCCA-3', c-strand

3'-end: 5'-TGGG-3'; (9): b-strand 3'-end: 5'-CCA-3', c-strand 5'-end: 5'-TGG-3'; (10): a-strand 5'-end: 5'-GCGGCGAGCGGCGA-3'(SEQ ID NO:162), c-strand 3'-end: 5'-UCGCCGCUCGCCGC-3'(SEQ ID NO:163); (11): a-strand 3'-end: 5'-GGCCGGAGGCCGG-3'(SEQ ID NO:164), b-strand 5'-end: 5'-CCGGCCUCCGGCC-3'(SEQ ID NO:165); (12): b-strand 3'-end: 5'-CCAGCCGCC-3'(SEQ ID NO:166), c-strand 5'-end: 5'-GGCGGCAGG-3'(SEQ ID NO:167); (13): a-strand 5'-end: 5'-GCGGCGAGCGGCGA-3'(SEQ ID NO:168), c-strand 3'-end: 5'-TCGCCGCTCGCCGC-3'(SEQ ID NO:169); and (14): a-strand 3'-end: 5'-GGCCGGAGGCCGG-3'(SEQ ID NO:170), b-strand 5'-end: 5'-CCGGCCTCCGGCC-3'(SEQ ID NO:171).

The above first extension fragment not only increases a length of any one or more of the three sequences for forming the nucleic acid nanostructure, but also the first extension fragment formed by the GC base further improves the stability of the formed nanoparticles. In addition, the first extension fragment formed by the above sequences also enables the sequence a, the sequence b and the sequence c to maintain the higher self-assembly activity and efficiency.

In view of the size of the formed nucleic acid nanoparticles and the stability thereof while delivered as a drug delivery carrier in vivo, it is necessary not to be filtered out by kidneys before reaching the target cells while the drugs may be delivered. In a preferred embodiment, the nucleic acid structural domain further includes a second extension fragment, the second extension fragment is positioned at the 5'-end and/or the 3'-end of any one sequence of the sequence a, the sequence b, or the sequence c, and the second extension fragment is an extension fragment of Watson-Crick pairing; more preferably, the second extension fragment is an extension sequence of a CG base pair; and further preferably, the second extension fragment is an extension sequence of 1-10 CG base pairs. The second extension fragment is the extension fragment further added on the basis of the first extension fragment.

In a preferred embodiment, the above nucleic acid structural domain further includes at least one group of the following second extension fragments: first group: a-strand 5'-end: 5'-CGCGCG-3', c-strand 3'-end: 5'-CGCGCG-3'; second group: a-strand 3'-end: 5'-CGCCGC-3', b-strand 5'-end: 5'-GCGGCG-3'; and third group: b-strand 3'-end: 5'-GGCGGC-3', c-strand 5'-end: 5'-GCCGCC-3'. Such a second extension fragment enables the nanoparticles not to have immunogenicity, and there is no such a situation that the secondary structure in each strand is folded and linked by itself.

It is to be noted that the above first extension fragment and/or the second extension fragment may also be separated by an unpaired base pair.

In order to enable the above nucleic acid nanoparticles to load the bioactive substance with the larger molecular weight, increase the drug loading amount and maintain the necessary stability, in a preferred embodiment, the second extension fragment is an extension sequence containing both CG base pair and AT/AU base pair, and preferably the second extension fragment is an extension sequence of 2-50 base pairs. Here, "/" in the "AT/AU base" is a relation of or, specifically, the second extension fragment is an extension sequence containing both CG base pair and AT base pair, or the second extension fragment is an extension sequence containing both CG base pair and AU base pair.

More specifically, the sequences a, b and c after the above second extension fragment is added may be the following sequences respectively:

The sequence a is (SEQ ID NO:49):

5'-CGCGCGAAAAAACGCGCGAAAAAACGCGCGCCCACCAGCGMMCCGGG
CGCGCGAAAAAACGCGCGAAAAAACGCGCG-3'.

The sequence b is (SEQ ID NO:50):

5'-CGCGCGMMMMMMCGCGCGMMMMMMCGCGCGCCCGGMMCGCCGCC
AGCCGCCMMMMMMGCCGCCMMMMMMGCCGCC-3'.

The sequence c is (SEQ ID NO:51):

5'-GGCGGCAAAAAAGGCGGCAAAAAAGGCGGCAGGCGGCAMAGCGGMGG
GCGCGCGMMMMMMCGCGCGMMMMMMCGCGCG-3'.

M in the above sequence a, sequence b and sequence c is U or T, when the M is T, a synthetic cost of the above sequences is greatly reduced.

In practical applications, specific setting positions of the extension sequences of the above CG base pair and AT/AU base pair may be rationally adjusted according to actual needs. In a more preferred embodiment, the second extension fragment is an extension sequence in which sequences of 2-8 continuous CG base pairs and sequences of 2-8 continuous AT/AU base pairs are alternately arranged; or the second extension fragment is an extension sequence in which a sequence of 1 CG base pair and a sequence of 1 AT/AU base pair are alternately arranged.

Specifically, positions of the extension fragment CGCGCG and the extension fragment CGCCGC in the sequence a as shown in the above SEQ ID NO:49 are interchanged with a position of the extension fragment AAAAAA, positions of the extension fragment GCGGCG and the extension fragment GGCGGC in the sequence b shown in the above SEQ ID NO:50 are interchanged with a position of the extension fragment TTTTTT, the extension fragment GCCGCC in the sequence c shown in the above SEQ ID NO:51 is interchanged with the extension fragment AAAAAA, and the extension fragment CGCCGC is interchanged with the extension fragment TTTTTT at the same time. The nucleic acid nanoparticles formed by the self-assembly of the above sequences are suitable for loading chemical drugs in indole molecular structures (indole drug molecules are preferably linked with base A).

In the past few years, three major challenges of the RNA as a widely used construction material includes: 1) sensitivity to RNA enzymatic degradation; 2) sensitivity to dissociation after systemic injection; and 3) toxicity and adverse immune response. At present, the three challenges are overcome to a large extent already: 1) 2'-fluoro(2'-F) or 2'-O-methyl(2'-OMe) modification of a ribose-OH group may make the RNA chemically stable in serum; 2) some natural existing linking sequence motifs are thermodynamically stable, and may keep the overall RNA nanoparticles to be integral at an ultra-low concentration; and 3) the immunogenicity of the RNA nanoparticles is sequence and shape-dependent, and may be adjusted, so that the RNA nanoparticles stimulate generation of inflammatory cytokines, or the RNA nanoparticles have non-immunogenicity and non-toxicity when administered by repeated intravenous injection of 30 mg/kg.

Therefore, in order to further reduce the sensitivity of the above nucleic acid nanoparticles to the RNA enzymatic degradation, and improve the stability in the delivery process, in a preferred embodiment, a base, a ribose and a phosphate in the sequence a, the sequence b and the sequence c have at least one modifiable site, and any one of the modifiable sites is modified by any one of the following modification adaptors: -F, a methyl, an amino, a disulfide, a carbonyl, a carboxyl, a sulfhydryl and a formyl; and preferably, the base C or U in the sequence a, the sequence b and the sequence c has 2'-F modification. While the modification adaptor is the sulfhydryl, it belongs to a thio modification, modification degree is weakr, and a cost is low.

The substance which may be loaded on the above nucleic acid nanoparticles provided in the present application as a carrier may be any substances with biological activity. Therefore, in a preferred embodiment, the above nucleic acid nanoparticles further include a bioactive substance, and the bioactive substance is linked with the nucleic acid structural domain.

In order to improve loading efficiency and delivering efficiency of the nucleic acid nanoparticles to the loaded bioactive substance, a relative molecular weight of the nucleic acid structural domain and a total relative molecular weight of the bioactive substance should preferably have a certain matching relation. In a preferred embodiment, a ratio of the relative molecular weight of the nucleic acid structural domain and the total relative molecular weight of the bioactive substance is ≥1:1; and preferably, the bioactive substance is one or more of a target head, a fluorescein, an interfering nucleic acid siRNA, a miRNA, a ribozyme, a riboswitch, an aptamer, a RNA antibody, a drug (usually explained as a small molecular drug, namely a chemical synthetic drug), a protein, a polypeptide, a flavonoid, a glucose, a natural salicylic acid, a monoclonal antibody, a vitamin, a phenol and a lecithin.

According to the different types of the bioactive substances loaded specifically, performance optimization effects thereof on the nucleic acid nanoparticles of the present application are not the same. For example, when the bioactive substance is a biotin or a folic acid, the function thereof is to make the nucleic acid nanoparticles have a targeting property, for example, specifically targeted to cancer cells. While the bioactive substance is the fluorescein, a function thereof is to make the nucleic acid nanoparticles have a luminous tracking effect. When the bioactive substance is some siRNA, miRNA, drug (usually explained as the small molecular drug), protein, peptide or RNA antibody, according to the different biological functions, the nucleic acid nanoparticles may be made into a new product with a specific therapeutic effect, such as a drug with more excellent performance. In addition, according to the different types of the bioactive substances loaded specifically, the DNA nanoparticles and the RNA nanoparticles are specifically preferably used, and may be rationally selected according to the actual needs. For example, while the bioactive substance is the drug, the DNA nanoparticles and the RNA nanoparticles are preferably used for loading, and there is no special requirement for a length of the single strand of the nanoparticles formed by assembly.

In a preferred embodiment, the bioactive substance is the target head, the fluorescein and the miRNA, herein, the target head is positioned on any one sequence of the sequences a, b and c, preferably the 5'-end or the 3'-end of any one sequence of the sequences a, b and c, or inserted between GC bonds of the nucleic acid structural domain, the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c; and preferably, the target head is the folic acid or the biotin, the fluorescein is any one or more of FAM, CY5 and CY3,

and the above anti-miRNA is anti-miR-21.

The above target head may be linked on any one sequence of the sequences a, b and c in a mode of linker covalent linkage, the available linker is selected from a disulfide bond, a p-phenylazide, bromopropyne or a PEG. Here, the "on any one sequence" refers to on a base in any one position of any one sequence of the sequences a, b and c, and it is more convenient and widely used to be linked at the 5'-end or 3'-end. Folic acid modification may be in a physical insertion mode linkage or a physical insertion + covalent linkage.

The above fluorescein may be a commonly used fluorescein, and preferably any one of more of FAM, CY5 and CY3.

The above miRNA may be a miRNA with a cancer suppression effect, or may be an anti-miRNA which may suppress a corresponding disease, and it may be rationally selected in practical applications according to medical needs. The above anti-miRNA may be synthesized at any one or more positions of the 3'-end of the above sequence a, the 5'-end and 3'-end of the sequence c. When the anti-miRNA is synthesized in the above three positions, the anti-miRNA has a relatively stronger suppression effect on the corresponding miRNA.

It is anti-miR-21 preferably, the miR-21 participates in initiation and progression of multiple types of cancers, and is a main oncogene for invasion and metastasis. The anti-miR-21 may effectively regulate a wide range of target genes at the same time, and is beneficial to solve a problem of cancer heterogeneity. Therefore, in the above preferred nucleic acid nanoparticles, the target head, such as the folic acid or the biotin, may be specifically targeted to the cancer cells, and after being linked and internalized with the cancer cells, the anti-miR-21 is complemented with a miR-21 base in very high affinity and specificity, thereby the expression of the oncogenic miR-21 is effectively reduced. Therefore, according to the actual needs, the above anti-miR-21 may be synthesized at any one or more positions of the 3'-end of the above sequence a, the 5'-end and the 3'-end of the sequence c. When the anti-miR-21 is synthesized in the above three positions, the anti-miR-21 has a relatively stronger suppression effect on the miR-21.

When the above bioactive substance to be loaded is a drug, according to types of diseases which may be treated by the different drugs, the drugs include but not limited to drugs for treating liver cancer, gastric cancer, lung cancer, breast cancer, head and neck cancer, uterine cancer, ovarian cancer, melanoma, leukemia, Alzheimer's disease, ankylosing spondylitis, malignant lymphoma, bronchial cancer, rheumatoid arthritis, HBV hepatitis B, multiple myeloma, pancreatic cancer, non-small cell lung cancer, prostate cancer, nasopharyngeal cancer, esophageal cancer, oral cancer, lupus erythematosus; and preferably, the head and neck cancer is brain cancer, neuroblastoma or glioblastoma.

When the above bioactive substance to be loaded is the drug, according to the differences of molecular structures of the drugs or the differences of characteristic groups which it has, the drug includes but not limited to a drug containing any one or more of the following groups: an amino group, a hydroxyl group, a carboxyl group, a mercapto group, a benzene ring group and an acetamido group.

In a preferred embodiment, the above protein is one or more of antibodies or aptamers of superoxide dismutase (SOD), survivin, human telomerase reverse transcriptase (hTERT), epidermal growth factor receptor (EGFR) and prostate-specific membrane antigen (PSMA); the above vitamin is L-Vc and/or esterified Vc; and the above phenol is a tea polyphenol and/or a grape polyphenol.

According to the different loaded bioactive substances, a suitable linkage mode may be selected for linkage with the above nucleic acid nanocarrier. In a preferred embodiment, the above bioactive substance is linked with the nucleic acid structural domain in any one of the following modes: mode I: physical insertion; and mode II: covalent linkage.

It is to be noted that the above classification does not mean that there is only one linkage mode to link a certain bioactive substance with the nucleic acid nanocarrier. However, some bioactive substances may be linked with the nucleic acid nanocarrier in the physical insertion mode, or may be linked with the nucleic acid nanocarrier in the physical insertion and covalent linkage modes, or may be linked using the click-linkage mode at the same time. However, for a certain specific bioactive substance, there may be only one linkage mode, or there may be multiple linkage modes, but it may be that the efficiency of certain linkage has an advantageous practical value.

In the above linkage modes, when the different drugs are linked with the nucleic acid structural domain in the physical insertion mode, insertion linkage sites and numbers are also different slightly. For example, when anthracycline and acridine drugs are inserted, they are usually inserted between the GC base pairs, the preferred number of the insertion sites is based on the different numbers of the GC base pairs in the nucleic acid structural domain, and the insertion is performed in a ratio of 1 to 100:1. When a naphthylamide drug is inserted, it is usually inserted between the AA base pairs, the preferred number of the insertion sites is based on the different numbers of the AA base pairs in the nucleic acid structural domain, and pyridocarbazoles are inserted according to the different numbers of the AA base pairs in a ratio of 1 to 200:1.

While the drug is linked with the nucleic acid structural domain in two modes of the physical insertion and the covalent linkage at the same time, and the anthracycline and acridine drugs are inserted, they are usually inserted between the GC base pairs, the preferred number of the insertion sites is based on the different numbers of the GC base pairs in the nucleic acid structural domain, and the insertion is performed in the ratio of 1 to 100:1. When the naphthylamide drug is inserted, it is usually inserted between the AA base pairs, the preferred number of the insertion

sites is based on the different numbers of the AA base pairs in the nucleic acid structural domain, and the pyridocarbazoles are inserted according to the different numbers of the AA base pairs in the ratio of 1 to 200:1.

Specifically, according to the different types of the bioactive substances, the lengths of the sequences a, b and c for forming the nucleic acid structural domain in the nucleic acid nanoparticles and the number of GC complementary base pairs thereof, the physical insertion may be performed by rationally selecting a molar ratio of the bioactive substance and the nucleic acid structural domain.

In a preferred embodiment, when the bioactive substance is linked with the nucleic acid structural domain in the physical insertion mode, the physical insertion is performed on the bioactive substance and the nucleic acid structural domain according to a molar ratio of 1 to 200:1. The linkage mode is suitable for the anthracycline and acridine drugs. The physical insertion performed within the ratio range may not only meet a loading requirement, but also meet a requirement for pharmaceutical efficacy.

In a preferred embodiment, when the bioactive substance is linked with the nucleic acid structural domain in the physical insertion mode and the covalent linkage mode, a molar ratio of the bioactive substance linked in the physical insertion mode and the drug linked in the covalent linkage mode is 1 to 200:1. The linkage mode is suitable for the anthracycline and acridine drugs. A proportion of the drugs linked in the above different linkage modes is not limited to the above range, as long as it may meet a requirement of high-efficient loading, non-toxic effect on the cells, and the effective release of the drug is achieved after reaching a target.

In a preferred embodiment, the bioactive substance linked in the covalent linkage mode is covalently linked through a solvent, covalently linked through a linker or click-linked; preferably, the solvent is selected from paraformaldehyde, DCM, DCC, DMAP, Py, DMSO, PBS or glacial acetic acid; preferably, the linker is selected from a disulfide bond, a p-phenylazide, bromopropyne or a PEG; and preferably, the click-linkage is that alkynyl or azide modification is simultaneously performed on a bioactive substance precursor and the nucleic acid structural domain, and then they are linked through the click reaction.

When a bioactive substance precursor and the nucleic acid structural domain are simultaneously modified by an alkynyl or an azide, and linked in the click-linkage mode, the different click-linkages are selected according to changes of the different structures of the drugs. In addition, along with the different structures of the bioactive substances, the linkage positions may also be changed correspondingly, this may be understood by those skilled in the art.

In a preferred embodiment, when the bioactive substance is linked with the nucleic acid structural domain in the click-linkage mode, a site, for performing the alkynyl or azide modification, of the bioactive substance precursor is selected from a hydroxyl, a carboxyl, a mercapto or an amino, and a site, for performing the alkynyl or azide modification, of the nucleic acid structural domain is selected from the amino, an imino or the hydroxyl.

It is to be noted that when the above nucleic acid structural domain is linked with the drug, the nucleic acid structural domain is water-soluble, and most of the drugs are poorer in water solubility, after it is linked with the nucleic acid structural domain, the water solubility is improved. When the above drugs are anthracyclines, these drugs are covalent-linked with the nucleic acid structural domain through a -NH bond (in a suitable pH value condition, the activity of the -NH group is hundreds of times greater than the activity of other groups which may be covalent-linked with the drugs) on a nucleotide guanosine, thereby the nucleic acid structural domain for loading the drugs is formed. Therefore, according to a size of a specific drug molecule and the number of the GC base pairs on the sequence a, the sequence b and the sequence c in the specifically designed nucleic acid structural domain, when linked, a linking reaction is performed theoretically according to 1.1-1.3 times of a supersaturated linking amount, and at most 35 to 45 drugs may be linked in one nucleic acid structural domain. When the above drugs are other structures, the loading amount is related to an occupation situation (including but not limited to a molecular structure, a morphology, a shape and a molecular weight) of the specific drug. Therefore, a linkage condition of an activity site of the drug and the -NH bond on the nucleotide guanosine of the nucleic acid structural domain is relatively harsh, and it may also be loaded but it is relatively difficult to cause a situation of excessive linkage.

In a preferred embodiment, a particle size of the nucleic acid nanoparticles is 1-100 nm, preferably 5-50 nm; more preferably 10-30 nm; and further preferably 10-15 nm. The size is appropriate within this range, it may not only enter a cell membrane through cell phagocytosis mediated by a cell surface receptor, but also avoid non-specific cell penetration so as to be filtered and removed by the kidneys. Therefore, the favorable particle size is helpful to improve pharmacokinetics, pharmacodynamics, biological distribution and toxicological distribution.

In a second typical implementation mode, a pharmaceutical composition is further provided, and the pharmaceutical composition includes any one of the above nucleic acid nanoparticles. In the drug containing the above nucleic acid nanoparticles provided and specified in the present application, the nucleic acid structural domain may be modified by the target head targeted to the target cell to have the good targeting property, and may also load corresponding therapeutic drugs and/or tracer molecules, thereby the therapeutic drugs and/or tracer molecules may be stably delivered, and the reliability is very high.

In a third typical implementation mode, a drug comprising doxorubicin is provided, and the drug including the doxorubicin includes the doxorubicin and any one of the previous nucleic acid nanoparticles (without loading a bioactive

substance); or the drug including the doxorubicin is any one of the previous nucleic acid nanoparticles (loading the bioactive substance), herein, the bioactive substance is at least a drug, and the drug includes the the doxorubicin.

The above provided drug including the doxorubicin includes the nucleic acid nanoparticles and the doxorubicin, and the doxorubicin is loaded on the nucleic acid nanoparticles. The nucleic acid nanoparticles are capable of, through including the above three sequences or the variant sequence thereof, not only self-assembling to form the nucleic acid structural domain, but also serving as the carrier, and linking the doxorubicin at the arbitrary 5'-end and/or 3'-end of the three strands, or enabling the doxorubicin to be stably inserted between the strands of the nucleic acid structural domain. The drug including the doxorubicin provided by the disclosure, after the nucleic acid structural domain thereof is modified by the target head, may have the better targeting property, and may deliver the doxorubicin stably, the reliability is very high.

As above, while the bioactive substance is the drug, and the drug is the doxorubicin, the doxorubicin may be loaded in the modes of the physical linkage and/or the covalent linkage. While the doxorubicin is linked with the nucleic acid structural domain by using two modes of the physical insertion and the covalent linkage simultaneously, the physical insertion is usually inserted between the GC base pairs, the number of the preferred insertion sites is based on the different numbers of the GC base pairs on the nucleic acid structural domain, and the insertion is performed according to the ratio of 1 to 100:1. While the covalent linkage mode is used for linkage, the doxorubicin usually chemically reacts with a G-exocyclic amino to form the covalent linkage. More preferably, a molar ratio between the doxorubicin and the nucleic acid nanoparticles is 2 to 300:1, preferably 10 to 50:1, and more preferably 15 to 25:1.

In the drug including the doxorubicin provided by the present application, the nucleic acid nanoparticles are served as a delivery carrier of the doxorubicin. In addition, according to different purposes of the drugs, in a preferred embodiment, the above nucleic acid nanoparticles further include a bioactive substance, the bioactive substance is linked with the nucleic acid structural domain, and the bioactive substance is one or more of a target head, a fluorescein, an interfering nucleic acid siRNA, a miRNA, a ribozyme, a riboswitch, an aptamer, a RNA antibody, a protein, a polypeptide, a flavonoid, a glucose, a natural salicylic acid, a monoclonal antibody, a vitamin, phenols, a lecithin and a small molecular drug except the doxorubicin.

In order to improve loading efficiency and delivering efficiency of the nucleic acid nanoparticles to the loaded bioactive substance, a relative molecular weight of the nucleic acid structural domain and a relative molecular weight of the doxorubicin and bioactive substance should preferably have a certain matching relation. In a preferred embodiment, the relative molecular weight of the nucleic acid structural domain is marked as $N_1$, and the total relative molecular weight of the doxorubicin and the bioactive substance is marked as $N_2$, $N_1/N_2 \geq 1:1$.

According to the different types of the bioactive substances loaded specifically, the drug including the doxorubicin in the disclosure has optimizations in different performance aspects. For example, while the bioactive substance is a biotin or a folic acid, a function thereof is to make the drug including the doxorubicin have a targeting property, for example, specifically targeted to the cancer cells. While the bioactive substance is the fluorescein, a function thereof is to make the nucleic acid nanoparticles have a luminous tracking effect. While the bioactive substance is some siRNA, miRNA, protein, peptide, RNA antibody, and a small molecular drug except the doxorubicin, according to the different biological functions, the drug including the doxorubicin may be made into a new product with a specific therapeutic effect, such as a drug with more excellent performance.

In a preferred embodiment, the bioactive substance is the target head, the fluorescein and the miRNA, herein, the target head is positioned on any one sequence of the sequences a, b and c, preferably the 5'-end or the 3'-end of any one sequence of the sequences a, b and c, or inserted between GC bonds of the nucleic acid structural domain, the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c; and preferably, the target head is the folic acid or the biotin, the fluorescein is any one or more of FAM, CY5 and CY3, and the anti-miRNA is anti-miR-21.

The above target head may be linked on any one sequence of the sequences a, b and c in a mode of linker covalent linkage, the available linker is selected from a disulfide bond, a p-phenylazide, bromopropyne or PEG. Here, the "on any one sequence" refers to on a base in any one position of any one sequence of the sequences a, b and c, and it is more convenient to be linked at the 5'-end or 3'-end, the application is wider. Folic acid modification may be physical insertion mode linkage or physical insertion + covalent linkage.

The above fluorescein may be a commonly used fluorescein, and preferably any one of more of FAM, CY5 and CY3.

The above miRNA may be a miRNA with a cancer suppression effect, or may be an anti-miRNA which may suppress a corresponding disease, and it may be rationally selected in practical applications according to medical needs. The above anti-miRNA may be synthesized at any one or more positions of the 3'-end of the above sequence a, the 5'-end and 3'-end of the sequence c. While the anti-miRNA is synthesized in the above three positions, the anti-miRNA has a relatively stronger suppression effect on the corresponding miRNA.

It is anti-miR-21 preferably, the miR-21 participates in initiation and progression of multiple types of the cancers,

and is a main oncogene for invasion and metastasis. The anti-miR-21 may effectively regulate a wide range of target genes at the same time, and is beneficial to solve a problem of cancer heterogeneity. Therefore, in the above preferred nucleic acid nanoparticles, the target head, such as the folic acid or the biotin, may be specifically targeted to the cancer cells, and after being linked and internalized with the cancer cells, the anti-miR-21 is complemented with a miR-21 base in very high affinity and specificity, thereby the expression of the oncogenic miR-21 is effectively reduced. Therefore, according to the actual needs, the above anti-miR-21 may be synthesized at any one or more positions of the 3'-end of the above sequence a, the 5'-end and the 3'-end of the sequence c. While the anti-miR-21 is synthesized in the above three positions, the anti-miR-21 has a relatively stronger suppression effect on the miR-21.

While the above bioactive substance capable of loading is other small molecular drugs except the doxorubicin, according to types of diseases which may be treated by the different drugs, the drugs include but not limited to drugs for treating liver cancer, gastric cancer, lung cancer, breast cancer, head and neck cancer, uterine cancer, ovarian cancer, melanoma, leukemia, Alzheimer's disease, ankylosing spondylitis, malignant lymphoma, bronchial cancer, rheumatoid arthritis, HBV hepatitis B, multiple myeloma, pancreatic cancer, non-small cell lung cancer, prostate cancer, nasopharyngeal cancer, esophageal cancer, oral cancer, lupus erythematosus; and preferably, the head and neck cancer is brain cancer, neuroblastoma or glioblastoma.

While the above bioactive substance capable of loading is the small molecular drug except the doxorubicin, according to a difference of molecular structures of the drugs or a difference of characteristic groups which it has, the drug includes but not limited to a drug containing any one or more of the following groups: an amino group, a hydroxyl group, a carboxyl group, a mercapto group, a benzene ring group and an acetamido group.

In a preferred embodiment, the above protein is one or more of antibodies or aptamers of superoxide dismutase (SOD), survivin, human telomerase reverse transcriptase (hTERT), epidermal growth factor receptor (EGFR) and prostate-specific membrane antigen (PSMA); the above vitamin is L-C and/or esterified C; and the above phenols are tea polyphenols and/or grape polyphenols.

In a preferred embodiment, a particle size of the nucleic acid nanoparticles is 1-100 nm, preferably 5-50 nm; more preferably 10-30 nm; and further preferably 10-15 nm. The size is appropriate within this range, it may not only enter a cell membrane through cell phagocytosis mediated by a cell surface receptor, but also avoid non-specific cell penetration so as to be filtered and removed by the kidneys. Therefore, the favorable particle size is helpful to improve pharmacokinetics, pharmacodynamics, biological distribution and toxicological distribution.

According to the third typical implementation mode of the disclosure, a preparation method for the above drug including the doxorubicin is further provided, and it includes the following steps: providing the nucleic acid nanoparticles (without loading a bioactive substance) mentioned above; enabling the doxorubicin to be loaded on the nucleic acid nanoparticles in modes of physical linkage and/or covalent linkage, to obtain the drug including the doxorubicin.

While the physical linkage mode is used, the doxorubicin is usually inserted between the GC base pairs in the physical linkage mode. While the covalent linkage mode is used for linkage, the doxorubicin usually chemically reacts with the G-exocyclic amino to form the covalent linkage. The drug including the doxorubicin prepared by using the above method, after it is modified by the target head, may have a better targeting property, and may deliver the doxorubicin stably, the reliability is very high.

In a preferred embodiment, the step of loading the doxorubicin in the physical linkage mode includes: enabling the doxorubicin, the nucleic acid nanoparticles and a first solvent to be mixed and stirring, to obtain a premixed system; and removing a free substance in the premixed system, to obtain the drug including the doxorubicin. Specific dosages of the doxorubicin and the nucleic acid nanoparticles may be adjusted according to a change of the loading amount, this may be understood by those skilled in the art, and it is not repeatedly described here.

In order to improve the efficiency and the stability of the physical linkage, an amount of the doxorubicin added per liter of the first solvent is preferably 0.1 to 1 g. Preferably, the first solvent is selected from one or more of DCM, DCC, DMAP, Py, DMSO, PBS and glacial acetic acid; preferably, the step of removing the free substance in the premixed system includes: enabling the premixed system to be mixed with absolute ethyl alcohol, and precipitating the drug including the doxorubicin at a temperature condition lower than 10 DEG C; and more preferably, precipitating the drug including the doxorubicin at a temperature condition of 0-5 DEG C.

In a preferred embodiment, the step of loading the doxorubicin in the covalent linkage mode includes: preparing doxorubicin solution; enabling the doxorubicin solution to react with the G-exocyclic amino of the nucleic acid nanoparticles under a mediating effect of the formaldehyde, to obtain a reaction system; and purifying the reaction system, to obtain the drug comprising the doxorubicin.

Through a formaldehyde-mediated form, the following reactions may occur:

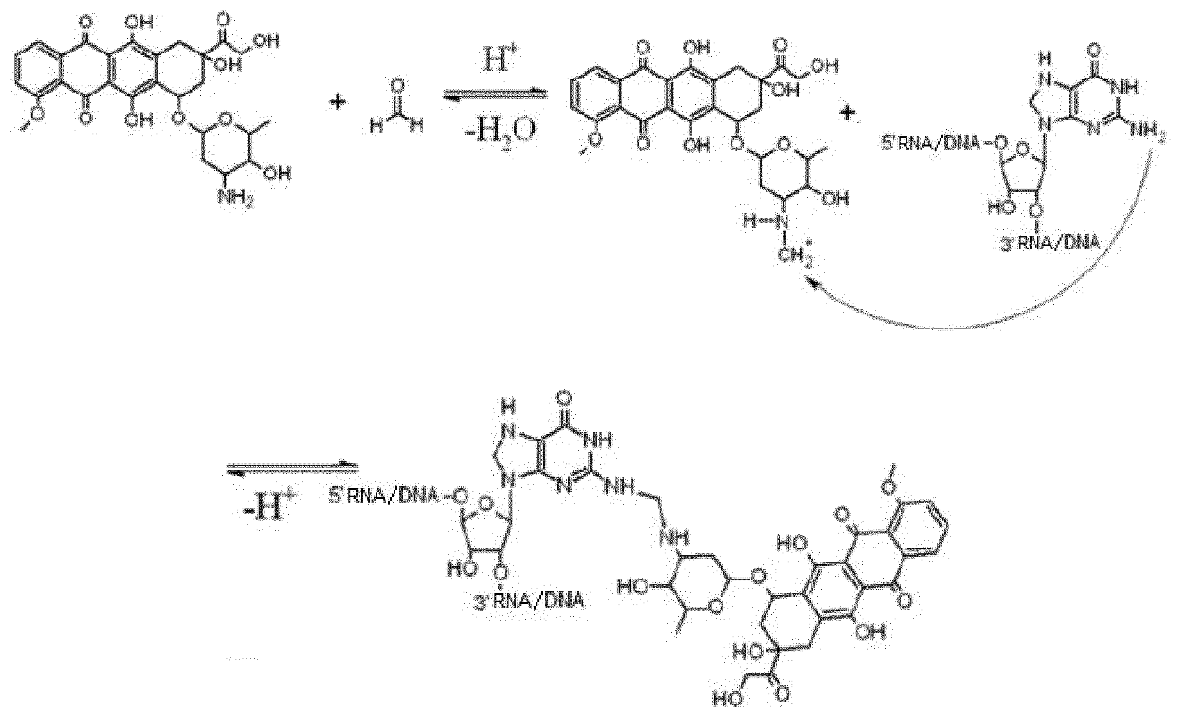


Preferably, the reaction step includes: enabling the doxorubicin solution to be mixed with paraformaldehyde solution and the nucleic acid nanoparticles, and reacting in a dark condition, to obtain the reaction system. The paraformaldehyde solution may release formaldehyde small molecules, as to participate in the above chemical reaction. In order to improve the reaction efficiency, the concentration of the paraformaldehyde solution is preferably 3.7-4wt%, and the paraformaldehyde solution is preferably solution formed by mixing paraformaldehyde and a second solvent, and the second solvent is one or more of DCM, DCC, DMAP, Py, DMSO, PBS and glacial acetic acid.

In the above preparation method, the nucleic acid nanoparticles may be prepared in a mode of self-assembly, for example: (1) enabling the RNA or DNA single strands a, b and c to be simultaneously mixed and dissolved in DEPC water or TMS buffer solution; (2) heating mixed solution to 80/95 DEG C (herein a RNA assembly temperature is 80 DEG C, and a DNA assembly temperature is 95 DEG C), after keeping for 5 min, slowly cooling to a room temperature at a rate of 2 DEG C/min; (3) enabling a product to be loaded on 8% (m/v) of non-denaturing PAGE gel and placed in TBM buffer solution under a condition of 4 DEG C, purifying a complex by 100 v of electrophoresis; and (4) cutting a target band and eluting in RNA/DNA elution buffer solution at 37 DEG C, after that, precipitating overnight in ethanol, volatilizing under reduced pressure and low temperature, to obtain a self-assembly product, namely the nucleic acid structural domain, and then acquiring the nucleic acid nanoparticles.

In order to make the above drug including the doxorubicin have other functions, in a preferred embodiment, after the nucleic acid structural domain is obtained, the preparation method further includes: enabling the above bioactive substance to be loaded on the nucleic acid structural domain in the modes of physical linkage and/or covalent linkage, to obtain the nucleic acid nanoparticles. The loading mode of the bioactive substance may also be the physical linkage and/or the covalent linkage. The covalent linkage mode includes but not limited to the solvent covalent linkage, the linker covalent linkage or the click-linkage; preferably, a third solvent used in the solvent covalent linkage is served as a linkage medium, and the third solvent is selected from one or more of paraformaldehyde, DCM, DCC, DMAP, Py, DMSO, PBS and glacial acetic acid; preferably, the linker is selected from a disulfide bond, a p-phenylazide, bromopropyne or PEG; and preferably, the click-linkage is that alkynyl or azide modification is simultaneously performed on a bioactive substance precursor and the nucleic acid structural domain, and then they are linked through the click-linkage.

It is to be noted that the above classification does not mean that there is only one linkage mode to link a certain bioactive substance with the nucleic acid nanocarrier. However, some bioactive substances may be linked with the nucleic acid nanocarrier in the physical insertion mode, or may be linked with the nucleic acid nanocarrier in the physical insertion and covalent linkage modes, or may be linked by using the click-linkage mode at the same time. However, for a certain specific bioactive substance, there may be only one linkage mode, or there may be multiple linkage modes, but it may be that the efficiency of certain linkage has an advantageous practical value.

In the above linkage modes, while the different drugs are linked with the nucleic acid structural domain in the physical insertion mode, insertion linkage sites and numbers are also different slightly. For example, while anthracycline and acridine drugs are inserted, they are usually inserted between the GC base pairs, the preferred number of the

insertion sites is based on the different numbers of the GC base pairs in the nucleic acid structural domain, and the insertion is performed in a ratio of 1 to 100:1. While a naphthylamide drug is inserted, it is usually inserted between the AA base pairs, the preferred number of the insertion sites is based on the different numbers of the AA base pairs in the nucleic acid structural domain, and pyridocarbazoles are inserted according to the different numbers of the AA base pairs in a ratio of 1 to 200:1.

Specifically, according to the different types of the bioactive substances, the lengths of the sequences a, b and c for forming the nucleic acid structural domain in the nucleic acid nanoparticles and the number of GC complementary base pairs thereof, the physical insertion may be performed by rationally selecting a molar ratio of the bioactive substance and the nucleic acid structural domain.

In a preferred embodiment, while the bioactive substance is linked with the nucleic acid structural domain in the physical insertion mode and the covalent linkage mode, a molar ratio of the bioactive substance linked in the physical insertion mode and the drug linked in the covalent linkage mode is 1 to 200:1. The linkage mode is suitable for the anthracycline and acridine drugs. The proportion of the drugs linked in the above different linkage modes is not limited to the above range, as long as it may meet a requirement of high-efficient loading, there is a non-toxic effect on the cells, and the effective release of the drug is achieved after reaching a target.

While a bioactive substance precursor and the nucleic acid structural domain are simultaneously modified by an alkynyl or an azide, and linked in the click-linkage mode, the different click-linkages are selected according to changes of the different structures of the drugs. In addition, along with the different structures of the bioactive substances, the linkage positions may also be changed correspondingly, this may be understood by those skilled in the art.

In a preferred embodiment, while the bioactive substance is linked with the nucleic acid structural domain in the click-linkage mode, a site, for performing the alkynyl or azide modification, of the bioactive substance precursor is selected from a hydroxyl, a carboxyl, a mercapto or an amino, and a site, for performing the alkynyl or azide modification, of the nucleic acid structural domain is selected from the amino, an imino or the hydroxyl.

It is to be noted that the nucleic acid nanoparticles formed by the self-assembly of the sequences or sequence variations provided by the present application may also be used as basic structural units, and may be further polymerized to form multimers, such as a dimer, a trimer, a tetramer, a pentamer, a hexamer or a heptamer, according to the actual application needs.

The beneficial effects of the present application are further described below in combination with specific embodiments.

**Assembly of nucleic acid nanoparticles**

Embodiment 1

I. RNA and DNA nanoparticle carrier:

(1) Three polynucleotide base sequences for assembling RNA nanoparticles are specifically shown in Table 1:

Table 1:

| Name | Sequence sense (5'-3') | Base number | Molecular weight ( MW) | Chemical modification | Fluore scence mark | Special modifica tion | Total molecular weight |
|---|---|---|---|---|---|---|---|
| a-strand (SEQ ID NO:52) | cGcGcGcccAc cAGcGuuccG GGcGccGc | 29 | 9678.7 | C/U base 2'F modification | / | 5'Biotin | 29524.9 |
| b-strand (SEQ ID NO:53) | GcGGcGcccG GuucGccGccA GGcGGc | 27 | 9116.8 | C/U base 2'F modification | / | 5'Biotin | |
| c-strand (SEQ ID NO:54) | GccGccAGGc GGccAuAGcG GuGGGcGcG cG | 31 | 10729.4 | C/U base 2'F modification | 5'CY3 | | |

(2) Three polynucleotide base sequences of DNA nanoparticles
DNA uses the same sequence as the above RNA, except that U is replaced by T. Herein, a molecular weight of the a-strand is 8802.66, a molecular weight of the b-strand is 8280.33, and a molecular weight of the c-strand is 9605.2.

The strands a, b and c of the above RNA nanoparticles and DNA nanoparticles are all commissioned to be synthesized by Sangon Bioengineering (Shanghai) Co., Ltd.

II. Self-assembly experiment steps:

(1) according to a molar ratio of 1:1:1, enabling the RNA or DNA single strands a, b and c to be simultaneously mixed and dissolved in DEPC treated water or TMS buffer solution;
(2) heating mixed solution to 80/95°C (herein a RNA assembly temperature is 80°C, and a DNA assembly temperature is 95°C), after keeping for 5 min, slowly cooling to a room temperature at a rate of 2°C/min;
(3) enabling a product to be loaded on 8% (m/v) of non-denaturing PAGE gel and placed in TBM buffer solution under a condition of 4°C, purifying a complex by 100 v of electrophoresis;
(4) cutting a target band and eluting in RNA/DNA elution buffer solution at 37°C, after that, precipitating overnight in ethanol, volatilizing under reduced pressure and low temperature, to obtain a self-assembly product; and
(5) electrophoresis analysis detection and laser scanning observation.

III. Self-assembly experiment result

Electrophoresis detection result

An electrophoresis detection result of the RNA self-assembly product is shown in Fig. 1. In Fig. 1, lanes 1 to 3 from left to right are successively: the a-strand, the b-strand, and the RNA self-assembly product. It may be seen from the figure that although the RNA self-assembly product is slightly diffused, it may be apparently seen that it is a single band. In addition, because the molecular weight is a molecular weight after assembly, it is larger than a single-stranded molecular weight, therefore a band position is behind the a-strand and the b-strand, and the actual situation is consistent with a theory, it is proved that a stable composite structure is formed between the above RNA single strands by the self-assembly, and RNA nanoparticles are formed.

An electrophoresis detection result of the DNA self-assembly product is shown in Fig. 2. In Fig. 2, lanes 1 to 3 from left to right are successively: the a-strand, the b-strand, and the DNA self-assembly product. It may be seen from the figure that a band of the DNA self-assembly product is bright and clear, and is a single band, it is proved that a stable composite structure is formed between the above DNA single strands by the self-assembly, and DNA nanoparticles are formed.

In the embodiment, it is verified by gel electrophoresis that the sequences a, b and c including the RNA core

sequences SEQ ID NO:1, SEQ ID NO:3 and SEQ ID NO:5 may be successfully self-assembled into the RNA nanoparticles. The sequences a, b and c including the DNA core sequences SEQ ID NO:2, SEQ ID NO:4 and SEQ ID NO:6 may also be successfully self-assembled into the DNA nanoparticles.

In addition to the core sequences which form a nucleic acid structural domain, the sequences a, b and c of the above RNA nanoparticles and DNA nanoparticles also have various extension sequences (including a drug loading linking sequence) which promote a loading function of the nucleic acid structural domain and a target head or a fluorescein which is linked with the nucleic acid structural domain. It may be seen that the presence of substances other than these core sequences does not affect the formation of the nucleic acid structural domain and the successful self-assembly of the nucleic acid nanoparticles. The self-assembled nucleic acid nanoparticles may have a targeting property under the guidance of the target head, and the fluorescein may make the nucleic acid nanoparticles have visibility and traceability.

Embodiment 2

I. 7 groups of short-sequence RNA nanoparticle carriers:

(1) 7 groups of three polynucleotide sequences for forming RNA nanoparticles:

Table 2: R-1:

| Name | Sequence sense(5'-3') | Base number | Chemical modification | Molecular weight | Total molecular weight |
|---|---|---|---|---|---|
| a-strand(SEQ ID NO:55) | **GG**AGcGuu**GG** | 10 | c/u base 2'F modification | 3262.9 | 9828.7 |
| b-strand(SEQ ID NO:56) | **cc**uuc**GCCG** | 9 | c/u base 2'F modification | 2780.6 | |
| c-strand(SEQ ID NO:57) | c**GGc**cAuAGc**cc** | 12 | c/u base 2'F modification | 3785.2 | |

Table 3: R-2:

| Name | Sequence sense(5'-3') | Base number | Chemical modification | Molecular weight | Total molecular weight |
|---|---|---|---|---|---|
| a-strand(SEQ ID NO:58) | **Gc**AGcGuu**cG** | 10 | c/u base 2'F modification | 3186.7 | 9829.4 |
| b-strand(SEQ ID NO:59) | **cG**uuc**GccG** | 9 | c/u base 2'F modification | 2820.2 | |
| c-strand(SEQ ID NO:60) | **cGGc**cAuAGc**Gc** | 12 | c/u base 2'F modification | 3822.5 | |

Table 4: R-3:

| Name | Sequence sense(5'-3') | Base number | Chemical modification | Molecular weight | Total molecular weight |
|---|---|---|---|---|---|
| a-strand(SEQ ID NO:61) | cGAGcGuuGc | 10 | c/u base 2'F modification | 3187.5 | 9829.9 |
| b-strand(SEQ ID NO:62) | GcuucGccG | 9 | c/u base 2'F modification | 2819.2 | |
| c-strand(SEQ ID NO:63) | cGGccAuAGccG | 12 | c/u base 2'F modification | 3823.2 | |

Table 5: R-4:

| Name | Sequence sense (5'-3') | Base number | Chemical modification | Molecular weight | Total molecular weight |
|---|---|---|---|---|---|
| a-strand(SEQ ID NO:64) | GGAGcGuuGG | 10 | c/u base 2'F modification | 3263.7 | 9830.9 |
| b-strand(SEQ ID NO:65) | ccuucGGGG | 9 | c/u base 2'F modification | 2858.2 | |
| c-strand(SEQ ID NO:66) | cccccAuAGccc | 12 | c/u base 2'F modification | 3709.0 | |

Table 6: R-5:

| Name | Sequence sense(5'-3') | Base number | Chemical modification | Molecular weight | Total molecular weight |
|---|---|---|---|---|---|
| a-strand(SEQ ID NO: 67) | GcAGcGuucG | 10 | c/u base 2'F modification | 3187.5 | 9830.2 |
| b-strand(SEQ ID NO: 68) | cGuucGGcG | 9 | c/u base 2'F modification | 2857.5 | |
| c-strand(SEQ ID NO: 69) | cGcccAuAGcGc | 12 | c/u base 2'F modification | 3785.2 | |

Table 7: R-6:

| Name | Sequence sense(5'-3') | Base number | Chemical modification | Molecular weight | Total molecular weight |
|---|---|---|---|---|---|
| a-strand(SEQ ID NO:70) | GcAGcGuucG | 10 | c/u base 2'F modification | 3187.2 | 9830.5 |
| b-strand(SEQ ID NO:71) | cGuucGGcc | 9 | c/u base 2'F modification | 2820.4 | |
| c-strand( SEQ ID NO:72) | GGcccAuAGcGc | 12 | c/u base 2'F modification | 3822.9 | |

Table 8: R-7:

| Name | Sequence sense(5'-3') | Base number | Chemical modification | Molecular weight | Total molecular weight |
|---|---|---|---|---|---|
| a-strand(SEQ ID NO:73) | cGAGcGuuGc | 10 | c/u base 2'F modification | 3187.6 | 9830.7 |
| b-strand(SEQ ID NO:74) | GcuucGGcG | 9 | c/u base 2'F modification | 2857.8 | |
| c-strand(SEQ ID NO:75) | cGcccAuAGccG | 12 | c/u base 2'F modification | 3785.3 | |

The single strands of the above 7 groups of the short-sequence RNA nanoparticle carriers are all commissioned to be synthesized by Sangon Bioengineering (Shanghai) Co., Ltd.

II. Self-assembly experiment steps:

(1) according to a molar ratio of 1:1:1, enabling the RNA single strands a, b and c to be simultaneously mixed and dissolved in DEPC treated water or TMS buffer solution;
(2) heating mixed solution to 80 DEG C, after keeping for 5 min, slowly cooling to a room temperature at a rate of 2 DEG C/min;
(3) enabling a product to be loaded on 8% (m/v) of non-denaturing PAGE gel and placed in TBM buffer solution under a condition of 4 DEG C, purifying a complex by 100 v of electrophoresis;
(4) cutting a target band and eluting in RNA elution buffer solution at 37 DEG C, after that, precipitating overnight in ethanol, volatilizing under reduced pressure and low temperature, to obtain a short-sequence RNA self-assembly product;
(5) electrophoresis analysis detection and laser scanning observation; and
(6) electric potential detection.

III. Self-assembly experiment result

(1) Electrophoresis detection result

A 2% agarose gel electrophoresis diagram of the 7 groups of the short-sequence RNA self-assembly products is shown in Fig. 3. From left to right, lanes 1 to 7 in Fig. 3 are successively: short-sequences R-1, R-2, R-3, R-4, R-5, R-6 and R-7.
A 4% agarose gel electrophoresis diagram of the 7 groups of the short-sequence RNA self-assembly products is shown in Fig. 4. From left to right, lanes 1 to 7 in Fig. 4 are successively: short-sequences R-1, R-2, R-3, R-4, R-5, R-6 and R-7.
It may be clearly seen from results of Fig. 3 and Fig. 4 that bands of the R-2, R-3, R-5 and R-7 in the 7 groups of the short-sequence self-assembly products are bright and clear, although the R-1, R-4 and R-6 are relatively diffused, it may still be seen as a single band, it is indicated that the 7 groups of the short-sequences may be self-assembled better into a RNA nanoparticle structure.

(2) Electric potential measurement

Measurement method: preparing a potential sample (the self-assembly product is dissolved in ultrapure water) and putting into a sample pool, opening a sample pool cover of an instrument, and placing the instrument;
opening software, clicking a menu MeasUre€ManUal, so a manual measurement parameter setting dialog box appears;
setting a software detection parameter;
and then clicking Ok to complete the settings, after a measurement dialog box appears, clicking Start to start.
Measurement result: potential detection results of the 7 groups of the short-sequence RNA nanoparticles are as follows:

Table 9:

| Sample name | Detection times | Potential ZP (mV) |
|---|---|---|
| R-1 | 1 | -33.7 |
| | 2 | -35.6 |
| | 3 | -34.6 |
| Experiment result | -34.65 mV. (±0.95mV) | |

Table 10:

| Sample name | Detection times | Potential ZP (mV) |
|---|---|---|
| R-2 | 1 | -37.9 |
| | 2 | -37.3 |
| | 3 | -36.8 |
| Experiment result | -37.35 mV. (±0.55mV) | |

Table 11:

| Sample name | Detection times | Potential ZP (mV) |
|---|---|---|
| R-3 | 1 | -31.5 |
| | 2 | -33 |
| | 3 | -32.7 |
| Experiment result | -32.425 mV. (±0.75mV) | |

Table 12:

| Sample name | Detection times | Potential ZP (mV) |
|---|---|---|
| R-4 | 1 | -35.40 |
| | 2 | -36.00 |
| | 3 | -35.50 |
| Experiment result | -35.7 mV.(±0.3mV) | |

Table 13:

| Sample name | Detection times | Potential ZP (mV) |
|---|---|---|
| R-5 | 1 | -34.00 |
| | 2 | -33.30 |
| | 3 | -34.90 |
| Experiment result | -34.1 mV. (±0.8mV) | |

Table 14:

| Sample name | Detection times | Potential ZP (mV) |
|---|---|---|
| R-6 | 1 | -33.10 |
| | 2 | -36.10 |
| | 3 | -35.60 |
| Experiment result | -34.6 mV. (±1.5mV) | |

Table 15:

| Sample name | Detection times | Potential ZP (mV) |
|---|---|---|
| R-7 | 1 | -35.60 |
| | 2 | -34.80 |
| | 3 | -34.00 |
| Experiment result | -34.80 mV. ±0.8mV | |

It may be seen from the above potential detection data that the 7 groups of the short-sequence RNA self-assembly products all have good stability, and it is further indicated that the nanoparticles formed by the self-assembly of each short-sequence RNA have a relatively stable self-assembly structure.

It is indicated from the embodiment that: different combinations of the core sequences a, b and c may form the RNA nanoparticles with the nucleic acid structural domain through the self-assembly, and the structure is stable. It may be seen on the basis of Embodiment 1 that the RNA nanoparticles may also be successfully assembled by adding various functional extension fragments or a linkage target head, a fluorescein and the like on the basis of these different core sequence combinations, and have functions such as drug loading, cell targeting, visibility and traceability.

In order to further verify these performances, the extension fragment is added on the basis of Embodiment 2, and it is specifically described in Embodiment 3. On the basis of the DNA core sequence corresponding to the RNA core sequence of Embodiment 2, the extension fragment is added, and the target head is connected or unconnected at the same time, it is specifically described in Embodiment 4.

Embodiment 3

I. 7 groups of conventional-sequence RNA nanoparticle carriers:

(1) 7 groups of three polynucleotide base sequences for forming RNA nanoparticles:

Table 16: R-8:

| Name | Sequence sense(5'-3') | Base number | Chemical modification | Molecular weight | Total molecular weight |
|---|---|---|---|---|---|
| a(SEQ ID NO:76) | cGcGcGcccAGGAGcGuuG GcGGGcGGcG | 29 | C/U 2'F modification | 9462.78 | 28084.13 |
| b(SEQ ID NO:77) | cGccGcccGccuucGccGccA GccGcc | 27 | C/U 2'F modification | 8522.18 | |
| c(SEQ ID NO:78) | GGcGGcAGGcGGccAuAG cccuGGGcGcGcG | 31 | C/U 2'F modification | 10099.17 | |

Table 17: R-9:

| Name | Sequence sense(5'-3') | Base number | Chemical modification | Molecular weight | Total molecular weight |
|---|---|---|---|---|---|
| a(SEQ ID NO: 79) | cGcGcGcccAGcAGcGuuc GcGGGcGGcG | 29 | C/U 2'F modification | 9386.73 | 28084.13 |
| b(SEQ ID NO: 80) | cGccGcccGcGuucGccGcc AGccGcc | 27 | C/U 2'F modification | 8560.20 | |
| c(SEQ ID NO: 81 ) | GGcGGcAGGcGGccAuAG cGcuGGGcGcGcG | 31 | C/U 2'F modification | 10137.20 | |

Table 18: R-10:

| Name | Sequence sense(5'-3') | Base number | Chemical modification | Molecular weight | Total molecular weight |
|---|---|---|---|---|---|
| a(SEQ ID NO:82) | cGcGcGcccAcGAGcGuuG cGGGGcGGcG | 29 | C/U 2'F modification | 9424.75 | 28084.13 |
| b(SEQ ID NO:83) | cGccGccccGcuucGccGccA GccGcc | 27 | C/U 2'F modification | 8522.18 | |
| c(SEQ ID NO:84) | GGcGGcAGGcGGccAuAG ccGuGGGcGcGcG | 31 | C/U 2'F modification | 10137.20 | |

Table 19: R-11:

| Name | Sequence sense(5'-3') | Base number | Chemical modification | Molecular weight | Total molecular weight |
|---|---|---|---|---|---|
| a(SEQ ID NO:85) | cGcGcGcccAGGAGCGuu GGcccGcGGcG | 29 | C/U 2'F modification | 9386.73 | 28084.13 |
| b(SEQ ID NO:86) | cGccGcGGGccuucGGGGc cAGccGcc | 27 | C/U 2'F modification | 8674.28 | |
| c(SEQ ID NO:87) | GGcGGcAGGcccccAuAGc ccuGGGcGcGcG | 31 | C/U 2'F modification | 10023.12 | |

Table 20: R-12:

| Name | Sequence sense(5'-3') | Base number | Chemical modification | Molecular weight | Total molecular weight |
|---|---|---|---|---|---|
| a(SEQ ID NO: 88) | cGcGcGcccAGcAGcGuuc GccccGccGc | 29 | C/U 2' F modification | 9234.62 | 28084.18 |
| b(SEQ ID NO: 89) | GcGGcGGGGcGuucGGcG GcAGGcGGc | 27 | C/U 2'F modification | 8864.41 | |
| c(SEQ ID NO: 90) | GccGccAGccGcccAuAGcG cuGGGcGcGcG | 31 | C/U 2'F modification | 9985.10 | |

Table 21: R-13:

| Name | Sequence sense(5'-3') | Base number | Chemical modification | Molecular weight | Total molecular weight |
|---|---|---|---|---|---|
| a(SEQ ID NO: 91 ) | cGcGcGcccAGcAGcGuuc GGGGcGccGc | 29 | C/U 2'F modification | 9348.70 | 28736.53 |
| b(SEQ ID NO: 92) | GcGGcGccccGuucGGccG GcAGGcGGc | 28 | C/U 2'F modification | 9057.52 | |
| c(SEQ ID NO: 93) | GccGccAGccGGcccAuAGc GcuGGGcGcGcG | 32 | C/U 2'F modification | 10330.31 | |

Table 22: R-14: (uGAcAGAuAAGGAAccuGcudTdT in the following a-strand is survivin siRNA)

| Name | Sequence sense(5'-3') | Base number | Chemical modification | Molecular weight | Total molecular weight |
|---|---|---|---|---|---|
| a(SEQ ID NO: 94) | cGcGcGcGAGcGuuGcAAu GAcAGAuAAGGAAccuGcu dTdT | 39 | C/U 2'F modification | 12901.91 | 31832.42 |
| b(SEQ ID NO: 95) | GGcAGGuuccuuAucuGucA AAGcuucGGcGGcAGc | 36 | C/U 2'F modification | 11555.97 | |
| c(SEQ ID NO: 96) | GcAGccGcccAuAGccGcGc GcG | 23 | C/U 2'F modification | 7374.54 | |

The single strands of the above 7 groups of conventional-sequence RNA nanoparticle carriers are all commissioned to be synthesized by Suzhou Gima Company, herein the sequence a, the sequence b and the sequence c in R-8 to R-14 are respectively extended RNA oligonucleotide sequences formed by adding the extension fragments on the basis of the sequence a, the sequence b and the sequence c in R-1 to R-7, a targeting module fragment is not extended, and C/U base 2'F modification (resistance to enzyme digestion and stability are enhanced) is performed. In addition, a siRNA nucleic acid interference therapeutic fragment of a survivin is modified in the above RNA nanoparticle R-14, specifically a sense strand (see an underlined part of the a-strand) of Survivin siRNA is extended at a-strand 3'-end, and an antisense strand (see an underlined part of the b-strand) is extended and connected at b-strand 5'-end, so base pair complementary is formed.

II. Self-assembly experiment steps:

(1) according to a molar ratio of 1:1:1, enabling the RNA single strands a, b and c to be simultaneously mixed and dissolved in DEPC treated water or TMS buffer solution;
(2) heating mixed solution to 80°C, after keeping for 5 min, slowly cooling to a room temperature at a rate of 2°C/min;
(3) enabling a product to be loaded on 8% (m/v) of non-denaturing PAGE gel and placed in TBM buffer solution under a condition of 4°C, purifying a complex by 100 v of electrophoresis;
(4) cutting a target band and eluting in RNA elution buffer solution at 37°C, after that, precipitating overnight in ethanol, volatilizing under reduced pressure and low temperature;
(5) electrophoresis analysis detection and laser scanning observation;
(6) electric potential detection.

III. Self-assembly experiment result

(1) Electrophoresis detection result

A 2% agarose gel electrophoresis diagram of the 7 groups of the conventional-sequence RNA self-assembly products is shown in Fig. 5. From left to right, lanes 1 to 7 in Fig. 5 are successively: conventional-sequence RNA self-assembly products R-8, R-9, R-10, R-11, R-12, R-13 and R-14.

A 4% agarose gel electrophoresis diagram of the 7 groups of the conventional-sequence RNA self-assembly products is shown in Fig. 6. From left to right, lanes 1 to 7 in Fig. 6 are successively: conventional-sequence RNA self-assembly products R-8, R-9, R-10, R-11, R-12, R-13 and R-14.

It may be clearly seen from results of Fig. 5 and Fig. 6 that bands of the 7 groups of the conventional-sequence self-assembly products are bright and clear single bands, it is indicated that the 7 groups of the conventional-sequences may be self-assembled into a nanostructure. Herein after a Survivin siRNA nucleic acid interference therapeutic fragment is modified in the conventional-sequence RNA self-assembly product R-14, it still has the stable self-assembly structure, it is also indicated that the nucleic acid nanoparticles of the disclosure may load a nucleic acid drug, and have a delivery carrier function of the nucleic acid drug.

(2) Electric potential measurement

Measurement method: preparing a potential sample (the self-assembly product is dissolved in ultrapure water) and putting into a sample pool, opening a sample pool cover of an instrument, and placing the instrument;
opening software, clicking a menu MeasUre€ManUal, so a manual measurement parameter setting dialog box appears;
setting a software detection parameter;
and then clicking Ok to complete the settings, after a measurement dialog box appears, clicking Start to start.

Measurement result: potential detection results of the 7 groups of the conventional-sequence RNA nanoparticles are as follows:

Table 23:

| Sample name | Detection times | Potential ZP (mV) |
|---|---|---|
| R-8 | 1 | -18.00 |
| | 2 | -16.10 |
| | 3 | -18.20 |
| Experiment result | -17.43 mV (±1.33mV) | |

Table 24:

| Sample name | Detection times | Potential ZP (mV) |
|---|---|---|
| R-9 | 1 | -16.90 |
| | 2 | -17.50 |
| | 3 | -20.20 |
| Experiment result | -18.20 mV (±1.3mV) | |

Table 25:

| Sample name | Detection times | Potential ZP (mV) |
|---|---|---|
| R-10 | 1 | -10.40 |
| | 2 | -11.80 |
| | 3 | -10.40 |
| Experiment result | -10.87 mV (±0.47mV) | |

Table 26:

| Sample name | Detection times | Potential ZP (mV) |
|---|---|---|
| R-11 | 1 | -28.30 |
| | 2 | -26.00 |
| | 3 | -33.10 |
| Experiment result | -29.13mV (±3.13mV) | |

Table 27:

| Sample name | Detection times | Potential ZP (mV) |
|---|---|---|
| R-12 | 1 | -7.59 |
| | 2 | -7.80 |
| | 3 | -17.20 |
| Experiment result | -10.86 mV (±3.27mV) | |

Table 28:

| Sample name | Detection times | Potential ZP (mV) |
|---|---|---|
| R-13 | 1 | -9.64 |
| | 2 | -15.60 |
| | 3 | -21.10 |
| Experiment result | -15.45 mV (±5.81mV) | |

Table 29:

| Sample name | Detection times | Potential ZP (mV) |
|---|---|---|
| R-14 | 1 | -21.40 |
| | 2 | -21.20 |
| | 3 | -28.00 |
| Experiment result | -23.53 mV. ±2.33mV | |

It can be seen from the above potential detection data that the 7 groups of the conventional-sequence RNA self-assembly products all have good stability, and it is further indicated that the nanoparticle formed by the self-assembly of each conventional-sequence RNA have a relatively stable self-assembly structure.

It is indicated from the embodiment that: on the basis of different combinations of the RNA core sequences, the RNA nanoparticle with the stable structure may also be successfully self-assembled by adding the extension fragments. At the same time, the added extension fragments make the RNA nanoparticle have superior drug loading performance (specifically described in Embodiment 5 and Embodiment 7).

Embodiment 4

I. 7 groups of conventional-sequence DNA nanoparticle carriers:

(1) 7 groups of three polynucleotide base sequences for forming DNA nanoparticle:

An EGFRapt target head or a PSMAapt(A9L) target head is extended in a part of a-strands in the table:

EGFRapt (SEQ ID NO:97): GCCTTAGTAACGTGCTTTGATGTCGATTCGACAGGAGGC;

PSMAapt (A9L, SEQ ID NO:98): GGGCCGAAAAAGACCTGACTTCTATACTAAGTCTACGTCCC.

Table 30: D-1:

| Name | Sequence sense(5'-3') | Base number | Chemical modification | Molecular weight | Total molecular weight |
|---|---|---|---|---|---|
| a(SEQ ID NO: 99) | CGCGCGCCCAGGAGCGTTGG CGGGCGGCGGCCTTAGTAACG TGCTTTGATGTCGATTCGACAG GAGGC | 68 | 3 bases before 5'-end and 3'-end, thio modification | 21214.63 | 39092.09 |
| b(SEQ ID NO: 100) | CGCCGCCCGCCTTCGCCGCCA GCCGCC | 27 | 3 bases before 5'-end and 3'-end, thio modification | 8176.24 | |
| c(SEQ ID NO: 101) | GGCGGCAGGCGGCCATAGCC CTGGGCGCGCG | 31 | 3 bases before 5'-end and 3'-end, thio modification | 9701.22 | |

Table 31: D-2:

| Name | Sequence sense(5'-3') | Base number | Chemical mod ificatio n | Molecular weight | Total molecular weight |
|---|---|---|---|---|---|
| a(SEQ ID NO: 10 2) | CGCGCGCCCAGCAGCGTTCGCG GGCGGCGGCCTTAGTAACGTGCT TTGATGTCGATTCGACAGGAGGC | 68 | 3 bases before 5'-end and 3'-end, thio modification | 21134.59 | 39092.11 |
| b(SEQ ID NO: 10 3) | CGCCGCCCGCGTTCGCCGCCAG CCGCC | 27 | 3 bases before 5'-end and 3'-end, thio modification | 8216.27 | |
| c(SEQ ID NO: 10 4) | GGCGGCAGGCGGCCATAGCGCT GGGCGCGCG | 31 | 3 bases before 5'-end and 3'-end, thio modification | 9741.25 | |

Table 32: D-3:

| Name | Sequence sense(5'-3') | Base number | Chemical mod ificatio n | Molecular weight | Total molecular weight |
|---|---|---|---|---|---|
| a(SE Q ID NO:1 05) | CGCGCGCCCACGAGCGTTGCGGG GCGGCGGCCTTAGTAACGTGCTTT GATGTCGATTCGACAGGAGGC | 68 | 3 bases before 5'-end and 3'-end, thio modification | 21174.60 | |
| b(SE Q ID NO:1 06) | CGCCGCCCCGCTTCGCCGCCAGC CGCC | 27 | 3 bases before 5'-end and 3'-end, thio modification | 8176.24 | 39092.09 |
| c(SE Q ID NO:1 07) | GGCGGCAGGCGGCCATAGCCGTG GGCGCGCG | 31 | 3 bases before 5'-end and 3'-end, thio modification | 9741.25 | |

Table 33: D-4:

| Name | Sequence sense(5'-3') | Base number | Chemical modification | Molecular weight | Total molecular weight |
|---|---|---|---|---|---|
| a(SEQ ID NO: 108 ) | CGCGCGCCCAGGAGCGTTGGCCC GCGGCGTGGGCCGAAAAAGACCT GACTTCTATACTAAGTCTACGTCCC | 71 | 3 bases before 5'-end and 3'-end, thio modification | 21923.12 | |
| b(SEQ ID NO: 109 ) | CGCCGCGGGCCTTCGGGGCCAGC CGCC | 27 | 3 bases before 5'-end and 3'-end, thio modification | 8236.34 | 39780.63 |
| c(SEQ ID NO: 110 ) | GGCGGCAGGCCCCCATAGCCCTG GGCGCGCG | 31 | 3 bases before 5'-end and 3'-end, thio modification | 9621.17 | |

Table 34:D-5

| Name | Sequence sense(5'-3') | Base number | Chemical modification | Molecular weight | Total molecular weight |
|---|---|---|---|---|---|
| a(SEQ ID NO: 111) | CGCGCGCCCAGCAGCGTTCGC CCCGCCGCTGGGCCGAAAAAGA CCTGACTTCTATACTAAGTCTACG TCCC | 71 | 3 bases before 5'-end and 3'-end, thio modification | 21763.03 | 39880.64 |
| b(SEQ ID NO: 112) | GCGGCGGGGCGTTCGGCGGCA GGCGGC | 27 | 3 bases before 5'-end and 3'-end, thio mod ification | 8536.46 | |
| c(SEQ ID NO: 113) | GCCGCCAGCCGCCCATAGCGCT GGGCGCGCG | 31 | 3 bases before 5'-end and 3'-end, thio mod ification | 9581.15 | |

Table 35: D-6:

| Name | Sequence sense(5'-3') | Base number | Chemical modificatio n | Molecular weight | Total molecular weight |
|---|---|---|---|---|---|
| a(SEQ ID NO: 114) | CGCGCGCCCAGCAGCG TTCGGGGCGCCGC | 29 | 3 bases before 5'-end and 3'-end, thio modification | 8978.76 | 27594.69 |
| b(SEQ ID NO: 115) | GCGGCGCCCCGTTCGG CCGGCAGGCGGC | 28 | 3 bases before 5'-end and 3'-end, thio modification | 8705.57 | |
| c(SEQ ID NO:116) | GCCGCCAGCCGGCCCA TAGCGCTGGGCGCGCG | 32 | 3 bases before 5'-end and 3'-end, thio modification | 9910.36 | |

Table 36: D-7:

| Name | Sequence sense(5'-3') | Base number | Chemical mod ificatio n | Molecular weight | Total molecular weight |
|---|---|---|---|---|---|
| a(SEQ ID NO: 117) | CGCGCGCCCACGAGCGTT GCGGGCGCCGC | 29 | 3 bases before 5'-end and 3'-end, thio modification | 8978.76 | 26976.30 |
| b(SEQ ID NO: 118) | GCGGCGCCCGCTTCGGCG GCAGGCGGC | 27 | 3 bases before 5'-end and 3'-end, thio modification | 8416.39 | |
| c(SEQ ID NO: 119) | GCCGCCAGCCGCCCATAGC CGTGGGCGCGCG | 31 | 3 bases before 5'-end and 3'-end, thio modification | 9581.15 | |

Single strands of the above 7 groups of the conventional-sequence DNA nanoparticles are all commissioned to be synthesized by Suzhou Hongxun Company, herein:

D-1 is the conventional-sequence DNA nanoparticles formed after adding an extension sequence containing the EGFRapt target head (see an underlined part) on the basis of the above core sequences (8) (sequence a: 5'-GGAGCGTTGG-3', sequence b: 5'-CCTTCGCCG-3', and sequence c: 5'-CGGCCATAGCCC-3');
D-2 is the conventional-sequence DNA nanoparticles formed after adding an extension sequence containing the EGFRapt target head (see an underlined part) on the basis of the above core sequences (9) (sequence a: 5'-GGAGCGTTGG-3', sequence b: 5'-CCTTCGCCG-3', and sequence c: 5'-CGGCCATAGCCC-3');
D-3 is the conventional-sequence DNA nanoparticles formed after adding an extension sequence containing the EGFRapt target head (see an underlined part) on the basis of the above core sequences (10) (sequence a: 5'-CGAGCGTTGC-3', sequence b: 5'-GCTTCGCCG-3', and sequence c: 5'-CGGCCATAGCCG-3');
D-4 is the conventional-sequence DNA nanoparticles formed after adding an extension sequence containing the PSMAapt target head (see an underlined part) on the basis of the above core sequences (11) (sequence a: 5'-GGAGCGTTGG-3', sequence b: 5'-CCTTCGGGG-3', and sequence c: 5'-CCCCCATAGCCC-3');
D-5 is the conventional-sequence DNA nanoparticles formed after adding an extension sequence containing the PSMAapt target head (see an underlined part) on the basis of the above core sequences (12) (sequence a: 5'-GCAGCGTTCG-3', sequence b: 5'-CGTTCGGCG-3', and sequence c: 5'-CGCCCATAGCGC-3');
D-6 is the conventional-sequence DNA nanoparticles formed after adding an extension sequence without containing a target head structure on the basis of the above core sequences (13) (sequence a: 5'-GCAGCGTTCG-3', sequence b: 5'-CGTTCGGCC-3', and sequence c: 5'-GGCCCATAGCGC-3'); and
D-7 is the conventional-sequence DNA nanoparticles formed after adding an extension sequence without containing a target head structure on the basis of the above core sequences (14) (sequence a: 5'-CGAGCGTTGC-3', sequence b: 5'-GCTTCGGCG-3', and sequence c: 5'-CGCCCATAGCCG-3').

II. Self-assembly experiment steps:

(1) According to a molar ratio of 1:1:1, enabling the DNA single strands a, b and c to be simultaneously mixed and dissolved in DEPC treated water or TMS buffer solution;
(2) heating mixed solution to 95 DEG C, after keeping for 5 min, slowly cooling to a room temperature at a rate of 2 DEG C/min;
(3) enabling a product to be loaded on 8% (m/v) of non-denaturing PAGE gel and placed in TBM buffer solution under a condition of 4 DEG C, purifying a complex by 100 v of electrophoresis;
(4) cutting a target band and eluting in DNA elution buffer solution at 37 DEG C, after that, precipitating overnight in ethanol, volatilizing under reduced pressure and low temperature, to obtain a conventional-sequence DNA self-assembly product;
(5) electrophoresis analysis detection and laser scanning observation;
(6) electric potential detection;
(7) particle size measurement; and
(8) observing by a transmission electron microscope.

III. Self-assembly experiment result

(1) Electrophoresis detection result

A 2% agarose gel electrophoresis diagram of the 7 groups of the conventional-sequence DNA self-assembly products is shown in Fig. 7. From left to right, lanes 1 to 7 in Fig. 7 are successively: conventional -sequence DNA self-assembly products D-1, D-2, D-3, D-4, D-5, D-6 and D-7.
A 4% agarose gel electrophoresis diagram of the 7 groups of the conventional-sequence DNA self-assembly products is shown in Fig. 8. From left to right, lanes 1 to 7 in Fig. 8 are successively: conventional-sequence DNA self-assembly products D-1, D-2, D-3, D-4, D-5, D-6 and D-7.
It may be clearly seen from results of Fig. 7 and Fig. 8 that bands of the 7 groups of the conventional-sequence DNA self-assembly products are bright and clear, it is indicated that the 7 groups of the conventional-sequence DNA strands are all self-assembled to form stable nanoparticle structures. Herein two groups of the self-assembly structures D-6 and D-7 carry the EGFRapt or PSMAapt target head, and the molecular weights are slightly low, positions of bands thereof are apparently in front of other bands, actual and theoretical conditions are completely consistent, so the stability of the self-assembly structures is further proved.
It is indicated from the embodiment: on the base of combinations of these different DNA core sequences, while various functional extension fragments are added or the target head is linked at the same time, the DNA nanoparticles may also be successfully assembled, and also have functions such as drug loading, cell targeting, visibility and traceability (specifically described in Embodiment 6 and Embodiment 8).

(2) Electric potential measurement

Measurement method: preparing a potential sample (the self-assembly product is dissolved in ultrapure water) and putting into a sample pool, opening a sample pool cover of an instrument, and placing the instrument;
opening software, clicking a menu MeasUre€ManUal, so a manual measurement parameter setting dialog box appears;
setting a software detection parameter;
and then clicking Ok to complete the settings, after a measurement dialog box appears, clicking Start to start.
Measurement result: potential detection results of the 3 groups of the conventional-sequence DNA nanoparticles are as follows:

Table 37:

| Sample name | Detection times | Potential ZP (mV) |
|---|---|---|
| D-2 | 1 | -36.00 |
| | 2 | -35.80 |
| | 3 | -37.60 |
| Experiment result | -36.47 mV (±0.67mV) | |

Table 38:

| Sample name | Detection times | Potential ZP (mV) |
|---|---|---|
| D-6 | 1 | -31.70 |
| | 2 | -32.10 |
| | 3 | -31.90 |
| Experiment result | -31.90 mV (±0.2mV) | |

Table 39:

| Sample name | Detection times | Potential ZP (mV) |
|---|---|---|
| D-7 | 1 | -32.10 |
| | 2 | -31.70 |
| | 3 | -32.80 |
| Experiment result | -32.20 mV (±0.0.5mV) | |

It may be seen from the above potential detection data that the 3 groups of the conventional-sequence RNA self-assembly products have good stability, it is further indicated that the nanoparticles formed by the self-assembly of each conventional-sequence RNA have a relatively stable self-assembly structure.

(3) Particle size measurement

1. Preparing a potential sample (conventional-sequence DNA self-assembly product D-7) and putting into a sample pool, opening a sample pool cover of an instrument, and placing the instrument;
2. opening software, and clicking a menu, so a manual measurement parameter setting dialog box appears;
3. setting a software detection parameter; and then
4. clicking Ok to complete the settings, while a measurement dialog box appears, clicking Start to start, a DLS measurement value result of a hydrodynamic size of the self-assembly product D-7 is as follows:

Table 40:

| Sample name | Detection times | Particle size Z-Ave (d.nm) |
|---|---|---|
| 1 | 1 | 10.76 |
| | 2 | 13.9 |
| | 3 | 10.36 |
| Experiment result | 12.33 d.nm(±1.57 d.nm) | |

(4) Observed result of transmission electron microscope

The above conventional-sequence DNA self-assembly product D-7 is irradiated by a transmission electron microscope, and steps are as follows:

1. taking a drop of a sample and suspending on 400 meshes of a carbon-coated film copper grid, keeping for 1 min at a room temperature;
2. absorbing liquid by filter paper;
3. staining for 1 min by 2% uranyl acetate;
4. absorbing by the filter paper, and drying at the room temperature; and
5. observing and taking a picture by a transmission electron microscope JEM-1400 at 120 kv.

The result is as shown in Fig. 9, it may be apparently seen from the figure that the above conventional-sequence DNA self-assembly product D-7 is an integral structure, and it may be clearly seen that it has a T-type structure.

**Doxorubicin (Dox) loading experiment**

Embodiment 5

Chemical loading:

I. Experiment material and experiment method

1. Experiment materials and reagents:

(1) Nucleic acid nanoparticles: RNA nanoparticles from Embodiment 1.
(2) DEPC treated water: Biyuntian Company.
(3) PBS buffer solution: cellgro.
(4) 4% paraformaldehyde.
(5) Doxorubicin (Dox).
(6) Chloroform: Beijing University of Chemical Technology.
(7) Anhydrous ethanol: Beijing University of Chemical Technology.

2. Experiment method:

(1) Accurately weighing the doxorubicin (5.0mg, 8.6μmol, 40eq.) and dissolving in the DEPC treated water (1.8 mL) and the PBS buffer solution (2.1 mL), adding 4% paraformaldehyde aqueous solution (0.4mL) under cooling of an ice-water bath and uniformly mixing, enabling the mixed solution to be totally uniformly mixed with the RNA nanoparticles (215 nmol), and reacting for 72 hours at 4 DEG C under a dark condition.
(2) Taking 10μL of reaction solution and diluting for 10 times, using 50 μM of the doxorubicin aqueous solution and 310 ng/μL of the RNA nanoparticles as controls, and performing HPLC analysis according to equal volume injection. According to a peak area ratio of each component, it may be adjusted that reaction conversion is basically completed.
(3) Extracting the reaction solution by the chloroform (10mLx3), and then adding 10 times of the anhydrous ethanol in volume, after uniformly mixing, keeping at 4 DEG C in the dark and enabling a product to be adequately precipitated (4 hours). Centrifuging, transferring supernatant, and washing the solid product again by the ethanol, evaporating a solvent under reduced pressure and lower temperature, to obtain a loading product which is a dark red solid.
(4) Taking a small amount of the product and dissolving in the DEPC treated water, loading into 8% PAGE gel, and electrophoresing in the TBM buffer solution at 100 V for 1 hour under a condition of 4 DEG C, herein an electrophoresis result is shown in Fig. 10. In Fig. 10, from left to right, lanes 1 to 5 are respectively: 1) 20bp DNA ladder, 2-4) RNA nanoparticle blank particles, and 5) a doxorubicin-loaded product. It may be seen from Fig. 10 that a band of the doxorubicin-loaded product is located a little behind the RNA nanoparticle blank particles.
(5) Loading rate calculation:

1. preparing a known concentration of doxorubicin-PBS standard solution: 2 μM, 4 μM, 6 μM, 8 μM, and 10 μM, 100 μL each;
2. enabling the doxorubicin-loaded product to be dissolved in 100 μL of the PBS;
3. putting the standard solution and the doxorubicin-loaded product into a PCR plate, heating at 85 DEG C for 5 min, and then cooling to the room temperature;
4. measuring an absorbance of the doxorubicin at 492 nm by using a microplate reader, drawing a standard curve (as shown in Fig. 11), and calculating the molar concentration of the doxorubicin in the loaded product;
5. measuring an absorbance of RNA at 260 nm by using a spectrophotometer, to obtain the mass concentration of the doxorubicin-loaded product contained in each sample; and
6. according to the doxorubicin molar concentration and the mass concentration of the doxorubicin-loaded product obtained by measuring, calculating a loading rate.

A specific calculation process is as follows:

$C_{RNAh}$-1 =9.5μg/μL, $M_{RNAh}$≈30000, 100μL; Cdoxorubicin -1 =8.033μM, 100μL; $C_{RNAh}$-2=1.21 μg/μL, $M_{RNAh}$=30000, 100μL; Cdoxorubicin -1 =9.200μM, 100μL;

$$N\text{-}1=\frac{8.033\times 10\text{-}6\times 100\times 10\text{-}6}{0.95\times 100\times 10\text{-}6/30000}=25.4;\quad N\text{-}2=\frac{9.200\times 10\text{-}6\times 100\times 10\text{-}6}{1.21\times 100\times 10\text{-}6/30000}=22.8;$$

An average value of N-1 and N-2 is taken so that the loading rate of RNAh-doxorubicin is about 24, and it means that about 24 doxorubicin molecules may be loaded on each nucleic acid nanoparticle carrier.

Physical loading:

1) enabling a mass ratio of the doxorubicin and the RNA nanoparticles to be 1:1;
2) weighing 0.1 mg of a doxorubicin original drug and dissolving in 50 μL of DMSO, and then adding 300 μL of the PBS and mixing uniformly;
3) enabling RNA particles to be dissolved in 200 μL of the DEPC treated water, and adding into the doxorubicin-PBS mixed solution, uniformly mixing, and adjusting a pH to be about 7.5;
4) putting the whole solution in a water bath kettle at 55 DEG C, and reacting for 3h;
5) after the reaction, directly adding 10 times of anhydrous ethanol in volume, and precipitating at 4 DEG C for 4 h; and
6) washing for 4 times with 10 times of the anhydrous ethanol, and transferring to 1.5 mL of a EP tube. Subsequently, measuring a loading rate, herein the measuring method is the same as above, and a measuring result is that a doxorubicin loading rate is 15.5.

It is indicated from Embodiment 5 that the RNA nanoparticles (in Embodiment 1) with the extension fragment, the target head and the fluorescein have a function of loaded drugs, and may achieve the drug loading in modes of physical insertion and covalent linkage (paraformaldehyde-solvent covalence).

Embodiment 6

According to the chemical loading method (unless otherwise specified, the method is the same as that of Embodiment 5) of Embodiment 5, the above DNA nanoparticles in Embodiment 1, the RNA nanoparticles formed by the self-assembly of the R-1, R-2, R-3, R-4, R-5, R-6 and R-7 in Embodiment 2, and the DNA nanoparticles formed by the self-assembly of the D-2, D-6 and D-7 in Embodiment 4 are respectively used as doxorubicin loading carriers, doxorubicin loading rates measured are respectively as follows:
The doxorubicin loading rate of the DNA nanoparticles in Embodiment 1 is 300 (in this method, the doxorubicin is 1.2 mg, the DEPC treated water is 0.5 mg, the PBS buffer solution is 8.5 mL, the 4% paraformaldehyde aqueous solution is 1 mL, the DNA nanoparticles are 2.5 nmol, and the DNA nanoparticles are dissolved in 20μl of water).
The doxorubicin loading rate of RNA nanoparticles R-1 is 3.5.
The doxorubicin loading rate of RNA nanoparticles R-2 is 2.4.
The doxorubicin loading rate of RNA nanoparticles R-3 is 4.8.
The doxorubicin loading rate of RNA nanoparticles R-4 is 3.5.
The doxorubicin loading rate of RNA nanoparticles R-5 is 12.5.
The doxorubicin loading rate of RNA nanoparticles R-6 is 2.8.
The doxorubicin loading rate of DNA nanoparticles D-2 is 14.
The doxorubicin loading rate of DNA nanoparticles D-6 is 11.
The doxorubicin loading rate of DNA nanoparticles D-7 is 10.

**Cell binding ability of RNA nanoparticles detected by Fluorescence Activated Cell Sorter (FACS) Experiment**

Embodiment 7

I. Experiment material and experiment method:

1. Samples to be tested as shown in Table 41:

Table 41:

| Nanoparticles | | MW | Dissolution solvent |
|---|---|---|---|
| 1 | RNAh | 28083 | PBS |
| 2 | RNAh-Biotin-quasar670 | 29552.6 | PBS |
| 3 | RNAh-Biotin-quasar670-Dox | 41232.6 | DMSO |
| Note: RNAh in the table refers to control nanoparticles without Biotin modification in the RNA nanoparticles formed by the self-assembly in Embodiment 1, RNAh-Biotine-quasar670 refers to nanoparticles formed after modifying quasar670 fluorescein at the 5'-end of the RNA nanoparticles formed by the self-assembly in Embodiment 1, and RNAh-Biotine-quasar670-Dox refers to nanoparticles formed after further loading a doxorubicin drug (the chemical loading in Embodiment 5). | | | |

2. Experiment reagents used and sources thereof are as follows:
RPMI-1640 medium (Gibco, C11875500BT-500mL); Fetal bovine serum (FBS) (ExCell Bio, FNA500-500mL); Penicillin/Streptomycin (PS) (Gibco, 15140-122 -100mL); PBS buffer solution (Gibco, C20012500BT-500mL); Trypsin-EDTA (Stemcell, 07901-500mL); and DMSO (Sigma, D5879-1L).
3. Experiment instruments used are as follows:
Inverted Microscope (Olympus IX71, TH4-200); and Flow Cytometer (Life Science, Attune NxT).
4. Experiment method:

(1) Culturing HepG2 cells at 37 DEG C and in 5% CO2 by using a RPMI1640 + 10% FBS + 1% PS medium.
(2) Digesting the HepG2 cells by trypsin, and washing once with the PBS.
(3) Incubating $2x10^5$ cells together with RNAh, RNAh-Biotin-quasar670, RNAh-Biotin-quasar670-Dox nanoparticles at 37 DEG C and in the 5% $CO_2$ for 1h, herein each sample has two concentrations 200nM and 400nM, and each sample has 3 replicates at each concentration.
(4) After the cells are washed with the PBS, resuspending in the PBS buffer solution and testing by using the FACS instrument.
(5) Collecting data and making statistical analysis.

II. Experiment result

An experiment result is shown in Table 42, Fig. 12 and Fig. 13.

Table 42: Fluorescence-positive HepG2 cell (%) Mean±SEM (n=3)

| | | |
|---|---|---|
| Cell control | HepG2 Cell | 0.5%± 0.2% |
| Nanoparticle control | RNAh | - |
| | RNAh-Biotin-quasar670 | - |
| | RNAh-Biotin-quasar670-Dox | - |
| HepG2+nanoparticle 200nM | RNAh | 0.3%± 0.0% |
| | RNAh-Biotin-quasar670 | 96.4%± 0.2% |
| | RNAh-Biotin-quasar670-Dox | 80.5%± 0.7% |
| HepG2+nanoparticle 400nM | RNAh | 0.6%± 0.2% |
| | RNAh-Biotin-quasar670 | 98.5%± 0.2% |
| | RNAh-Biotin-quasar670-Dox | 87.1%± 0.3% |

In Fig. 12, A corresponds to a HepG2 cell control group, B corresponds to 200 nM concentration of RNAh control nanoparticles, C corresponds to 200 nM concentration of RNAh-Biotin-quasar670 nanoparticles, D corresponds to 200 nM concentration of RNAh-Biotin-quasar670-Dox nanoparticles, E corresponds to 400 nM concentration of RNAh control nanoparticles, F corresponds to 400 nM concentration of RNAh-Biotin-quasar670 nanoparticles, and G corresponds to 400 nM concentration of RNAh-Biotin-quasar670-Dox nanoparticles.

It may be seen from Table 42 and Fig. 12 that the RNA nanoparticles which simply do not modify the target head do not have cell targeting properties, and may be bound with the HepG2 cells after being loaded with the biotin. In addition, the FACS result in Fig. 12 shows that RNAh-Biotin-quasar670 and RNAh-Biotin-quasar670-Dox nanoparticles have a strong binding ability to the HepG2 cells (P<0.0001).

Fig. 13 shows a nanoparticle and HepG2 cell binding and internalization result detected by a microscope. The cell binding and internalization experiment result shows that RNAh-Biotin-quasar670 and RNAh-Biotin-quasar670-Dox nanoparticles may be bound and internalized with the HepG2 cells (herein, after the doxorubicin-loaded RNAh-Biotin-quasar670-Dox nanoparticles are co-incubated with the HepG2 cells, the cells are apparently stained red, and a color is deepened along with the increase of the concentration of the RNAh-Biotin-quasar670-Dox nanoparticles and time, it may be seen that the drug-loaded RNA nanoparticles have a relatively strong ability to bind and internalize with the HepG2 cells. The RNAh-Bio-quasar670 also has the ability to bind and internalize with the HepG2 cells, because it does not contain the Dox, it may not be stained red).

Embodiment 8

I. Experiment material and experiment method

1. Samples to be tested are shown in Table 43:

Table 43:

| Serial number | Sample name | Loading amount | Weight Mg Original drug/ carrier particles | Original drug molecular weight | Indications/cell lines |
|---|---|---|---|---|---|
| 1 | DOX-D-1 -EGFR | 4 | 0.03/0.5 | 580/39098.2 | Glioma-U87-MG |
| 2 | DOX-D-2 -EGFR | 4 | 0.03/0.5 | 580/39096.5 | Human breast cancer cell MDA-MB-231 |
| 3 | DOX-D-5 -PSMA | 4 | 0.029/0.5 | 580/39885.9 | Lung cancer-HCC-78 |
| Note: DOX-D-1-EGFR refers to the nanoparticles formed after the doxorubicin (the loading step is the same as Embodiment 5, similarly hereinafter) is loaded in the DNA nanoparticles D-1 formed by the self-assembly in the previous Embodiment 4 (EGFR is loaded in the D-1 by itself, and it is expressed as DOX-D-1-EGFR here to clarify the target head type and doxorubicin loading, similarly hereinafter); DOX-D-2-EGFR refers to the nanoparticles after the doxorubicin is loaded in the DNA nanoparticles D-2 formed by the self-assembly in the previous embodiment; and DOX-D-5-PSMA refers to the nanoparticles after the doxorubicin is loaded in the DNA nanoparticles D-5 formed by the self-assembly in the previous embodiment. | | | | | |

2. Cell information is shown in Table 44:

Table 44:

| Serial number | Cell name | Growth characteristic | Cell type | Culture medium |
|---|---|---|---|---|
| 1 | HCC-78 | Adherent | Lung cancer | RPMI1640+10%FBS |
| 2 | U87MG | Adherent | Glioma | (MEM+0.01 mM NEAA)+10%FBS |
| 3 | MDA-MB-23 1 | Adherent | Breast cancer | RPM11640+10%FBS |

3. Experiment reagents used and sources thereof are as follows:

RPMI-1640 medium (YY0167-500MI);
MEM (YS4150-500mL);
MEM NEAA (100x) (GBICO,Cat#1872982); and

Fetal Bovine Serum (FBS) (GBICO, Cat#10099141).

4. Experiment instruments used are as follows:

Flow cytometer Guava EasyCyte 8HT (Millipore); and
Multi-label microplate detector SpectraMax, MD, 2104-0010A.

5. Experiment method:
5.1. Cell culture

a) Cells were resuscitated in a corresponding medium, and cultured in a cell incubator under 37 DEG C and 5% $CO_2$.

b) While the cells reached a logarithmic growth phase (about 80% of a confluence rate) in a T75 cell culture flask, the original medium was replaced with a medium which did not contain a folic acid and a biotin.

5.2. Binding experiment
a) The cells were collected and counted on the first day, and spread in a 24-well plate at a density of $2\times10^5$ cell/well.
b) The next day, samples were diluted with the PBS. The PBS was used to dilute all samples to 100 μM, 1 μM of solution was prepared, and it was detected whether a fluorophore (doxorubicin: Ex=480nm, Em=580nm;) normally emitted light on a microplate reader.
c) The cells were washed twice with the PBS.
d) The nanoparticles dissolved in the culture medium were added, and the cells were incubated in a $CO_2$ incubator at 37 DEG C for 16 hours. The concentration of the nanoparticles was 2 μM, and an order of the samples was shown in Table 45 below.

Table 45:

| Serial number | Sample name | Fluorophore | Detection wavelength | Final concentration | Cell line |
|---|---|---|---|---|---|
| 1 | DOX-D-1-EGFR | Cy5 | Ex=480nm Em=580nm | 2μM | U87MG |
| 2 | DOX-D-2-EGFR | Cy5 | | 2μM | MDA-MB-231 |
| 3 | DOX-D-5-PSMA | Cy5 | | 2μM | HCC-78 |

e) The cells were washed twice with the PBS.
f) Cells digested by a trypsin were collected and the cells were washed twice with the PBS.
g) The cells washed with the PBS were resuspended in 400 μL of the PBS, and transferred to 5 mL of a flow cytometry tube.
h) The sample needed to be kept in a dark place before being loaded on a flow cytometer.
i) It was detected by the flow cytometer. The doxorubicin was used to detect a cell fluorescence intensity at Ex=480nm, Em=580nm (yellow channel).
j) FACS data was analyzed with FlowJo software.
k) A gate was set based on the background fluorescence intensity of a blank cell group, and a binding ratio of each DNA nanoparticle and the cells was analyzed.

II. Experiment result

Experiment results are shown in Table 46, 47 and 48.

Table 46: Fluorescence detection result of sample microplate reader

| Serial number | Sample | Doxorubicin, Ex=480nm, Em=580nm |
|---|---|---|
| | PBS | 4.37 |

(continued)

| Serial number | Sample | Doxorubicin, Ex=480nm, Em=580nm |
|---|---|---|
| 1 | DOX-D-1-EGFR | 280.178 |
| 2 | DOX-D-2-EGFR | 260.175 |
| 3 | DOX-D-5-PSMA | 295.964 |

Table 47: Flow cytometric sample and cell binding rate

| Cell line | Sample name | Binding time | Binding rate % |
|---|---|---|---|
| | | | Final concentration ( 2μM) |
| U87MG | Blank | 16h | 2.36 |
| U87MG | DOX-D-1-EGFR | 16h | 100 |
| MDA-MB-231 | Blank | 16h | 2.36 |
| MDA-MB-231 | DOX-D-2-EGFR | 16h | 100 |
| HCC-78 | Blank | 16h | 2.39 |
| HCC-78 | DOX-D-5-PSMA | 16h | 100 |

Table 48: Flow cytometric MFI

| Cell line | Sample name | Binding time | Geometric Mean : FL2-H |
|---|---|---|---|
| | | | Final concentration ( 2μM) |
| U87MG | Blank | 16h | 11.8 |
| U87MG | DOX-D-1-EGFR | 16h | 1232 |
| MDA-MB-231 | Blank | 16h | 11.0 |
| MDA-MB-231 | DOX-D-2-EGFR | 16h | 1071 |
| HCC-78 | Blank | 16h | 11.3 |
| HCC-78 | DOX-D-5-PSMA | 16h | 1387 |

It may be seen from the data results in the above Tables 47 and 48 that: the binding ability of DOX-D-1-DNAh-EGFR to U87MG cells is very strong, while the administration concentration is 2 μM and the administration time is 16 h, the binding efficiency is 100%. The binding ability of DOX-D-2-EGFR to MDA-MB-231 cells is very strong, while the administration concentration is 2 μM and the administration time is 16 h, the binding efficiency is 100%. The binding ability of DOX-D-3-EGFR to HCC-78 cells is very strong, while the administration concentration is 2 μM and the administration time is 16 h, the binding efficiency is 100%.

For other nucleic acid nanoparticles (including RNA nanoparticles and remaining DNA nanoparticles), because they all carry or may carry the target head EGFRapt or PSMAapt the same as DOX-D-1-DNAh-EGFR, DOX-D-2-EGFR or DOX-D-5-PSMA in the mode of adding the extension fragment, both have the same binding efficiency as the corresponding cells. In addition, they all carry the same drug loading sequence (GC loading site sequence) as DOX-D-1-DNAh-EGFR, DOX-D-2-EGFR or DOX-D-5-PSMA, so they all have the equivalent drug loading function.

**Stability detection of nucleic acid nanoparticles in serum**

Embodiment 9

I. Experiment material and experiment method

1. Sample to be tested: RNA nanoparticles prepared in Embodiment 1 dissolved in PBS solution
2. Experiment reagents:
RPMI-1640 medium (Gibco, C11875500BT-500mL); Fetal bovine serum (FBS) (ExCell Bio, FNA500-500mL); Penicillin/Streptomycin (PS) (Gibco, 15140-122 -100mL); PBS buffer solution (Gibco, C20012500BT-500mL); Novex TM Tris-Glycine Native Sample Buffer (2X) (Invitrogen, LC2673-20mL); Novex TM 8% Tris-Glycine Mini Gels (Invitrogen, XP00080BOX-1.0mm); Tris-Glycine Native Running buffer (10x) (Life science, LC2672-500mL); and G250 staining solution (Beyotime, P0017-250mL).
3. Experiment instruments:
Spectrophotometer (Thermo, ND2000C); Mini Gel Tank (Invitrogen, PS0301); and Imaging System (Bio-Rad, ChemiDoc MP).
4. Experiment method:

(1) 350 μl of the PBS was absorbed into a RNA nanoparticle sample, and adequately mixed uniformly.
(2) 2 μM of the RNA nanoparticles were incubated in the RPMI 1640 medium containing 10% serum.
(3) After being incubated at 37 DEG C for 10 min, 1 h, 12 h, and 36 h, samples were taken respectively.
(4) After NanoDrop used for quantification, 200 ng of the RNA nanoparticlesnanoparticle were taken, the same volume of Tris-Glycine SDS sample buffer solution (2X) was added, and adequately mixed uniformly.
(5) A piece of Novex™ 8% Tris-Glycine Mini gel was taken, the samples were loaded in order, a program was set in 200 V, 30 min, and electrophoresis was started.
(6) After the electrophoresis was over, G250 staining was performed, it was placed on a horizontal shaker for 30 min to 1 h, and pictures were taken for imaging.

II. Experiment result

Table 49: RNA quantification result and sampling loading volume

| Sample | RNA nanoparticle (ng/μL) | 200ng RNA nanoparticle (μL) | Buffer solution (μL) |
|---|---|---|---|
| 0 | 178.8 | 1.12 | 1.12 |
| 10 min | 193.5 | 1.03 | 1.03 |
| 1 h | 176.0 | 1.14 | 1.14 |
| 12 h | 175.5 | 1.14 | 1.14 |
| 36 h | 214.8 | 0.93 | 0.93 |

The results of electrophoresis detection are shown in Fig. 14 and Fig. 15. Herein, Fig. 14 shows the electrophoresis results of 8% non-denaturing gel (Coomassie Blue program), and Fig. 15 shows the electrophoresis results of 8% non-denaturing gel (Stain Free Gel program). The serum stability test results of RNA nanoparticle show that: the non-denaturing gel results (Fig. 14 and Fig. 15) at 10 min, 1 h, 12 h and 36 h show that there is no significant difference between the bands of the RNA nanoparticle samples at different times, it is indicated that the RNA nanoparticle are relatively stable in the 1640 medium of the 10% FBS without significant degradation.

**Cytotoxicity research of RNA nanoparticles in HepG2 cells**

Embodiment 10

I. Experiment material and experiment method

1. Samples to be tested are three samples in Embodiment 7.
2. Experiment reagents:
RPMI-1640 medium (Gibco, C11875500BT-500mL); Fetal bovine serum (FBS) (ExCell Bio, FNA500-500mL); Penicillin/Streptomycin (PS) (Gibco, 15140-122 -100mL); PBS buffer solution (Gibco, C20012500BT-500mL); Trypsin-EDTA (Stemcell, 07901-500mL); DMSO (Sigma, D5879-1L); Dox (HISUN Pharm, H33021980-10mg); and CellTiter-Glo Luminescent Cell Viability Assay kit (CTG) (Promega, G7572-100mL).
3. Experiment instruments:
Inverted Microscope (Olympus IX71, TH4-200); and 96-well Plate Reader (Molecular Devices, Flexstation 3).
4. Experiment method:

(1) A RPMI1640 + 10% FBS + 1% PS medium was used to culture HepG2 cells under 37 DEG C and 5% $CO_2$.
(2) The HepG2 cells were digested by a trypsin, 100 μL of 5000 cells per well was inoculated in a 96-well plate, and cultured overnight under 37 DEG C and 5% $CO_2$.
(3) Cell supernatant was removed on the second day, a sample to be tested was diluted with the culture medium, 200 nM of RNAh, RNAh-Biotine, RNAh-Dox and Dox were respectively added, 100 μL each was added to the plated cells, and each sample was repeated for 4 times.
(4) After being cultured for 72 hours, 100 μL of a CTG reagent was added to each well, shaking was performed for 2 min, and standing was performed at a room temperature for 10 minutes, and a whole process was performed in the dark.
(5) Finally Soft Max Pro5 software was used for reading.

II. Experiment result:

Table 50: HepG2 cell proliferation (%) Mean±SEM (n=4)

| PBS | DMSO | Dox | RNAh | RNAh-Biotin-quasar670 | RNAh-Biotin-quasar670-Dox |
|---|---|---|---|---|---|
| 100% ± 2.37% | 102.28% ± 4.18% | 39.56% ± 1.39% | 92.29% ± 3.31% | 92.90% ± 4.61% | 29.27% ± 0.99% |

Experiment results are shown in Table 50 and Fig. 16. In Fig. 16, a corresponds to a cell proliferation result of the PBS, b corresponds to a cell proliferation result of the DMSO, c corresponds to a cell proliferation result of the Dox (doxorubicin), d corresponds to a cell proliferation result of the RNAh, e corresponds to a cell proliferation result of the RNAh-Biotin-quasar670, and f corresponds to a cell proliferation result of the RNAh-Biotin-quasar670-Dox.
It may be seen from Table 50 and Fig. 16 that the CTG results show that 200 nM of drug-loaded nanoparticles RNAh-Biotine-Dox have apparent cytotoxicity (P<0.0001) to the HepG2 cells, and 200 nM of the RNAh-Biotine is non-cytotoxic to the HepG2 cells.

**Assembly of nucleic acid nanoparticles**

Embodiment 11

I. 7 groups of extension fragment deformation + core short-sequence RNA nanoparticle carriers:

(1) 7 groups of three polynucleotide base sequences for forming extension fragment deformation + core short-sequence RNA nanoparticles:

Table 51: R-15:

| Name | Sequence sense(5’-3’) | Base number | Chemical modification | Molecular weight | Total molecular weight |
|---|---|---|---|---|---|
| a(SEQ ID NO: 120) | GCGGCGAGCGGCGAGGAGCGUUGGGGCCGGAGGCCGG | 37 | 3’-end is linked with a biotin (Bio); bases C and U, and 2’F modification | 12668.8 | |
| b(SEQ ID NO: 121) | CCGGCCUCCGGCCCCUUCGGGGCCAGCCGCC | 31 | bases C and U, and 2’F modification | 9866.8 | 33713 |
| c(SEQ ID NO: 122) | GGCGGCAGGCCCCCAUAGCCCUCGCCGCUCGCCGC | 35 | bases C and U, and 2’F modification | 11177.4 | |

Table 52: R-16:

| Name | Sequence sense(5’-3’) | Base number | Chemical modification | Molecular weight | Total molecular weight |
|---|---|---|---|---|---|
| a (SEQ ID NO: 123) | GCGGCGAGCGGCGAGCAGCGUUCGGGCCGGAGGCCGG | 37 | 3’-end is linked with a biotin (Bio); bases C and U, and 2’F modification | 12591.6 | |
| b (SEQ ID NO: 124) | CCGGCCUCCGGCCCGUUCGCCGCCAGCCGCC | 31 | bases C and U, and 2’F mod ificatio n | 9827.6 | 33709.8 |
| c(SEQ ID NO: 125) | GGCGGCAGGCGGCCAUAGCGCUCGCCGCUCGCCGC | 35 | bases C and U, and 2’F modification | 11290.6 | |

Table 53: R-17:

| Name | Sequence sense(5’-3’) | Base number | Chemical modification | Molecular weight | Total molecular weight |
|---|---|---|---|---|---|
| a (SEQ ID NO: 126) | GCGGCGAGCGGCGAGGAGCGUUGGGGCCGGAGGCCGG | 37 | 3’-end is linked with a biotin (Bio); bases C and U, and 2’F modification | 12668.9 | |
| b (SEQ ID NO: 127) | CCGGCCUCCGGCCCCUUCGCCGCCAGCCGCC | 31 | bases C and U, and 2’F modification | 9790.6 | 33713 |
| c(SEQ ID NO: 128) | GGCGGCAGGCGGCCAUAGCCCUCGCCGCUCGCCGC | 35 | bases C and U, and 2’F modification | 11253.5 | |

Table 54:R-18:

| Name | Sequence sense(5'-3') | Base number | Chemical modification | Molecular weight | Total molecular weight |
|---|---|---|---|---|---|
| a(SEQ ID NO; 129) | GCGGCGAGCGGCGAGCAGCG UUCGGGCCGGAGGCCGG | 37 | 3'-end is linked with a biotin (Bio); bases C and U, and 2'F modification | 12591.6 | 33709.8 |
| b(SEQ ID NO: 130) | CCGGCCUCCGGCCCGUUCGG CGCCAGCCGCC | 31 | bases C and U, and 2'F modification | 9865.7 | |
| c(SEQ ID NO: 131 ) | GGCGGCAGGCGCCCAUAGCG CUCGCCGCUCGCCGC | 35 | bases C and U, and 2'F mod ificatio n | 11252.5 | |

Table 55: R-19:

| Name | Sequence sense(5'-3') | Base number | Chemical modification | Molecular weight | Total molecular weight |
|---|---|---|---|---|---|
| a(SEQ ID NO: 132) | GCGGCGAGCGGCGAGCAGCG UUCGGGCCGGAGGCCGG | 37 | 3'-end is linked with a biotin (Bio); bases C and U, and 2'F modification | 12591.6 | 33709.8 |
| b(SEQ ID NO: 133) | CCGGCCUCCGGCCCGUUCGG CCCCAGCCGCC | 31 | bases C and U, and 2'F modification | 9827.6 | |
| c(SEQ ID NO: 134) | GGCGGCAGGGGCCCAUAGCG CUCGCCGCUCGCCGC | 35 | bases C and U, and 2'F modification | 11290.6 | |

Table 56: R-20:

| Name | Sequence sense(5'-3') | Base number | Chemical modification | Molecular weight | Total molecular weight |
|---|---|---|---|---|---|
| a(SEQ ID NO: 135) | GCGGCGAGCGGCGACGAGCG UUGCGGCCGGAGGCCGG | 37 | 3'-end is linked with a biotin (Bio); bases C and U, and 2'F modification | 12591.6 | 33709.8 |
| b(SEQ ID NO: 136) | CCGGCCUCCGGCCGCUUCGC CGCCAGCCGCC | 31 | bases C and U, and 2'F modification | 9827.6 | |
| c(SEQ ID NO: 137) | GGCGGCAGGCGGCCAUAGCC GUCGCCGCUCGCCGC | 35 | bases C and U, and 2'F modification | 11290.6 | |

Table 57: R-21:

| Name | Sequence sense(5'-3') | Base number | Chemical modification | Molecular weight | Total molecular weight |
|---|---|---|---|---|---|
| a(SEQ ID NO: 138) | GCGGCGAGCGGCGACGAGCG UUGCGGCCGGAGGCCGG | 37 | 3'-end is linked with a biotin (Bio); bases C and U, and 2'F modification | 11290.6 | 32408.8 |
| b(SEQ ID NO: 139) | CCGGCCUCCGGCCGCUUCGG CGCCAGCCGCC | 31 | bases C and U, and 2'F modification | 9865.7 | |
| c(SEQ ID NO: 140) | GGCGGCAGGCGCCCAUAGCC GUCGCCGCUCGCCGC | 35 | bases C and U, and 2'F modification | 11252.5 | |

II. Self-assembly experiment steps:

(1) according to a molar ratio of 1:1:1, enabling the RNA single strands a, b and c to be simultaneously mixed and dissolved in DEPC treated water or TMS buffer solution;
(2) heating mixed solution to 80 DEG C, after keeping for 5 min, slowly cooling to a room temperature at a rate of 2 DEG C/min;
(3) enabling a product to be loaded on 8% (m/v) of non-denaturing PAGE gel and placed in TBM buffer solution under a condition of 4 DEG C, purifying a complex by 100 v of electrophoresis;
(4) cutting a target band and eluting in RNA elution buffer solution at 37 DEG C, after that, precipitating overnight in ethanol, and volatilizing under reduced pressure and low temperature;
(5) electrophoresis analysis detection and laser scanning observation;
(6) electric potential detection;
(7) particle size measurement; and
(8) Tm value measurement.

III. Self-assembly experiment result

(1) Electrophoresis detection

Main reagents and instruments are as follows:

Table 58:

| Reagent name | Article number | Manufacturer |
|---|---|---|
| 6xDNA Loading buffer | TSJ010 | Qingke Biotechnology Co.,Ltd |
| 20bp DNA Ladder | 3420A | TAKARA |
| 10000*SolarGelRed nucleic acid dye | E1020 | solarbio |
| 8% non-denaturing PAGE gel | / | Self-made |
| 1 × TBE Buffer (RNA enzyme-free) | / | Self-made |

Table 59:

| Name | Model | Manufacturer |
|---|---|---|
| Electrophoresis apparatus | PowerPac Basic | Bio-rad |
| Vertical electrophoresis | Mini PROTEAN Tetra Cell | Bio-rad |

(continued)

| Name | Model | Manufacturer |
|---|---|---|
| Discoloration shaker | TS-3D | orbital shaker |
| Gel imager | Tanon 3500 | Tanon |

Operations:

① The RNA nanoparticles were diluted with ultrapure water by using a method shown in Table 60 below.

Table 60:

| | Measured concentration (μg/mL) |
|---|---|
| R-15 | 165.937 |
| R-16 | 131.706 |
| R-17 | 144.649 |
| R-18 | 164.743 |
| R-19 | 126.377 |
| R-20 | 172.686 |
| R-21 | 169.455 |

② 10μL (500ng) of the processed sample was taken and uniformly mixed with 2μL of 6xDNA Loading Buffer, it was operated on ice, and a label was made.
③A 8% non-denaturing PAGE gel was taken, a piece of the gel was applied to samples with different incubation times, 12 μL of the processed samples were all loaded, and a program was set to run the gel at 100V for 40 minutes.
④ After running the gel, dyeing was performed, it was placed on a horizontal shaker for 30 minutes, and pictures were taken for imaging.

Detection result:

Non-denaturing PAGE gel running results of the 7 groups of extension fragment deformation + core short-sequence RNA self-assembly products are shown in Fig. 17. Lanes 1 to 7 in Fig. 17 from left to right are successively: the 7 groups of extension fragment deformation + core short-sequence RNA self-assembly products R-15, R-16, R-17, R-18, R-19, R-20 and R-21.
It may be clearly seen from the results in Fig. 17 that the bands of the 7 groups of extension fragment deformation + core short-sequence RNA self-assembly products are bright and clear, it is indicated that the 7 groups of extension fragment deformation + core short-sequence RNA strands are all self-assembled completely, to form a stable nanoparticle structure.

(2) Electric potential measurement

Measurement method: preparing a potential sample (the self-assembly product is dissolved in ultrapure water) and putting into a sample pool, opening a sample pool cover of an instrument, and placing the instrument;
opening software, clicking a menu MeasUre€ManUal, so a manual measurement parameter setting dialog box appears;
setting a software detection parameter;
and then clicking Ok to complete the settings, after a measurement dialog box appears, clicking Start to start.
Measurement result: potential detection results of the 7 groups of the extension fragment deformation + core short-sequence RNA nanoparticles at 25 DEG C are as follows:

Table 61:

| Sample name | Detection times | Potential ZP (mV) |
|---|---|---|
| R-15 | 1 | -22.50 |
| | 2 | -23.90 |
| | 3 | -23.30 |
| Experiment result | -23.23 mV | |

Table 62:

| Sample name | Detection times | Potential ZP (mV) |
|---|---|---|
| R-16 | 1 | -21.10 |
| | 2 | -19.80 |
| | 3 | -21.90 |
| Experiment result | -20.93 mV | |

Table 63:

| Sample name | Detection times | Potential ZP (mV) |
|---|---|---|
| R-17 | 1 | -24.90 |
| | 2 | -20.90 |
| | 3 | -24.70 |
| Experiment result | -23.50 mV | |

Table 64:

| Sample name | Detection times | Potential ZP (mV) |
|---|---|---|
| R-18 | 1 | -20.80 |
| | 2 | -21.30 |
| | 3 | -21.70 |
| Experiment result | -21.27 mV | |

Table 65:

| Sample name | Detection times | Potential ZP (mV) |
|---|---|---|
| R-19 | 1 | -16.80 |
| | 2 | -16.90 |
| | 3 | -21.20 |
| Experiment result | -18.30 mV | |

Table 66:

| Sample name | Detection times | Potential ZP (mV) |
|---|---|---|
| R-20 | 1 | -16.00 |
| | 2 | -16.90 |
| | 3 | -21.20 |
| Experiment result | -17.90 mV | |

Table 67:

| Sample name | Detection times | Potential ZP (mV) |
|---|---|---|
| R-21 | 1 | -15.20 |
| | 2 | -16.70 |
| | 3 | -16.40 |
| Experiment result | -16.10 mV | |

It may be seen from the above potential detection data that: the 7 groups of extension fragment deformation + core short-sequence RNA nanoparticles all have the good stability, and it is further indicated that the nanoparticles formed by the self-assembly of each extension fragment deformation + core short-sequence RNA have a relative stable self-assembly structure.

(3) Particle size measurement

1. Preparing a potential sample (7 groups of extension fragment deformation + core short-sequence RNA) and putting into a sample pool, opening a sample pool cover of an instrument, and placing the instrument;
2. opening software, and clicking a menu, so a manual measurement parameter setting dialog box appears;
3. setting a software detection parameter; and then
4. clicking Ok to complete the settings, while a measurement dialog box appears, clicking Start to start, a DLS measurement value result of a hydrodynamic size of the 7 groups of extension fragment deformation + core short-sequence RNA is as follows:

Table 68:

| | Mean particle size (nm) |
|---|---|
| R-15 | 6.808 |
| R-16 | 6.978 |
| R-17 | 7.592 |
| R-18 | 7.520 |
| R-19 | 6.936 |
| R-20 | 7.110 |
| R-21 | 6.720 |

(4) TM value detection

A solubility curve method was used to detect TM values of the 7 groups of the extension fragment deformation + core short-sequence RNA nanoparticles, and the samples were consistent with the potential samples.

Reagents and instruments are as follows:

Table 69:

| Reagent name | Article number | Manufacturer |
|---|---|---|
| AE buffer | / | Takara |
| SYBR Green I dye | / | Self-made |

Table 70:

| Name | Model | Manufacturer |
|---|---|---|
| Real-Time System | CFX Connect | Bio-rad |
| Clean bench | HDL | Beijing Donglian Haer Instrument Manufacturing Co., Ltd. |

Operations:

①After the sample was diluted with ultrapure water, 5μg of the diluted sample was mixed with 2μL of SYBR Green I dye (diluted by 1:200), a final volume was 20μL, and the dilution concentration was as follows:

Table 71:

| | Measured concentration (μg/mL) |
|---|---|
| R-15 | 773.009 |
| R-16 | 782.098 |
| R-17 | 740.607 |
| R-18 | 806.163 |
| R-19 | 829.996 |
| R-20 | 723.082 |
| R-21 | 721.674 |

②It was incubated for 30 min in the dark at a room temperature; and
③On-machine detection was performed, a program was set to start at 20 DEG C, the temperature rises per second from 0.1 DEG C to 95 DEGC, and reading was performed every 5 s.

Detection result:

The TM values of the 7 groups of extension fragment deformation + core short-sequence RNA nanoparticles are as follows, a solubility curve diagram of the R-15 is shown in Fig. 18, a solubility curve diagram of the R-16 is shown in Fig. 19, a solubility curve diagram of the R-17 is shown in Fig. 20, a solubility curve diagram of the R-18 is shown in Fig. 21, a solubility curve diagram of the R-19 is shown in Fig. 22, a solubility curve diagram of the R-20 is shown in Fig. 23, and a solubility curve diagram of the R-21 is shown in Fig. 24. Due to the particularity of the RNA samples, a temperature corresponding to 1/2 RFUmax in a range of 20 to 90 DEG C is used as a sample Tm value in this test.

Table 72:

| | TM value(°C) |
|---|---|
| R-15 | 57.5°C |
| R-16 | 53.8°C |
| R-17 | 55.2°C |
| R-18 | 54.5°C |
| R-19 | 54.0°C |

(continued)

| | TM value(°C) |
|---|---|
| R-20 | 59.5°C |
| R-21 | 65.0°C |

The TM values of the 7 groups of extension fragment deformation + core short-sequence RNA nanoparticles are all high, it is indicated that the self-assembly products have the good structural stability.

Embodiment 12

I. 7 groups of extension fragment deformation + core short-sequence DNA nanoparticle carriers:

(1) 7 groups of three polynucleotide base sequences for forming extension fragment deformation + core short-sequence DNA nanoparticles:

Table 73: D-8:

| Name | Sequence sense(5'-3') | Base number | Chemical modification | Molecular weight | Total molecular weight |
|---|---|---|---|---|---|
| a(SEQ ID NO:141) | GCGGCGAGCGGCGA**GGAGCGTTGG**GGCCGGAGGCCGG | 37 | 3'-end is linked with a biotin (Bio) | 12087.3 | 32081.8 |
| b(SEQ ID NO:142) | CCGGCCTCCGGCC**CCTTCGGGG**CCAGCCGCC | 31 | | 9375.1 | |
| c(SEQ ID NO:143) | GGCGGCAGG**CCCCCATAGCCC**TCGCCGCTCGCCGC | 35 | | 10619.4 | |

Table 74: D-9:

| Name | Sequence sense (5'-3') | Base number | Chemical modification | Molecular weight | Total molecular weight |
|---|---|---|---|---|---|
| a(SEQ ID NO:144) | GCGGCGAGCGGCGAGCAGCGTTCGGGCCGGAGGCCGG | 37 | 3'-end is linked with a biotin (Bio) | 12000.4 | 3207 1.6 |
| b(SEQ ID NO:145) | CCGGCCTCCGGCCCGTTCGCCGCCAGCCGCC | 31 | | 9333.2 | |
| c(SEQ ID NO:146) | GGCGGCAGGCGGCCATAGCGCTCGCCGCTCGCCGC | 35 | | 10738 | |

Table 75: D-10:

| Name | Sequence sense(5'-3') | Base number | Chemical modification | Molecular weight | Total molecular weight |
|---|---|---|---|---|---|
| a(SEQ ID NO:147) | GCGGCGAGCGGCGA**GGAGCGTTGG**GGCCGGAGGCCGG | 37 | 3'-end is linked with a biotin (Bio) | 12087.7 | 32081.6 |
| b(SEQ ID NO:148) | CCGGCCTCCGGCC**CCTTCGCCG**CCAGCCGCC | 31 | | 9294.3 | |
| c(SEQ ID NO:149) | GGCGGCAGG**CGGCCATAGCCC**TCGCCGCTCGCCGC | 35 | | 10699.6 | |

Table 76: D-11:

| Name | Sequence sense(5'-3') | Base number | Chemical modification | Molecular weight | Total molecular weight |
|---|---|---|---|---|---|
| a(SEQ ID NO:150) | GCGGCGAGCGGCGAGCAGCGTTCGGGCCGGAGGCCGG | 37 | 3'-end is linked with a biotin (Bio) | 12000.4 | 32071.6 |
| b(SEQ ID NO:151 ) | CCGGCCTCCGGCCCGTTCGGCGCCAGCCGCC | 31 | | 9333.2 | |
| c(SEQ ID NO:152) | GGCGGCAGGCGCCCATAGCGCTCGCCGCTCGCCGC | 35 | | 10738 | |

Table 77: D-12:

| Name | Sequence sense(5'-3') | Base number | Chemical modification | Molecular weight | Total molecular weight |
|---|---|---|---|---|---|
| a(SEQ ID NO:153) | GCGGCGAGCGGCGAGCAGCGTTCGGGCCGGAGGCCGG | 37 | 3'-end is linked with a biotin (Bio) | 12000.4 | 32071.6 |
| b(SEQ ID NO:154) | CCGGCCTCCGGCCCGTTCGGCCCCAGCCGCC | 31 | | 9333.2 | |
| c(SEQ ID NO:155) | GGCGGCAGGGGCCCATAGCGCTCGCCGCTCGCCGC | 35 | | 10738 | |

Table 78: D-13:

| Name | Sequence sense(5'-3') | Base number | Chemical modification | Molecular weight | Total molecular weight |
|---|---|---|---|---|---|
| a(SEQ ID NO:156) | GCGGCGAGCGGCGACGAGCG TTGCGGCCGGAGGCCGG | 37 | 3'-end is linked with a biotin (Bio) | 12000.4 | 32071.6 |
| b(SEQ ID NO:157) | CCGGCCTCCGGCCGCTTCGCC GCCAGCCGCC | 31 | | 9333.2 | |
| c(SEQ ID NO:158) | GGCGGCAGGCGGCCATAGCC GTCGCCGCTCGCCGC | 35 | | 10738 | |

Table 79: D-14:

| Name | Sequence sense(5'-3') | Base number | Chemical modification | Molecular weight | Total molecular weight |
|---|---|---|---|---|---|
| a(SEQ ID NO:159) | GCGGCGAGCGGCGACGAGCG TTGCGGCCGGAGGCCGG | 37 | 3'-end is linked with a biotin (Bio) | 12000.4 | 32071.6 |
| b(SEQ ID NO:160) | CCGGCCTCCGGCCGCTTCGG CGCCAGCCGCC | 31 | | 9373.2 | |
| c(SEQ ID NO:161 ) | GGCGGCAGGCGCCCATAGCC GTCGCCGCTCGCCGC | 35 | | 10698 | |

II. Self-assembly experiment steps:

(1) according to a molar ratio of 1:1:1, enabling the DNA single strands a, b and c to be simultaneously mixed and dissolved in DEPC treated water or TMS buffer solution;
(2) heating mixed solution to 95 DEG C, after keeping for 5 min, slowly cooling to a room temperature at a rate of 2 DEG C/min;
(3) enabling a product to be loaded on 8% (m/v) of non-denaturing PAGE gel and placed in TBM buffer solution under a condition of 4 DEG C, purifying a complex by 100 v of electrophoresis;
(4) cutting a target band and eluting in DNA elution buffer solution at 37 DEG C, after that, precipitating overnight in ethanol, and volatilizing under reduced pressure and low temperature, to obtain the DNA self-assembly products;
(5) electrophoresis analysis detection and laser scanning observation;
(6) electric potential detection;
(7) particle size detection; and
(8) TM value detection.

III. Self-assembly experiment result

(1) Electrophoresis detection

Main reagents and instruments are as follows:

Table 80:

| Reagent name | Article number | Manufacturer |
|---|---|---|
| 6xDNA Loading buffer | TSJ010 | Qingke Biotechnology Co., Ltd. |
| 20bp DNA Ladder | 3420A | TAKARA |
| 10000*SolarGelRed nucleic acid dye | E1020 | solarbio |
| 8% non-denaturing PAGE gel | / | Self-made |
| 1 × TBE Buffer(RNA enzyme-free) | / | Self-made |

Table 81:

| Name | Model | Manufacturer |
|---|---|---|
| Electrophoresis apparatus | PowerPac Basic | Bio-rad |
| Vertical electrophoresis | Mini PROTEAN Tetra Cell | Bio-rad |
| Discoloration shaker | TS-3D | orbital shaker |
| Gel imager | Tanon 3500 | Tanon |

Operations:

① The DNA nanoparticles were diluted with ultrapure water by using a method shown in Table 82below.

Table 82:

| | Measured concentration (μg/mL) |
|---|---|
| D-8 | 2890.932 |
| D-9 | 2238.682 |
| D-10 | 2075.084 |
| D-11 | 3117.389 |
| D-12 | 2880.939 |
| D-13 | 2704.757 |
| D-14 | 3216.917 |

②10μL (500ng) of the processed sample was taken and uniformly mixed with 2μL of 6xDNA Loading Buffer, it was operated on ice, and a label was made.
③A 8% non-denaturing PAGE gel was taken, a piece of the gel was applied to samples with different incubation times, 12 μL of the processed samples were all loaded, and a program was set to run the gel at 100V for 40 minutes.
④After running the gel, dyeing was performed, it was placed on a horizontal shaker for 30 minutes, and pictures were taken for imaging.

Detection result:

Non-denaturing PAGE gel running results of the 7 groups of extension fragment deformation + core short-sequence DNA self-assembly products are shown in Fig. 25. Lanes 1 to 7 in Fig. 25 from left to right are successively: the 7 groups of extension fragment deformation + core short-sequence DNA self-assembly products D-8, D-9, D-10, D-11, D-12, D-13 and D-14.

It may be clearly seen from the results in Fig. 37 that the bands of the 7 groups of extension fragment deformation + core short-sequence DNA self-assembly products are bright and clear, it is indicated that the 7 groups of extension fragment deformation + core short-sequence DNA strands are all self-assembled completely, to form a stable nanoparticle structure.

(2) Electric potential measurement

Measurement method: preparing a potential sample (the self-assembly product is dissolved in ultrapure water) and putting into a sample pool, opening a sample pool cover of an instrument, and placing the instrument; opening software, clicking a menu MeasUre€ManUal, so a manual measurement parameter setting dialog box appears; setting a software detection parameter;
and then clicking Ok to complete the settings, after a measurement dialog box appears, clicking Start to start.

Measurement result: potential detection results of the 7 groups of the extension fragment deformation + core short-sequence DNA nanoparticles at 25 DEG C are as follows:

Table 83:

| Sample name | Detection times | Potential ZP (mV) |
|---|---|---|
| D-8 | 1 | -29.20 |
| | 2 | -32.90 |
| | 3 | -28.60 |
| Experiment result | -30.23 mV | |

Table 84:

| Sample name | Detection times | Potential ZP (mV) |
|---|---|---|
| D-9 | 1 | -39.20 |
| | 2 | -34.20 |
| | 3 | -31.80 |
| Experiment result | -35.07 mV | |

Table 85:

| Sample name | Detection times | Potential ZP (mV) |
|---|---|---|
| D-10 | 1 | -27.90 |
| | 2 | -28.30 |
| | 3 | -21.10 |
| Experiment result | -25.77 mV | |

Table 86:

| Sample name | Detection times | Potential ZP (mV) |
|---|---|---|
| D-11 | 1 | -29.70 |
| | 2 | -24.80 |
| | 3 | -27.80 |
| Experiment result | -27.43 mV | |

Table 87:

| Sample name | Detection times | Potential ZP (mV) |
|---|---|---|
| D-12 | 1 | -33.50 |
| | 2 | -29.80 |
| | 3 | -34.20 |
| Experiment result | -32.50 mV | |

Table 88:

| Sample name | Detection times | Potential ZP (mV) |
|---|---|---|
| D-13 | 1 | -26.80 |
| | 2 | -27.80 |
| | 3 | -26.40 |
| Experiment result | -27.00 mV | |

Table 89:

| Sample name | Detection times | Potential ZP (mV) |
|---|---|---|
| D-14 | 1 | -31.70 |
| | 2 | -32.10 |
| | 3 | -22.40 |
| Experiment result | -28.73 mV | |

It may be seen from the above potential detection data that: the 7 groups of extension fragment deformation + core short-sequence DNA nanoparticles all have the good stability, and it is further indicated that the nanoparticles formed by the self-assembly of each extension fragment deformation + core short-sequence DNA have a relative stable self-assembly structure.

(3) Particle size measurement

①Preparing a potential sample (7 groups of extension fragment deformation + core short-sequence DNA) and putting into a sample pool, opening a sample pool cover of an instrument, and placing the instrument;
②opening software, and clicking a menu, so a manual measurement parameter setting dialog box appears;
③setting a software detection parameter; and then
④clicking Ok to complete the settings, while a measurement dialog box appears, clicking Start to start, a DLS measurement value result of a hydrodynamic size of the 7 groups of extension fragment deformation + core short-sequence DNA is as follows:

Table 90:

| | Mean particle size (nm) |
|---|---|
| D-8 | 7.460 |
| D-9 | 7.920 |
| D-10 | 7.220 |
| D-11 | 7.472 |
| D-12 | 6.968 |

(continued)

| | Mean particle size (nm) |
|---|---|
| D-13 | 7.012 |
| D-14 | 6.896 |

(4) TM value detection

A solubility curve method was used to detect TM values of the 7 groups of the extension fragment deformation + core short-sequence DNA nanoparticles, and the samples were consistent with the potential samples.

Reagents and instruments are as follows:

Table 91:

| Reagent name | Article number | Manufacturer |
|---|---|---|
| AE buffer | / | Takara |
| SYBR Green I dye | / | Self-made |

Table 92:

| Name | Model | Manufacturer |
|---|---|---|
| Real-Time System | CFX Connect | Bio-rad |
| Clean bench | HDL | Beijing Donglian Haer Instrument Manufacturing Co., Ltd. |

Steps:

①After the sample was diluted with ultrapure water, 5 μg of the diluted sample was mixed with 2 μL of SYBR Green I dye (diluted by 1:200), a final volume was 20 μL, and the dilution concentration is as follows:

Table 93:

| | Measured concentration (μg/mL) |
|---|---|
| D-8 | 2890.932 |
| D-9 | 2238.682 |
| D-10 | 2075.084 |
| D-11 | 3117.389 |
| D-12 | 2880.939 |
| D-13 | 2704.757 |
| D-14 | 3216.917 |

②It was incubated for 30 min in the dark at a room temperature; and

③On-machine detection was performed, a program was set to start at 20 DEG C, the temperature rose per second from 0.1 DEG C to 95 DEGC, and reading was performed every 5 s.

Detection result:

The TM values of the 7 groups of extension fragment deformation + core short-sequence DNA nanoparticles are as follows, a solubility curve diagram of the D-8 is shown in Fig. 26, a solubility curve diagram of the D-9 is shown in Fig. 27, a solubility curve diagram of the D-10 is shown in Fig. 28, a solubility curve diagram of the D-11 is shown in Fig. 29, a solubility curve diagram of the D-12 is shown in Fig. 30, a solubility curve diagram of the D-13 is shown in Fig.

31, and a solubility curve diagram of the D-14 is shown in Fig. 32.

Table 94:

| | TM value(°C) |
|---|---|
| D-8 | 48.5 |
| D-9 | 52.5 |
| D-10 | 54.5-55.0 |
| D-11 | 48.7 |
| D-12 | 51.5 |
| D-13 | 51.0 |
| D-14 | 49.2 |

It may be seen from Fig. 26 to 32 that the TM values of the 7 groups of extension fragment deformation + core short-sequence DNA nanoparticles are all high, it is indicated that the self-assembly products have the good structural stability.

**Stability detection of nucleic acid nanoparticles in serum**

Embodiment 13

A non-denaturing PAGE method was used to characterize the stability of 7 groups of extension fragment deformation + core short-sequence RNA nanoparticles in serum.

Main reagents and instruments are as follows:

Table 95:

| Reagent name | Article number | Manufacturer |
|---|---|---|
| 6xDNA Loading buffer | TSJ010 | Qingke Biotechnology Co.,Ltd. |
| 20bp DNA Ladder | 3420A | TAKARA |
| 10000*SolarGelRed nucleic acid dye | E1020 | solarbio |
| 8% non-denaturing PAGE gel | / | Self-made |
| 1 × TBE Buffer(RNA enzyme-free) | / | Self-made |
| Serum(FBS) | / | Excel |
| RPMI 1640 | / | GBICO |

Table 96:

| Name | Model | Manufacturer |
|---|---|---|
| Electrophoresis apparatus | PowerPac Basic | Bio-rad |
| Vertical electrophoresis | Mini PROTEAN Tetra Cell | Bio-rad |
| Discoloration shaker | TS-3D | orbital shaker |
| Gel imager | GenoSens1880 | Shanghai Qinxiang Scientific Instrument Co., Ltd. |

Steps:

① The RNA nanoparticles were prepared to the concentration in the table below, and then the prepared sample was diluted according to a method in the table below. It was diluted by 5 tubes. After being diluted, the sample was placed

in a water bath at 37 DEG C for different times (0, 10 min, 1 h, 12 h, and 36 h);

Table 97:

| Sample name | Sample concentration (μg/mL) | Sample volume | 50%FBS-1640 / μL | 1640/ μL | Diluted concentration (μg/mL) |
|---|---|---|---|---|---|
| R-15 | 773.009 | 1.3 | 4 | 14.7 | 50 μg/m L |
| R-16 | 782.098 | 1.3 | 4 | 14.7 | 50 μg/m L |
| R-17 | 740.607 | 1.4 | 4 | 14.6 | 50 μg/m L |
| R-18 | 806.163 | 1.2 | 4 | 14.8 | 50 μg/m L |
| R-19 | 829.996 | 1.2 | 4 | 14.8 | 50 μg/mL |
| R-20 | 723.082 | 1.4 | 4 | 14.6 | 50 μg/mL |
| R-21 | 721.674 | 1.4 | 4 | 14.6 | 50 μg/mL |

②10μL of the processed sample was taken and uniformly mixed with 2μL of 6xDNA Loading Buffer, it was operated on ice, and a label was made;
③a 8% non-denaturing PAGE gel was taken, a piece of the gel was applied on the samples with different incubation times, 12 μL of the processed samples were all loaded, and a program was set to run the gel at 100V for 40 min; and
④ after running the gel, dyeing was performed, it was placed on a horizontal shaker and shaken slowly for 30 min, pictures were taken for imaging.

An electrophoresis detection result of the R-15 is shown in Fig. 33, an electrophoresis detection result of the R-16 is shown in Fig. 34, an electrophoresis detection result of the R-17 is shown in Fig. 35, an electrophoresis detection result of the R-18 is shown in Fig. 36, an electrophoresis detection result of the R-19 is shown in Fig. 37, an electrophoresis detection result of the R-20 is shown in Fig. 38, and an electrophoresis detection result of the R-21 is shown in Fig. 39. In Fig. 33 to 39, lanes from left to right are respectively M: marker; 1: 36 h; 2: 12 h; 3: 1 h; 4: 10 min; 5: 0 min. It may be seen from the results of the serum stability test that: the results of the non-denaturing gel at 10 min, 1 h, 12 h and 36 h show that there is no significant difference between bands of RNA nanoparticle samples at different times, it is indicated that RNA nanoparticles R-15 to R-21 are relatively stable in a 1640 medium of 50% FBS without apparent degradation.

Embodiment 14

A non-denaturing PAGE method was used to characterize the stability of 7 groups of extension fragment deformation + core short-sequence DNA nanoparticles in serum.

Main reagents and instruments are as follows:

Table 98:

| Reagent name | Article number | Manufacturer |
|---|---|---|
| 6xDNA Loading buffer | TSJ010 | Qingke Biotechnology Co.,Ltd. |
| 20bp DNA Ladder | 3420A | TAKARA |
| 10000*SolarGelRed nucleic acid dye | E1020 | solarbio |
| 8% non-denaturing PAGE gel | / | Self-made |
| 1 × TBE Buffer (RNA enzyme-free) | / | Self-made |
| Serum (FBS) | / | Excel |
| RPMI 1640 | / | GBICO |

Table 99:

| Name | Model | Manufacturer |
|---|---|---|
| Electrophoresis apparatus | PowerPac Basic | Bio-rad |

(continued)

| Name | Model | Manufacturer |
|---|---|---|
| Vertical electrophoresis | Mini PROTEAN Tetra Cell | Bio-rad |
| Discoloration shaker | TS-3D | orbital shaker |
| Gel imager | GenoSens1880 | Shanghai Qinxiang Scientific Instrument Co., Ltd. |

Steps:

①The DNA nanoparticles were prepared to the concentration in the table below, and then the prepared sample was diluted according to a method in the table below. It was diluted by 5 tubes. After being diluted, the sample was placed in a water bath at 37 DEG C for different times (0, 10 min, 1 h, 12 h, and 36 h);

Table 100:

| Sample name | Sample concentrati on (μg/mL) | Sample volume | 50%FBS-1640 / μL | 1640/ μL | Diluted concentrati on (μg/mL) |
|---|---|---|---|---|---|
| D-8 | 2890.932 | 1.4 | 8 | 30.6 | 100 |
| D-9 | 2238.682 | 1.8 | 8 | 30.2 | 100 |
| D-10 | 2075.084 | 1.9 | 8 | 30.1 | 100 |
| D-11 | 3117.389 | 1.3 | 8 | 30.7 | 100 |
| D-12 | 2880.939 | 1.4 | 8 | 30.6 | 100 |
| D-13 | 2704.757 | 1.5 | 8 | 30.5 | 100 |
| D-14 | 3216.917 | 1.2 | 8 | 30.8 | 100 |

②5μL of the processed sample was taken and uniformly mixed with 1μL of 6xDNA Loading Buffer, it was operated on ice, and a label was made;
③a 8% non-denaturing PAGE gel was taken, a piece of the gel was applied on the samples with different incubation times, 6 μL of the processed samples were all loaded, and a program was set to run the gel at 100V for 40 min; and
④ after running the gel, dyeing was performed, it was placed on a horizontal shaker and shaken slowly for 30 min, pictures were taken for imaging.

An electrophoresis detection result of the D-8 is shown in Fig. 40, an electrophoresis detection result of the D-9 is shown in Fig. 41, an electrophoresis detection result of the D-10 is shown in Fig. 42, an electrophoresis detection result of the D-11 is shown in Fig. 43, an electrophoresis detection result of the D-12 is shown in Fig. 44, an electrophoresis detection result of the D-13 is shown in Fig. 45, and an electrophoresis detection result of the D-14 is shown in Fig. 46. In Fig. 40 to 46, lanes from left to right are respectively M: marker; 1: 36 h; 2: 12 h; 3: 1 h; 4: 10 min; 5: 0 min. It may be seen from the results of the serum stability test that: the results of the non-denaturing gel at 10 min, 1 h, 12 h and 36 h show that there is no significant difference between bands of DNA nanoparticle samples at different times, it is indicated that DNA nanoparticles D-8 to D-14 are relatively stable in a 1640 medium of 50% FBS without apparent degradation.

## Drug loading test of nucleic acid nanoparticle

Embodiment 15

Doxorubicin loading experiment:

According to the chemical loading method (unless otherwise specified, the method is the same as that of Embodiment 5) of Embodiment 5, the RNA nanoparticles formed by the self-assembly of the previous R-15, R-16, R-17, R-18, R-19, R-20 and R-21 in Embodiment 11, and the DNA nanoparticles formed by the self-assembly of the D-8, D-9, D-10, D-11, D-12, D-13 and D-14 in Embodiment 12 are respectively used as doxorubicin loading carriers, doxorubicin loading rates measured are respectively as follows:

The doxorubicin loading rate of RNA nanoparticles R-15 is 20.5.

The doxorubicin loading rate of RNA nanoparticles R-16 is 29.4.
The doxorubicin loading rate of RNA nanoparticles R-17 is 30.9.
The doxorubicin loading rate of RNA nanoparticles R-18 is 34.1.
The doxorubicin loading rate of RNA nanoparticles R-19 is 27.1.
The doxorubicin loading rate of RNA nanoparticles R-20 is 30.2.
The doxorubicin loading rate of RNA nanoparticles R-21 is 20.1.
The doxorubicin loading rate of DNA nanoparticles D-8 is 28.0.
The doxorubicin loading rate of DNA nanoparticles D-9 is 27.9.
The doxorubicin loading rate of DNA nanoparticles D-10 is 18.9.
The doxorubicin loading rate of DNA nanoparticles D-11 is 26.8.
The doxorubicin loading rate of DNA nanoparticles D-12 is 27.6.
The doxorubicin loading rate of DNA nanoparticles D-13 is 31.8.
The doxorubicin loading rate of DNA nanoparticles D-14 is 32.

**Cell binding ability of DNA nanoparticles and carrier drugs detected by Fluorescence Activated Cell Sorter (FACS) Experiment**

Embodiment 16

I. Cell information

HepG2 (from Concord Cell Bank), a medium was DMEM+10% FBS+1% double antibody (gibco, 15140-122), and culture conditions were 37 DEG C, 5% $CO_2$ and saturated humidity.

II. Substances to be tested

Blank carrier: DNA nanoparticle carriers formed by the self-assembly of the previous D-8, D-9, D-10, D-11, D-12, D-13 and D-14 in Embodiment 12.
Carrier drug: according to the chemical loading method (unless otherwise specified, the method is the same as that of Embodiment 5) of Embodiment 5, the DNA nanoparticles formed by the self-assembly of the previous D-8, D-9, D-10, D-11, D-12, D-13 and D-14 in Embodiment 12 were used to load a doxorubicin, they were respectively marked as D-8-doxorubicin, D-9-doxorubicin, D-10-doxorubicin, D-11-doxorubicin, D-12-doxorubicin, D-13-doxorubicin and D-14-doxorubicin.

III. Main devices and consumables

Table 101:

| | **Manufacturer** | **Model** |
|---|---|---|
| Biosafety cabinet | Beijing Donglian Haer Instrument Manufacturing Co., Ltd. | BSC-1360 II A2 |
| Low-speed centrifuge | Zhongke Zhongjia Instrument Co., Ltd. | SC-3612 |
| $CO_2$ Incubator | Thermo | 3111 |
| Inverted microscope | UOP | DSZ2000X |
| Fluorescence activated cell sorter | BD | BD FACSCalibur™ |

IV. Main reagent

Table 102:

| Reagent name | Manufacturer | Article number | Remark |
|---|---|---|---|
| DMEM (biotin-free) | Provided by Baiyao Zhida Nano-biotechnology Co., Ltd. | YS3160 | |

(continued)

| Reagent name | Manufacturer | Article number | Remark |
|---|---|---|---|
| 1%BSA-PBS | Self-made | - | |

V. Experiment method

1. A cell state was adjusted to a logarithmic growth phase, the medium was changed into a medium without biotin and folic acid, and it was incubated overnight in an incubator at 37 DEG C.
2. After incubation, cell suspension was trypsinized and collected, and centrifuged at 1000 rpm for 5 min, after the concentration was adjusted, a $2\times10^5$-$5\times10^5$ cell/EP tube was taken and washed twice with 1 mL/tube of 1% BSA-PBS, and cells at the bottom of the tube was observed to prevent them from being absorbed.
3. A substance to be tested was dissolved, and the substance to be tested was diluted to a use concentration.
4. Cell supernatant was absorbed, 100μL of a corresponding sample was added to each tube in order, light was avoided, and it was incubated at 37 DEG C for 2 h.
5. It was washed twice with 1% BSA-PBS; and centrifuged at 1000 rpm for 5min.
6. Finally, it was precipitated with 300 μL of PBS cell resuspension, and flow cytometric on-machine detection (the blank carrier used in the present embodiment was labeled by Quasar670, and the doxorubicin in the carrier drug had its own fluorescence, so the detection could be performed through FL4-APC and FL2-APC respectively) was performed.
7. Data analysis.

VI. Experiment result

1. An experiment result is shown in the table below:

Table 103:

| Detected substance | | Experiment cell | Positive rate |
|---|---|---|---|
| PBS | | HepG2 | 2.11% |
| D-8-doxorubicin(carrier drug) | 1μM | HepG2 | 93.1% |
| | 2μM | HepG2 | 96.3% |
| D-8(blank carrier) | 1μM | HepG2 | 96.9% |
| | 2μM | HepG2 | 98.4% |
| D-9-doxorubicin(carrier drug) | 1μM | HepG2 | 88.6% |
| | 2μM | HepG2 | 95.4% |
| D-9(blank carrier) | 1μM | HepG2 | 96.7% |
| | 2μM | HepG2 | 98.1% |
| D-10-doxorubicin(carrier drug) | 1μM | HepG2 | 89.4% |
| | 2μM | HepG2 | 95.5% |
| D-10(blank carrier) | 1μM | HepG2 | 97.9% |
| | 2μM | HepG2 | 98.3% |
| D-11-doxorubicin(carrier drug) | 1μM | HepG2 | 89.3% |
| | 2μM | HepG2 | 95.5% |
| D-11 (blank carrier) | 1μM | HepG2 | 97.7% |
| | 2μM | HepG2 | 97.8% |

(continued)

| Detected substance | | Experiment cell | Positive rate |
|---|---|---|---|
| D-12-doxorubicin(carrier drug) | 1μM | HepG2 | 94.6% |
| | 2μM | HepG2 | 95.9% |
| D-12(blank carrier) | 1μM | HepG2 | 96.9% |
| | 2μM | HepG2 | 97.1% |
| D-13-doxorubicin(carrier drug) | 1μM | HepG2 | 89.6% |
| | 2μM | HepG2 | 94.0% |
| D-13(blank carrier) | 1μM | HepG2 | 97.6% |
| | 2μM | HepG2 | 98.2% |
| D-14-doxorubicin(carrie r drug) | 1μM | HepG2 | 90.3% |
| | 2μM | HepG2 | 96.1% |
| D-14(blank carrier) | 1μM | HepG2 | 97.4% |
| | 2μM | HepG2 | 98.3% |

2. Conclusion

1. After the HepG2 cells are incubated with D-8-doxorubicin (carrier drug) and D-8 (blank carrier), the binding rates are very high (93.1%-98.4%).
2. After the HepG2 cells are incubated with D-9-doxorubicin (carrier drug) and D-9 (blank carrier), the binding rates are very high (88.6%-98.1%).
3. After the HepG2 cells are incubated with D-10-doxorubicin (carrier drug) and D-10 (blank carrier), the binding rates are very high (89.4%-98.3%).
4. After the HepG2 cells are incubated with D-11-doxorubicin (carrier drug) and D-11 (blank carrier), the binding rates are very high (89.3%-97.8%).
5. After the HepG2 cells are incubated with D-12-doxorubicin (carrier drug) and D-12 (blank carrier), the binding rates are very high (94.6%-97.1%).
6. After the HepG2 cells are incubated with D-13-doxorubicin (carrier drug) and D-13 (blank carrier), the binding rates are very high (89.6%-98.2%).
7. After the HepG2 cells are incubated with D-14-doxorubicin (carrier drug) and D-14 (blank carrier), the binding rates are very high (90.3%-98.3%).

**Cytotoxicity research of DNA nanoparticles and carrier drugs in HepG2 cells**

Embodiment 17

A CCK8 method was used to detect the toxicity of DNA naoparticles and carrier drugs to HepG2.

I. Main reagent

Table 104:

| Reagent name | Manufacturer | Article number |
|---|---|---|
| PBS | - | - |
| DMSO | SIGMA | D2650 |
| DMEM (biotin-free) | Provided by Baiyao Zhida Nano-biotechnology Co., Ltd. | YS3160 |
| FBS | Excell Bio | FSP500 |

(continued)

| Reagent name | Manufacturer | Article number |
|---|---|---|
| Double-antibody | gibco | 15140-122 |
| Pancreatin | gibco | 25200-056 |
| CCK8 kit | Biyuntian Company | C0038 |

II. Main consumables and instruments

Table 105:

| Name | Manufacturer | Model |
|---|---|---|
| 96-well cell culture plate | NEST | 701001 |
| Biosafety cabinet | Beijing Donglian Haer Instrument Manufacturing Co., Ltd. | BSC-1 360 II A2 |
| Low-speed centrifuge | Zhongke Zhongjia Instrument Co., Ltd. | SC-3612 |
| $CO_2$ Incubator | Thermo | 3111 |
| Inverted microscope | UOP | DSZ2000X |
| Microplate reader | Shanghai Ouying Experimental Equipment Co., Ltd. | K3 |

III. Cell information

HepG2 (from Concord Cell Bank), a medium was DMEM+10% FBS+1% double antibody (gibco, 15140-122), and culture conditions were 37 DEG C, 5% $CO_2$ and saturated humidity.

IV. Experiment material

1. Samples to be tested

Blank carrier: DNA nanoparticle carriers formed by the self-assembly of the previous D-8, D-9, D-10, D-11, D-12, D-13 and D-14 in Embodiment 12, respectively marked as: D-8, D-9, D-10, D-11, D-12, D-13 and D-14.

Carrier drug: according to the chemical loading method (unless otherwise specified, the method is the same as that of Embodiment 5) of Embodiment 5, the DNA nanoparticles formed by the self-assembly of the previous D-8, D-9, D-10, D-11, D-12, D-13 and D-14 in Embodiment 12 are used to load a doxorubicin, they are respectively marked as D-8-doxorubicin, D-9-doxorubicin, D-10-doxorubicin, D-11-doxorubicin, D-12-doxorubicin, D-13-doxorubicin and D-14-doxorubicin.

Original drug doxorubicin.

DMSO.

V. Experiment procedure

1. HepG2 cells in a logarithmic growth phase were taken, trypan blue was used for staining and the cell viability was counted to be 98.3%, plating was performed with 5000 Cell/well, a volume was 100 μL/well, 8 96-well plates were paved with 57 wells per plate, and it was incubated overnight at 37 DEG C.

2. A sample to be tested was diluted according to the following table and added: the original culture medium was removed and 100 μL of a culture medium of the sample to be tested with the different concentration was added, and there were 3 replicate wells in each group.

Table 106:

| Well number | C9 | C8* | C7 | C6 | C5 | C4 | C3 | C2 | C1 |
|---|---|---|---|---|---|---|---|---|---|
| Final concentration of loaded drug | 10μM | 3.16μM | 1μM | 316nM | 100nM | 31.6nM | 10nM | 3.16nM | 1nM |
| Final concentration of blank carrier | 1μM | 316nM | 100nM | 31.6nM | 10nM | 3.16nM | 1nM | 0.316nM | 0.1nM |
| Final concentration of original drug doxorubicin | 10μM | 3.16μM | 1μM | 316nM | 100nM | 31.6nM | 10nM | 3.16nM | 1nM |
| DMSO(%) | 0.1 | 0.0316 | 0.01 | 0.00316 | 0.001 | 0.00036 | 0.0001 | 0.000036 | 0.00001 |

A preparation method for the sample in a C8 well is as follows: 324 μL of the complete culture medium was used, and then it was absorbed from a C9 well.

In the present embodiment, the loaded drug and the blank carrier were firstly prepared into 100 μM of stock solution by using the PBS, and then diluted with the complete culture medium (biotin-free DMEM). The original drug doxorubicin was firstly prepared into 100 μM of stock solution by using the DMSO, and then diluted with the complete culture medium (biotin-free DMEM). The DMSO was directly diluted with the complete medium (biotin-free DMEM).

3. After the sample to be tested was added, the 96-well plate was placed in an incubator under 37 DEG C and 5% $CO_2$ and incubated for 72 hours.

4. A kit was taken out and melted at a room temperature, 10 μL of CCK-8 solution was added to each well, or the CCK8 solution was mixed with the culture medium at a ratio of 1:9, and then added to the well in an amount of 100 μL/well.

5. It was continuously incubated in the cell incubator for 4 hours, and a length of time depends on experimental conditions such as a cell type and a cell density.

6. A microplate reader was used to measure an absorbance at 450 nm.

7. Calculation: cell viability (%)=(OD experimental group-OD blank group)x100%/(OD control group-OD blank group), $IC_{50}$ is calculated by GraphPad Prism 5.0.

VI. Experiment result

Table 107:

| Cell line | Detected sample | $IC_{50}$ (μM) |
|---|---|---|
| HepG2 | Original drug doxorubicin | 0.2725 |
| | D-8-doxorubicin (loaded drug) | 0.05087 |
| | D-8(blank carrier) | >1 |
| | D-9-doxorubicin (loaded drug) | 0.0386 |
| HepG2 | D-9(blanking carrier) | >1 |
| | D-10-doxorubicin (loaded drug) | 0.03955 |
| | D-10(blank carrier) | >1 |
| | D-11-doxorubicin (loaded drug) | 0.04271 |
| HepG2 | D-11 (blank carrier) | >1 |
| | D-12-doxorubicin (loaded drug) | 0.02294 |
| | D-12(blank carrier) | >1 |
| | D-13-doxorubicin (loaded drug) | 0.03017 |
| HepG2 | D-13(blank carrier) | >1 |
| | D-14-doxorubicin (loaded drug) | 0.03458 |
| | D-14(blank carrier) | >1 |
| | DMSO | >0.1% |

Conclusion:

It may be seen from the above table and Fig. 47a, Fig. 47b, Fig. 47c, Fig. 47d, Fig. 47e, Fig. 47f. Fig. 47g and Fig. 47h that the $IC_{50}$ of the original drug doxorubicin and the loaded drugs D-8-doxorubicin, D-9-doxorubicin, D-10-doxorubicin, D-11-doxorubicin, D-12-doxorubicin, D-13-doxorubicin and D-14-doxorubicin acting on the HepG2 cells is 0.2725 μM, 0.05087 μM, 0.0386, 0.03955, 0.04271, 0.02294, 0.03017 and 0.03458 respectively; the $IC_{50}$ of the DMSO acting on the HepG2 cells is >0.1%; the $IC_{50}$ of the D-8 (blank carrier), D-9 (blank carrier), D-10 (blank carrier), D-11 (blank carrier), D-12 (blank carrier), D-13 (blank carrier) and D-14 (blank carrier) acting on the HepG2 cells is >1μM. It is indicated that for the HepG2 cell line, compared with the simple blank carriers D-8, D-9, D-10, D-11, D-12, D-13 and D-14, the original drug doxorubicin of small molecular drug and the loaded drugs D-8-doxorubicin, D-9-doxorubicin, D-10-doxorubicin, D-11-doxorubicin, D-12-doxorubicin, D-13-doxorubicin Both D-14-doxorubicin and D-14-doxorubicin are

toxic to the HepG2 cells, and compared with the original drug doxorubicin, the loaded drugs D-8-doxorubicin, D-9-doxorubicin, D-10-doxorubicin, and D-11-doxorubicin, D-12-doxorubicin, D-13-doxorubicin and D-14-doxorubicin have an apparent synergistic effect.

**Daunorubicin loading experiment**

Embodiment 18

According to the chemical loading method (unless otherwise specified, the method is the same as that of Embodiment 5) of Embodiment 5, the DNA nanoparticles formed by the self-assembly of the previous D-10 and D-14 in Embodiment 12 were used as a daunorubicin loading carrier. A microplate reader was used to measure an absorbance of the daunorubicin at 492 nm, and a standard curve (as shown in Fig. 48) was drawn.

Measured loading rates of the daunorubicin are respectively as follows:

The daunorubicin loading rate of the DNA nanoparticles D-10 is 24.0.

The daunorubicin loading rate of the DNA nanoparticles D-14 is 25.1.

It may be seen from the above description that the above embodiments of the disclosure achieve the following technical effects: the present application provides a series of nucleic acid nanoparticle carriers with thermodynamic stability, chemical stability, high loading rate and multivalent combination modules. A unique modular design of this type of the carriers is performed to obtain a core modular structure which not only maintains a naturally compatible affinity, but also has high stability and diverse combination characteristics. This structure may flexibly and efficiently integrate various functional modules, including a targeting module, an imaging and probe module, a treatment module and other composite intelligent modules, so that it may be used for targeted delivery in vivo to achieve precise diagnosis and treatment.

The above are only the preferred embodiments of the disclosure, and are not used to limit the disclosure, and various modifications and changes may be made to the disclosure by those skilled in the art. Any modifications, equivalent replacements, improvements and the like made within spirit and principle of the disclosure should be included in the scope of protection of the disclosure.

## Claims

**1.** A nucleic acid nanoparticle, wherein the nucleic acid nanoparticle has a nucleic acid structural domain, and the nucleic acid structural domain comprises a sequence a, a sequence b and a sequence c,
the sequence a comprises a sequence a1 or a sequence obtained by insertion, deletion or substitution of at least one base in the sequence a1, the sequence b comprises a sequence b1 or a sequence obtained by insertion, deletion or substitution of at least one base in the sequence b1, and the sequence c comprises a sequence c1 or

a sequence obtained by insertion, deletion or substitution of at least one base in the sequence c1,
wherein the sequence a1 is SEQ ID NO:1:5’-CCAGCGUUCC-3’or SEQ ID NO:2 :5’ -CCAGCGTTCC-3’;
the sequence b1 is SEQ ID NO:3:5’-GGUUCGCCG-3’or SEQ ID NO:4:5’-GGTTCGCCG-3’; and
the sequence c1 is SEQ ID NO:5:5’-CGGCCAUAGCGG-3’or SEQ ID NO:6:5’ -CGGCCATAGCGG-3’.

**2.** The nucleic acid nanoparticle as claimed in claim 1, wherein when the sequence a1 is the SEQ ID NO:1, the sequence b1 is the SEQ ID NO:3, and the sequence c1 is the SEQ ID NO:5, at least one sequence of the sequence a, the second b and the sequence c comprises a sequence obtained by insertion, deletion or substitution of at least one base in the sequence a, the sequence b and/or the sequence c.

**3.** The nucleic acid nanoparticle as claimed in claim 1 or 2, wherein the insertion, deletion or substitution of at least one base is occurred:

(1) at 1, 2, 4 or 5-th base starting from a 5 ’-end of the sequence shown in the SEQ ID NO:1 or the SEQ ID NO:2; and/or
(2) between 8-th and 10-th bases starting from the 5 ’-end of the sequence shown in the SEQ ID NO:1 or the SEQ ID NO:2; and/or
(3) between 1-th and 3-th bases starting from a 5 ’-end of the sequence shown in the SEQ ID NO:3 or the SEQ ID NO:4; and/or
(4) between 6-th and 9-th bases starting from the 5 ’-end of the sequence shown in the SEQ ID NO:3 or the SEQ ID NO:4; and/or
(5) between 1-th and 4-th bases starting from a 5 ’-end of the sequence shown in the SEQ ID NO:5 or the SEQ ID NO:6; and/or
(6) between 9-th and 12-th bases starting from the 5 ’-end of the sequence shown in the SEQ ID NO:5 or the SEQ ID NO:6.

**4.** The nucleic acid nanoparticle as claimed in claim 1 or 2, wherein the sequence a, the sequence b and the sequence c are self-assembled into a structure shown in Formula (1):

```
a 5' WWNWWNNNWW3'
  3' CC   CC   N'N'CC5' b
          N
          N   N'
          N
          N
          W   C
          W   C
          W   C
          W   C
          5'  3'
          c
```

Formula (1),

wherein, W-C represents a Watson-Crick pairing, N and N’ represent a non-Watson-Crick pairing, the W-C in any one position is independently selected from C-G or G-C;
in the sequence a, the first N from the 5’-end is A, the second N is G, the third N is U or T, and the fourth N is any one of U, T, A, C or G;
in the sequence b, the first N’ from the 5’-end is any one of U, T, A, C or G, the second N’ is U or T, and the third N’ is C; and
in the sequence c, a sequence NNNN along a direction from the 5’-end to the 3’-end is CAUA or CATA.

**5.** The nucleic acid nanoparticle as claimed in claim 4, wherein the sequence a, the sequence b and the sequence c are any one of the following groups:

(1)

sequence a: 5'-GGAGCGUUGG-3',
sequence b: 5'-CCUUCGCCG-3',
sequence c: 5'-CGGCCAUAGCCC-3';

(2)

sequence a: 5'-GCAGCGUUCG-3',
sequence b: 5'-CGUUCGCCG-3',
sequence c: 5'-CGGCCAUAGCGC-3';

(3)

sequence a: 5'-CGAGCGUUGC-3',
sequence b: 5'-GCUUCGCCG-3',
sequence c: 5'-CGGCCAUAGCCG-3';

(4)

sequence a: 5'-GGAGCGUUGG-3',
sequence b: 5'-CCUUCGGGG-3',
sequence c: 5'-CCCCCAUAGCCC-3';

(5)

sequence a: 5'-GCAGCGUUCG-3',
sequence b: 5'-CGUUCGGCG-3',
sequence c: 5'-CGCCCAUAGCGC-3';

(6)

sequence a: 5'-GCAGCGUUCG-3',
sequence b: 5'-CGUUCGGCC-3',
sequence c: 5'-GGCCCAUAGCGC-3';

(7)

sequence a: 5'-CGAGCGUUGC-3',
sequence b: 5'-GCUUCGGCG-3',
sequence c: 5'-CGCCCAUAGCCG-3';

(8)

sequence a: 5'-GGAGCGTTGG-3',
sequence b: 5'-CCTTCGCCG-3',
sequence c: 5'-CGGCCATAGCCC-3';

(9)

sequence a: 5'-GCAGCGTTCG-3',
sequence b: 5'-CGTTCGCCG-3',
sequence c: 5'-CGGCCATAGCGC-3';

(10)

sequence a: 5'-CGAGCGTTGC-3',
sequence b: 5'-GCTTCGCCG-3',
sequence c: 5'-CGGCCATAGCCG-3';

(11)

sequence a: 5’-GGAGCGTTGG-3’,
sequence b: 5’-CCTTCGGGG-3’,
sequence c: 5’-CCCCCATAGCCC-3’;

(12)

sequence a: 5’-GCAGCGTTCG-3’,
sequence b: 5’-CGTTCGGCG-3’,
sequence c: 5’-CGCCCATAGCGC-3’;

(13)

sequence a: 5’-GCAGCGTTCG-3’, sequence b: 5’-CGTTCGGCC-3’, sequence c: 5’-GGCCCATAGCGC-3’; and

(14)

sequence a: 5’-CGAGCGTTGC-3’,
sequence b: 5’-GCTTCGGCG-3’,
sequence c: 5’-CGCCCATAGCCG-3’.

**6.** The nucleic acid nanoparticle as claimed in claim 4, wherein in the nucleic acid structural domain, a first extension fragment is further comprised, the first extension fragment is an extension fragment of Watson-Crick pairing, and the first extension fragment is positioned at the 5’-end and/or the 3’-end of any one sequence of the sequence a, the sequence b or the sequence c;
preferably, the first extension fragment is at least selected from any one of the following groups:

(1): a-strand 5’-end: 5’-CCCA-3’, c-strand 3’-end: 5’-UGGG-3’;
(2): a-strand 3’-end: 5’-GGG-3’, b-strand 5’-end: 5’-CCC-3’;
(3): b-strand 3’-end: 5’-CCA-3’, c-strand 5’-end: 5’-UGG-3’;
(4): a-strand 5’-end: 5’-CCCG-3’, c-strand 3’-end: 5’-CGGG-3’;
(5): a-strand 5’-end: 5’-CCCC-3’, c-strand 3’-end: 5’-GGGG-3’;
(6): b-strand 3’-end: 5’-CCC-3’, c-strand 5’-end: 5’-GGG-3’;
(7): b-strand 3’-end: 5’-CCG-3’, c-strand 5’-end: 5’-CGG-3’;
(8): a-strand 5’-end: 5’-CCCA-3’, c-strand 3’-end: 5’-TGGG-3’; and
(9): b-strand 3’-end: 5’-CCA-3’, c-strand 5’-end: 5’-TGG-3’.

**7.** The nucleic acid nanoparticle as claimed in any one of claims 1 to 6, wherein the nucleic acid structural domain further comprises a second extension fragment, the second extension fragment is positioned at the 5’-end and/or the 3’-end of any one sequence of the sequence a, the sequence b, or the sequence c, and the second extension fragment is an extension fragment of Watson-Crick pairing;
preferably, the second extension fragment is an extension sequence of a CG base pair; and
more preferably, the second extension fragment is an extension sequence of 1-10 CG base pairs.

**8.** The nucleic acid nanoparticle as claimed in claim 7, wherein the nucleic acid structural domain further comprises at least one group of the following second extension fragments:

a first group: a-strand 5’-end: 5’-CGCGCG-3’, c-strand 3’-end: 5’-CGCGCG-3’;
a second group: a-strand 3’-end: 5’-CGCCGC-3’, b-strand 5’-end: 5’-GCGGCG-3’; and
a third group: b-strand 3’-end: 5’-GGCGGC-3’, c-strand 5’-end: 5’-GCCGCC-3’.

**9.** The nucleic acid nanoparticle as claimed in claim 7, wherein the second extension fragment is an extension sequence containing both CG base pair and AT/AU base pair, and preferably the second extension fragment is an extension sequence of 2-50 base pairs.

**10.** The nucleic acid nanoparticle as claimed in claim 9, wherein,

the second extension fragment is an extension sequence in which sequences of 2-8 continuous CG base pairs and sequences of 2-8 continuous AT/AU base pairs are alternately arranged; or
the second extension fragment is an extension sequence in which a sequence of 1 CG base pair and a sequence of 1 AT/AU base pair are alternately arranged.

**11.** The nucleic acid nanoparticle as claimed in any one of claims 1 to 10, wherein a base, a ribose and a phosphate in the sequence a, the sequence b and the sequence c have at least one modifiable site, and any one of the modifiable sites is modified by any one of the following modificationadaptors: -F, a methyl, an amino, a disulfide, a carbonyl, a carboxyl, a sulfhydryl and a formyl; and
preferably, the base C or U in the sequence a, the sequence b and the sequence c has 2'-F modification.

**12.** The nucleic acid nanoparticle as claimed in any one of claims 1 to 11, wherein the nucleic acid nanoparticle further comprises a bioactive substance, and the bioactive substance is linked with the nucleic acid structural domain.

**13.** The nucleic acid nanoparticle as claimed in claim 12, wherein a ratio of a relative molecular weight of the nucleic acid structural domain and a total relative molecular weight of the bioactive substance is ≥1:1; and
preferably, the bioactive substance is one or more of a target head, a fluorescein, an interfering nucleic acid siRNA, a miRNA, a ribozyme, a riboswitch, an aptamer, a RNA antibody, a drug, a protein, a polypeptide, a flavonoid, a glucose, a natural salicylic acid, a monoclonal antibody, a vitamin, an phenol and a lecithin.

**14.** The nucleic acid nanoparticle as claimed in claim 13, wherein the bioactive substance is the target head, the fluorescein and the miRNA,
wherein, the target head is positioned on any one sequence of the sequences a, b and c, preferably the 5'-end or the 3'-end of any one sequence of the sequences a, b and c, or inserted between GC bonds of the nucleic acid structural domain,
the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c; and
preferably, the target head is a folic acid or a biotin, the fluorescein is any one or more of FAM, CY5 and CY3, and the anti-miRNA is anti-miR-21.

**15.** The nucleic acid nanoparticle as claimed in claim 13, wherein the drug is a drug for treating liver cancer, gastric cancer, lung cancer, breast cancer, head and neck cancer, uterine cancer, ovarian cancer, melanoma, leukemia, Alzheimer's disease, ankylosing spondylitis, malignant lymphoma, bronchial cancer, rheumatoid arthritis, HBV hepatitis B, multiple myeloma, pancreatic cancer, non-small cell lung cancer, prostate cancer, nasopharyngeal cancer, esophageal cancer, oral cancer, lupus erythematosus; and preferably, the head and neck cancer is brain cancer, neuroblastoma or glioblastoma.

**16.** The nucleic acid nanoparticle as claimed in claim 13, wherein the drug is a drug containing any one or more of the following groups: an amino group, a hydroxyl group, a carboxyl group, a mercapto group, a benzene ring group and an acetamido group.

**17.** The nucleic acid nanoparticle as claimed in claim 13, wherein the protein is one or more of antibodies or aptamers of SOD, survivin, hTERT, EGFR and PSMA; the vitamin is L-Vc and/or esterified Vc; and the phenol is a tea polyphenol and/or a grape polyphenol.

**18.** The nucleic acid nanoparticle as claimed in claim 12, wherein the bioactive substance is linked with the nucleic acid structural domain in any one of the following modes:

mode I: physical insertion; and
mode II: covalent linkage.

**19.** The nucleic acid nanoparticle as claimed in claim 18, wherein while the bioactive substance is linked with the nucleic acid structural domain in the physical insertion mode, the physical insertion is performed on the bioactive substance and the nucleic acid structural domain according to a molar ratio of 1 to 200:1.

**20.** The nucleic acid nanoparticle as claimed in claim 19, wherein while the bioactive substance is linked with the nucleic acid structural domain in the physical insertion mode and the covalent linkage mode, a molar ratio of the bioactive

substance linked in the physical insertion mode and the drug linked in the covalent linkage mode is 1 to 200:1.

**21.** The nucleic acid nanoparticle as claimed in claim 18, wherein the bioactive substance linked in the covalent linkage mode is covalently linked through a solvent, covalently linked through a linker or click-linked;
preferably, the solvent is selected from paraformaldehyde, DCM, DCC, DMAP, Py, DMSO, PBS or glacial acetic acid;
preferably, the linker is selected from a disulfide bond, a p-phenylazide, bromopropyne or a PEG; and
preferably, the click-linkage is that a bioactive substance precursor and the nucleic acid structural domain are modified by a alkynyl or a azide simultaneously , and then linked through a clickreaction.

**22.** The nucleic acid nanoparticle as claimed in claim 21, wherein when the bioactive substance is linked with the nucleic acid structural domain in a click-linkage mode, a site, for performing the alkynyl or azide modification, of the bioactive substance precursor is selected from a 2'-hydroxyl, a carboxyl or an amino, and a site, for performing the alkynyl or azide modification, of the nucleic acid structural domain is selected from a G-exocyclic amino, a 2'-hydroxyl, an $\alpha$-amino or a 2'-hydroxyl.

**23.** The nucleic acid nanoparticle as claimed in claim 1, wherein a particle size of the nucleic acid nanoparticle is 1-100 nm, preferably 5-50 nm; more preferably 10-30 nm; and further preferably 10-15 nm.

**24.** A pharmaceutical composition, wherein the pharmaceutical composition comprises the nucleic acid nanoparticle as claimed in any one of claims 1 to 23.

**25.** A drug comprising doxorubicin, wherein the drug comprising the doxorubicin comprises the doxorubicin and the nucleic acid nanoparticle as claimed in any one of claims 1 to 12 or claim 23.

**26.** The drug comprising the doxorubicin as claimed in claim 25, wherein the doxorubicin is loaded on the nucleic acid nanoparticle in the physical linkage and/or covalent linkage mode, and a molar ratio between the doxorubicin and the nucleic acid nanoparticle is 2 to 300:1, preferably 10 to 50:1, and more preferably 15 to 25:1.

**27.** The drug comprising the doxorubicin as claimed in claim 25, wherein the nucleic acid nanoparticle further comprise a bioactive substance, the bioactive substance is linked with the nucleic acid structural domain, and the bioactive substance is one or more of a target head, a fluorescein, an interfering nucleic acid siRNA, a miRNA, a ribozyme, a riboswitch, an aptamer, a RNA antibody, a protein, a polypeptide, a flavonoid, a glucose, a natural salicylic acid, a monoclonal antibody, a vitamin, phenols, a lecithin and a small molecular drug except the doxorubicin.

**28.** The drug comprising the doxorubicin as claimed in claim 27, wherein a relative molecular weight of the nucleic acid structural domain is marked as $N_1$, and a total relative molecular weight of the doxorubicin and the bioactive substance is marked as N2, $N_1/N_2 \geq 1:1$.

**29.** The drug comprising the doxorubicin as claimed in claim 27, wherein the bioactive substance is one or more of the target head, the fluorescein and the miRNA,
wherein, the target head is positioned on any one sequence of the sequences a, b and c, preferably the 5'-end or the 3'-end of any one sequence of the sequences a, b and c, or inserted between GC bonds of the nucleic acid structural domain,
the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c; and
preferably, the target head is a folic acid or a biotin, the fluorescein is any one or more of FAM, CY5 and CY3, and the anti-miRNA is anti-miR-21.

**30.** The drug comprising the doxorubicin as claimed in claim 27, wherein the small molecular drug except the doxorubicin is a drug containing any one or more of the following groups: an amino group, a hydroxyl group, a carboxyl group, a mercapto group, a benzene ring group and an acetamido group.

**31.** The drug comprising the doxorubicin as claimed in claim 27, wherein the protein is one or more of SOD, survivin, hTERT, EGFR and PSMA; the vitamin is L-Vc and/or esterified Vc; and the phenol is a tea polyphenol and/or a grape polyphenol.

**32.** A method for preparing a drug comprising doxorubicin, wherein the method comprises the following steps:

providing the nucleic acid nanoparticle as claimed in any one of claims 1 to 12;
loading the doxorubicin on the nucleic acid nanoparticle in a physical linkage mode and/or a covalent linkage mode, to obtain the drug comprising the doxorubicin.

**33.** The method as claimed in claim 32, wherein the step of loading the doxorubicin in the physical linkage mode comprises:

mixing and stirring the doxorubicin, the nucleic acid nanoparticle and a first solvent to obtain a premixed system; and
removing a free substance in the premixed system, to obtain the drug comprising the doxorubicin;
preferably, the first solvent is selected from one or more of DCM, DCC, DMAP, Py, DMSO, PBS and glacial acetic acid;
preferably, the step of removing the free substance in the premixed system comprises: mixing the premixed system with absolute ethyl alcohol, and precipitating the drug comprising the doxorubicin in a temperatureof lower than 10 DEG C; and more preferably, precipitating the drug comprising the doxorubicin at a temperature of 0-5 DEG C.

**34.** The method as claimed in claim 32, wherein the step of loading the doxorubicin in the covalent linkage mode comprises:

preparing doxorubicin solution;
enabling the doxorubicin solution to react with the G-exocyclic amino of the nucleic acid nanoparticle under a mediating effect of the formaldehyde, to obtain a reaction system; and
purifying the reaction system, to obtain the drug comprising the doxorubicin;
preferably, the reaction step comprises:
mixing the doxorubicin solution with a paraformaldehyde solution and the nucleic acid nanoparticle, and reacting in a dark condition, to obtain the reaction system; wherein a concentration of the paraformaldehyde solution is preferably 3.7-4wt%, and the paraformaldehyde solution is preferably a solution formed by mixing paraformaldehyde and a second solvent, and the second solvent is one or more of DCM, DCC, DMAP, Py, DMSO, PBS and glacial acetic acid.

**35.** The method as claimed in any one of claims 32 to 34, wherein the preparation method further comprises a step of preparing the nucleic acid nanoparticle, the step comprises: self-assembling single strands corresponding to the nucleic acid structural domain in the nucleic acid nanoparticle as claimed in any one of claims 1 to 12, to obtain the nucleic acid structural domain;
preferably, after the nucleic acid structural domain is obtained, the preparation method further comprises: loading the bioactive substance as claimed in any one of claims 13 to 17 on the nucleic acid structural domain in the physical mode of physical linkage and/or the covalent linkage mode, to obtain the nucleic acid nanoparticle, wherein the drug in the bioactive substance is a small molecular drug except the doxorubicin.

**36.** The method as claimed in claim 34, wherein in a process of loading the bioactive substance in the covalent linkage mode, the loading is performed through a solvent covalent linkage, a linker covalent linkage or a click-linkage;
preferably, a third solvent used in the solvent covalent linkage is served as a linkage medium, and the third solvent is selected from one or more of paraformaldehyde, DCM, DCC, DMAP, Py, DMSO, PBS and glacial acetic acid;
preferably, the linker is selected from a disulfide bond, a p-phenylazide, bromopropyne or a PEG; and
preferably, the click-linkage is that a bioactive substance precursor and the nucleic acid structural domain is modified by alkynyl or azide simultaneously, and then linked through a clickreaction.

**37.** The method as claimed in claim 35, wherein when the bioactive substance is linked with the nucleic acid structural domain in the click-linkage mode, a site, for performing the alkynyl or azide modification, of the bioactive substance precursor is selected from a 2'-hydroxyl, a carboxyl or an amino, and a site, for performing the alkynyl or azide modification, of the nucleic acid structural domain is selected from a G-exocyclic amino, a 2'-hydroxyl, an A-amino or a 2'-hydroxyl.

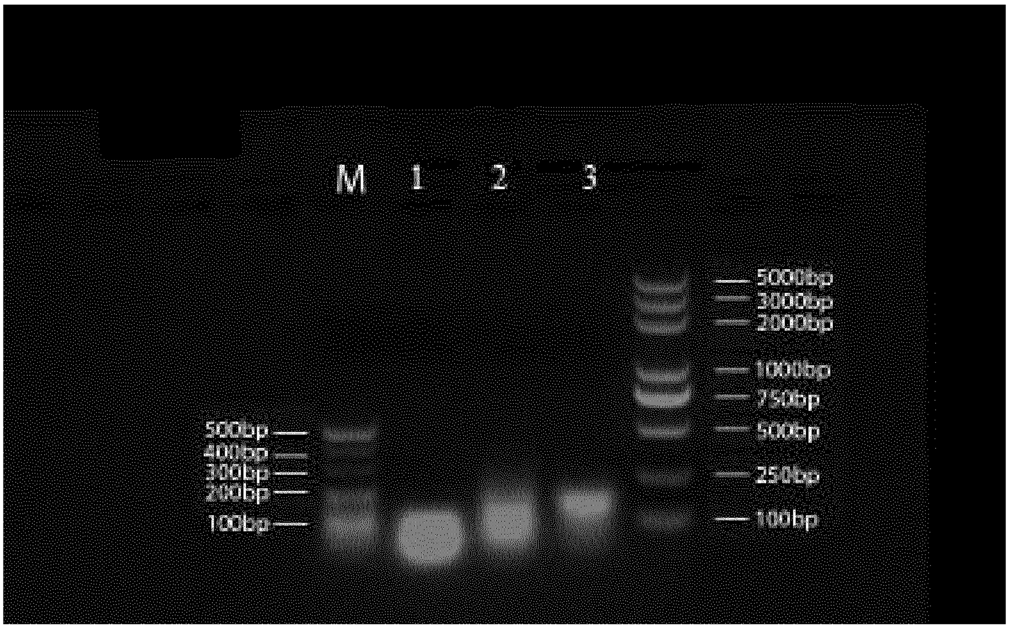
M
1
2
3
500bp
400bp
300bp
200bp
100bp
5000bp
3000bp
2000bp
1000bp
750bp
500bp
250bp
100bp

Fig.1

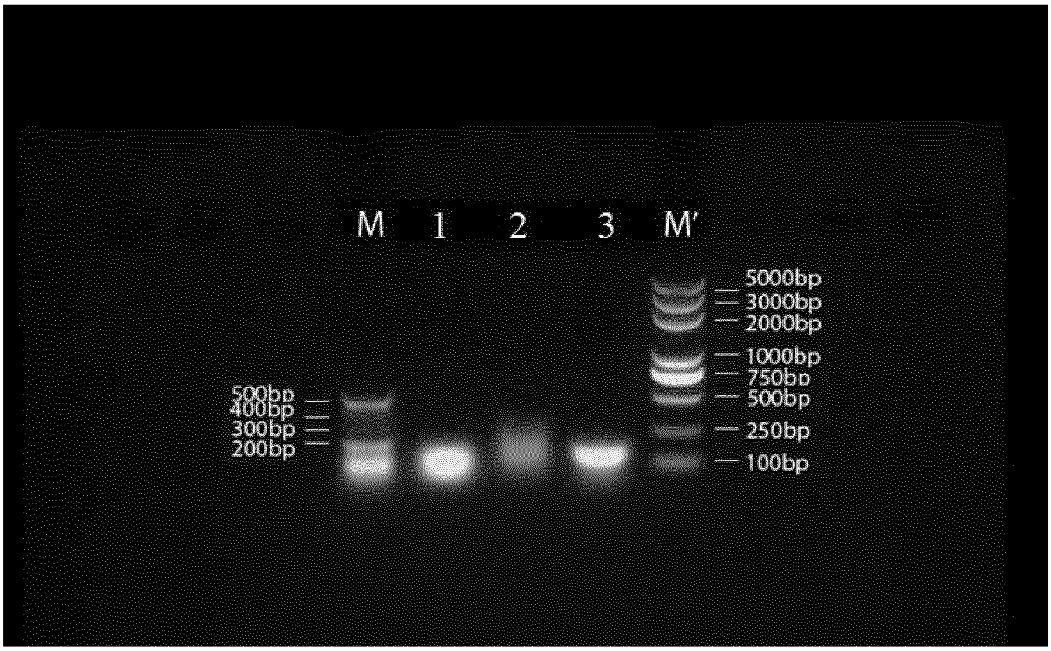
M
1
2
3
M'
500bp
400bp
300bp
200bp
5000bp
3000bp
2000bp
1000bp
750bp
500bp
250bp
100bp

Fig.2

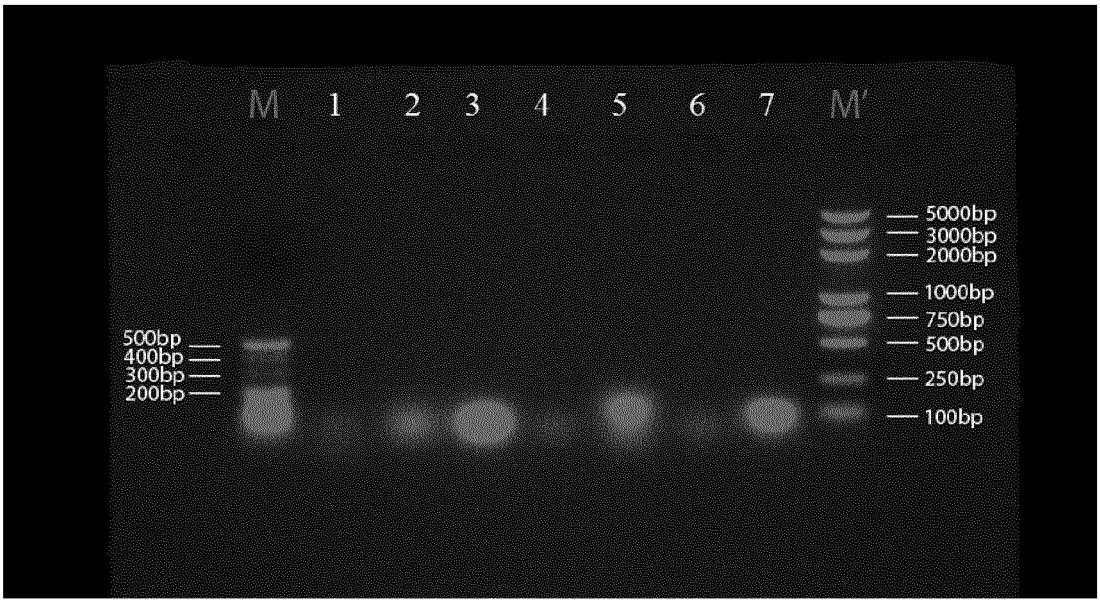
M
1
2
3
4
5
6
7
M'
5000bp
3000bp
2000bp
1000bp
750bp
500bp
250bp
100bp
500bp
400bp
300bp
200bp

**Fig.3**

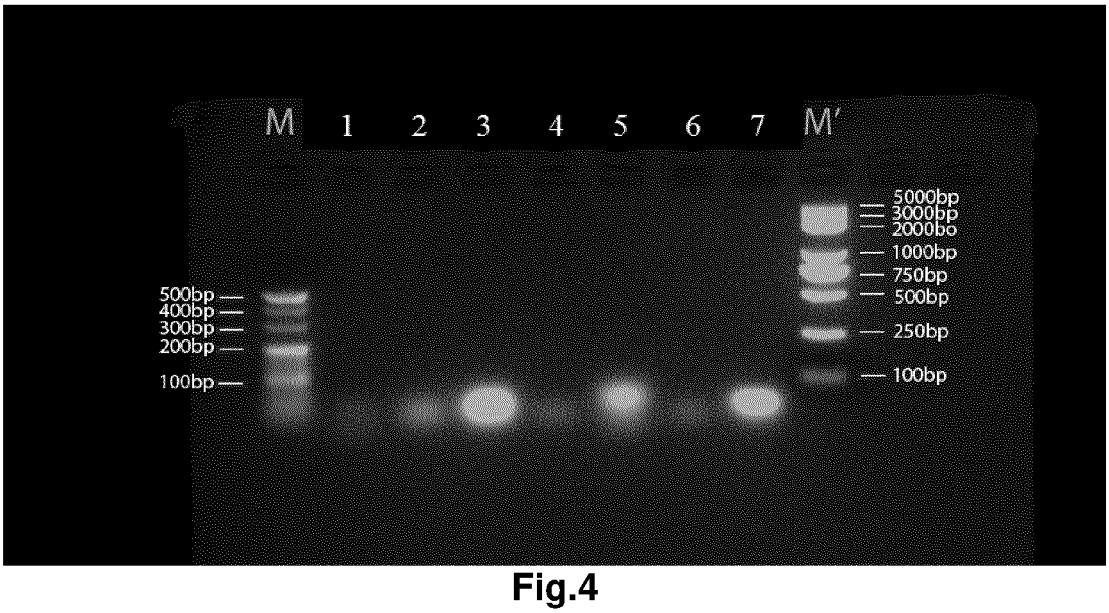
M
1
2
3
4
5
6
7
M'
5000bp
3000bp
2000bo
1000bp
750bp
500bp
250bp
100bp
500bp
400bp
300bp
200bp
100bp

**Fig.4**

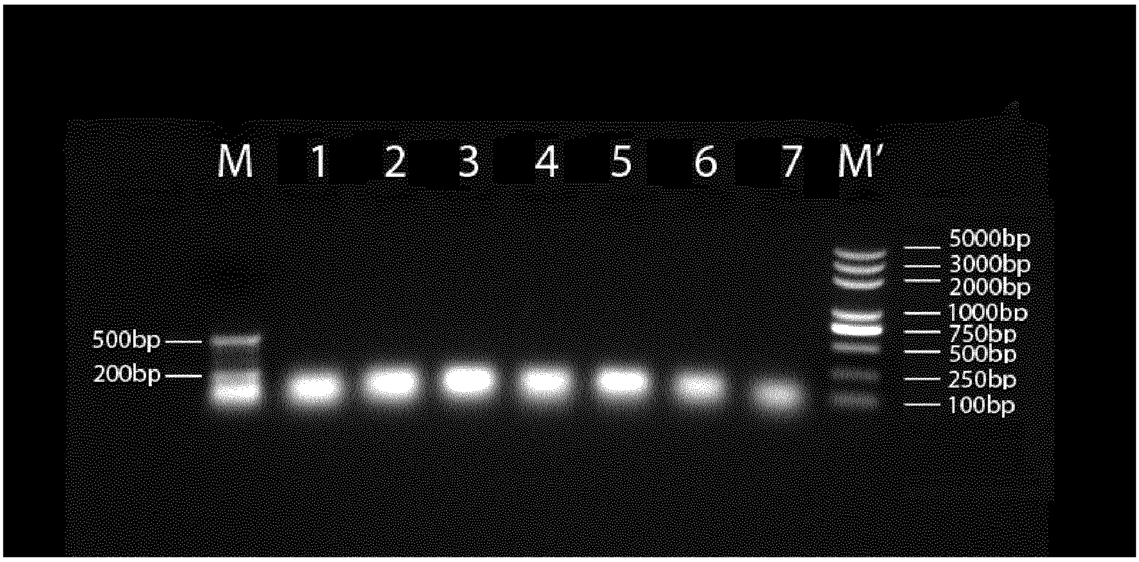
M
1
2
3
4
5
6
7
M′
500bp
200bp
5000bp
3000bp
2000bp
1000bp
750bp
500bp
250bp
100bp

Fig.5

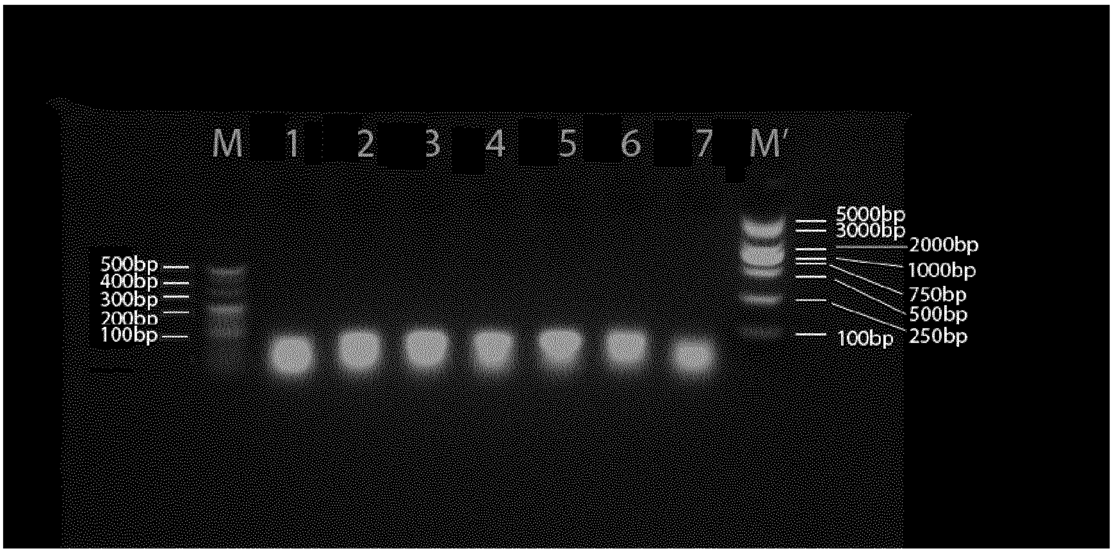
M
1
2
3
4
5
6
7
M′
500bp
400bp
300bp
200bp
100bp
5000bp
3000bp
2000bp
1000bp
750bp
500bp
250bp
100bp

Fig.6

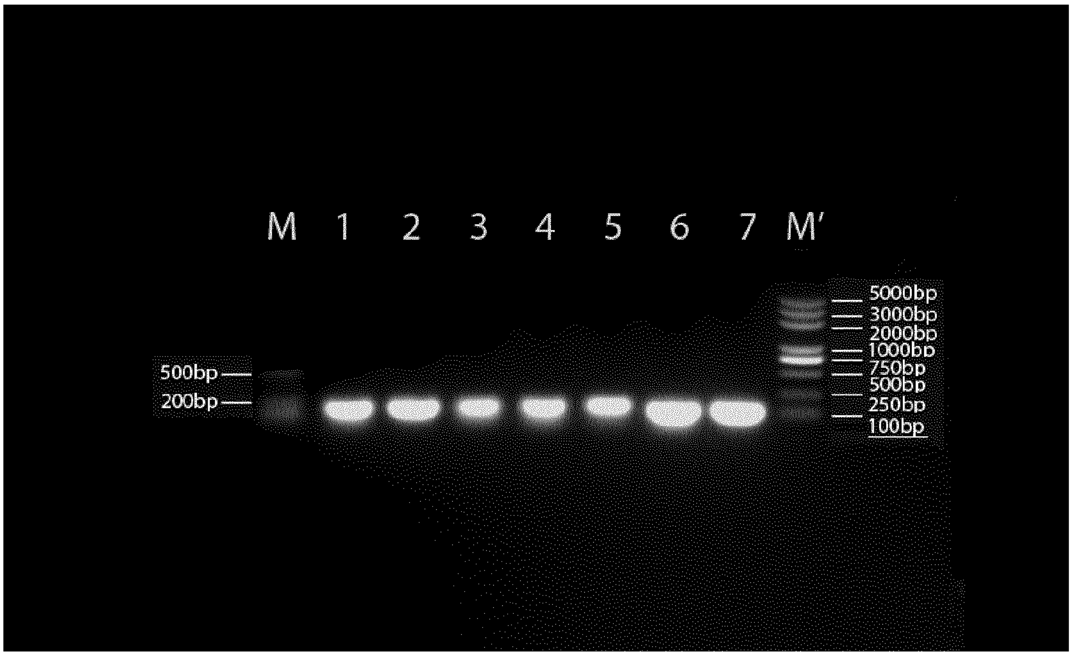
M 1 2 3 4 5 6 7 M'
500bp
200bp
5000bp
3000bp
2000bp
1000bp
750bp
500bp
250bp
100bp

Fig.7

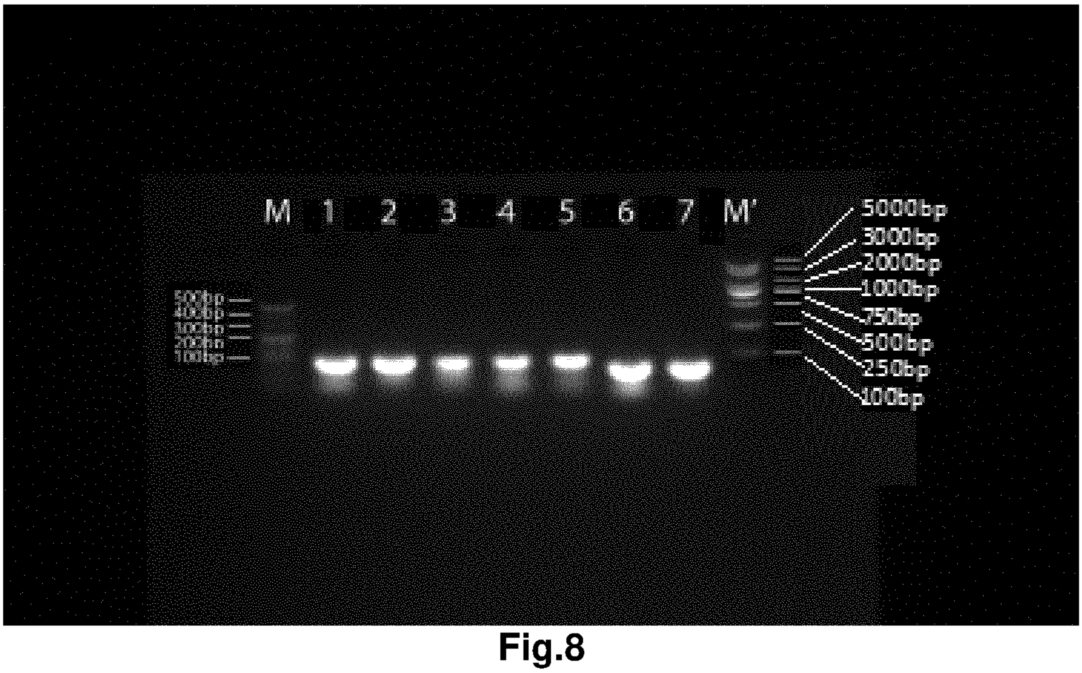
M 1 2 3 4 5 6 7 M'
500bp
400bp
300bp
200bp
100bp
5000bp
3000bp
2000bp
1000bp
750bp
500bp
250bp
100bp

Fig.8

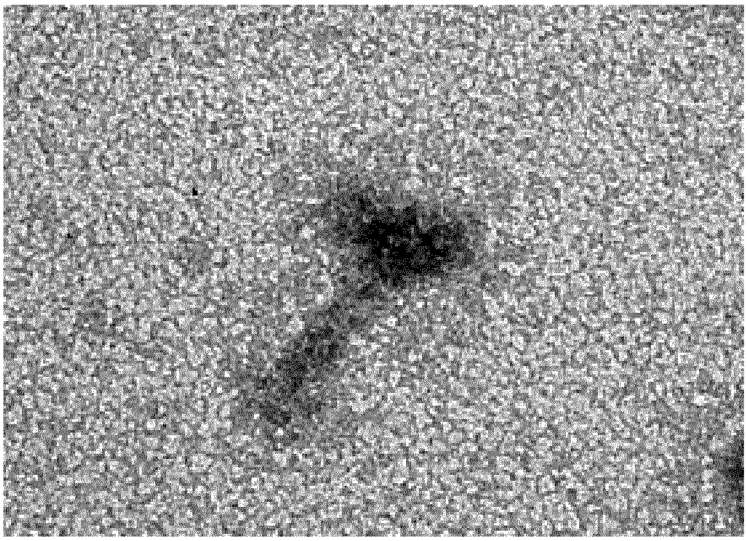

Fig. 9

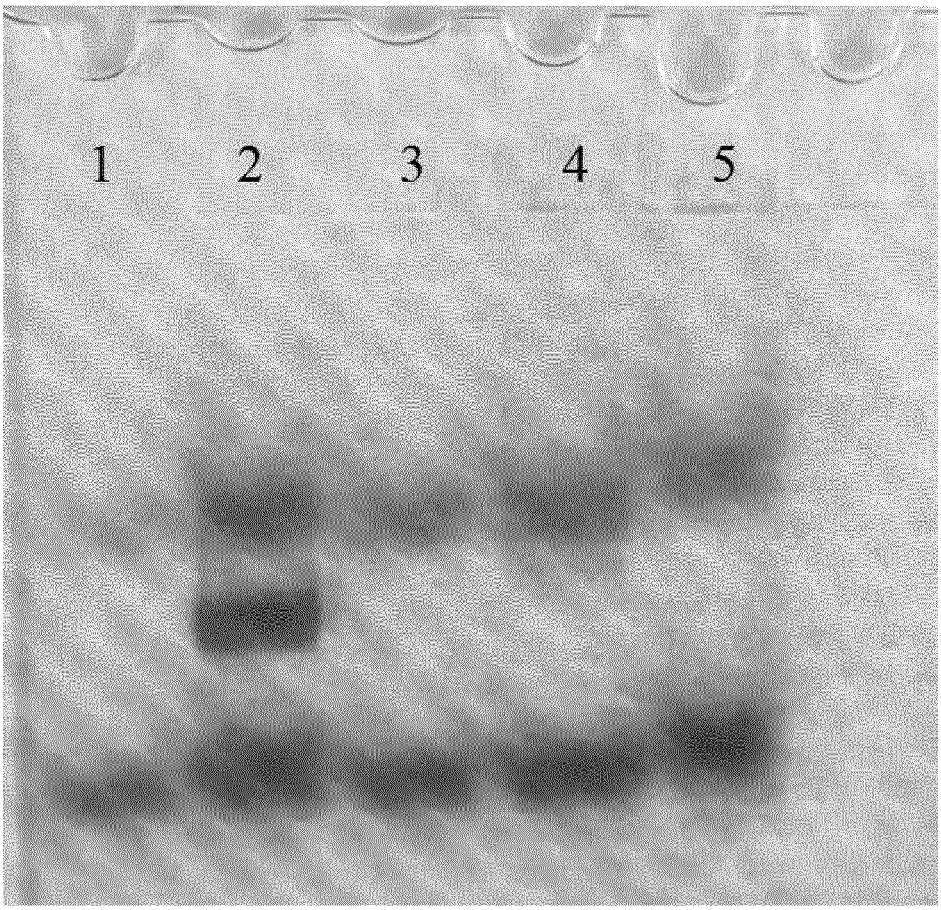


Fig. 10

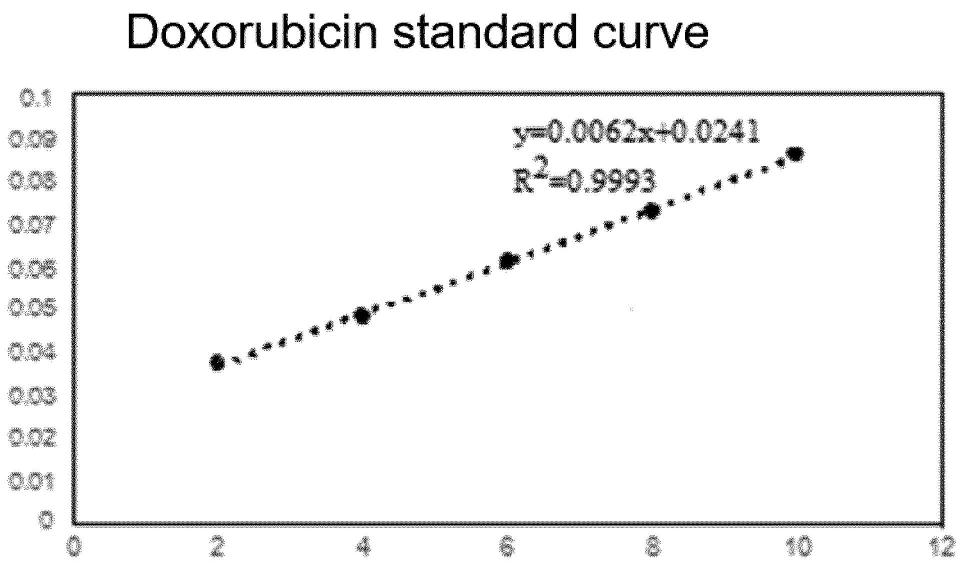
Doxorubicin standard curve
y=0.0062x+0.0241
R2=0.9993
0.1
0.09
0.08
0.07
0.06
0.05
0.04
0.03
0.02
0.01
0
0
2
4
6
8
10
12

Fig. 11

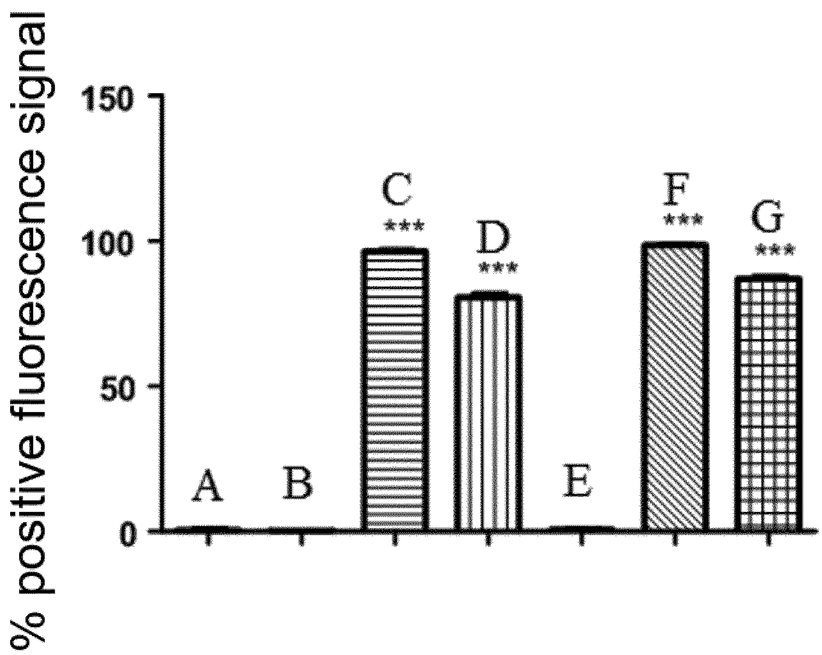
% positive fluorescence signal
150
100
50
0
A
B
C
***
D
***
E
F
***
G
***

Fig. 12

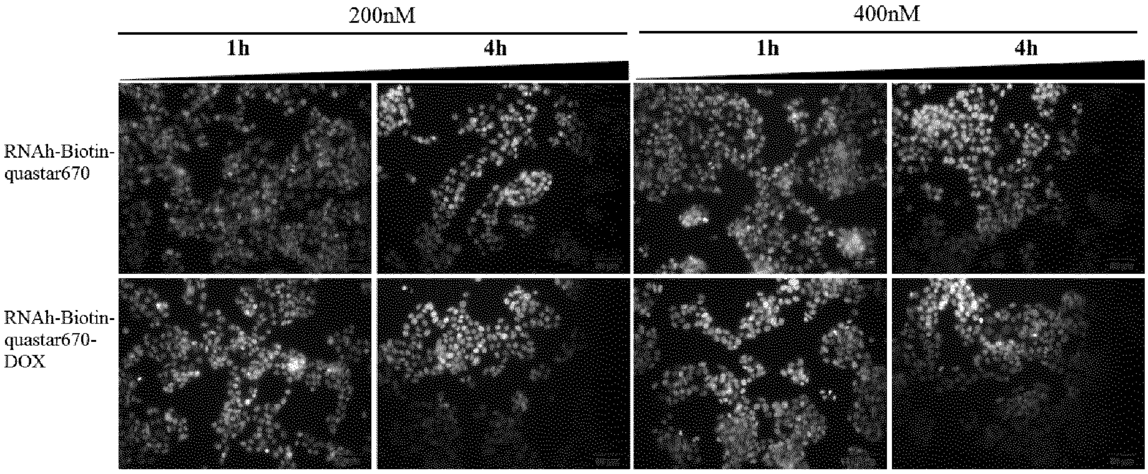


Fig. 13

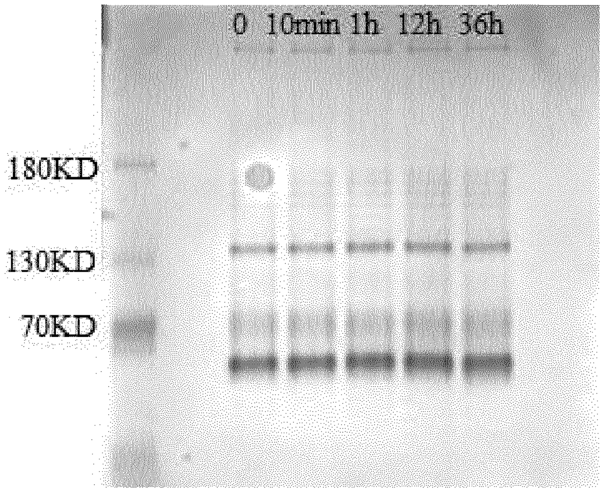


Fig. 14

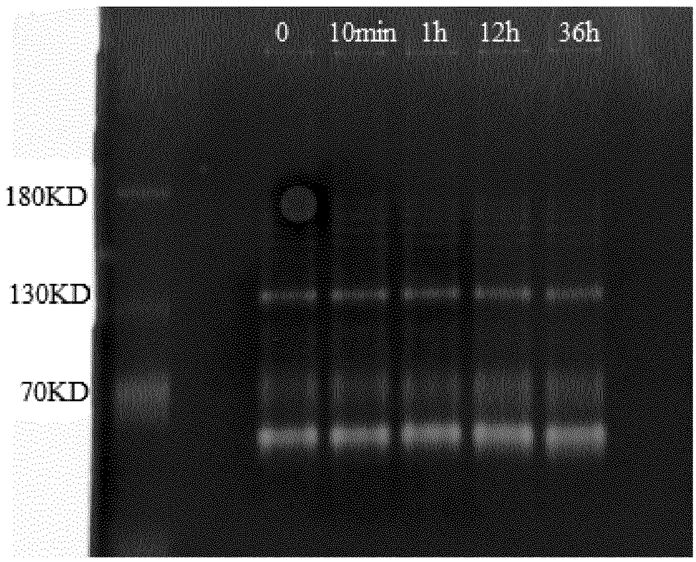


Fig. 15

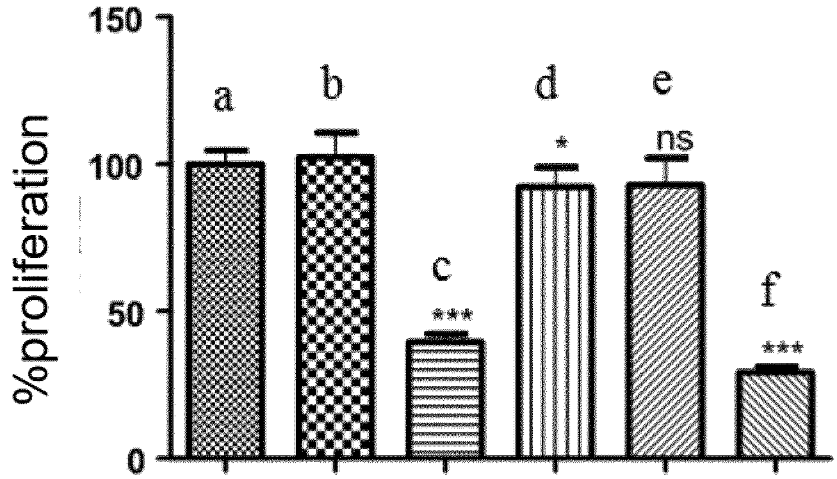
150
100
50
0
%proliferation
a
b
c
***
d
*
e
ns
f
***

Fig. 16

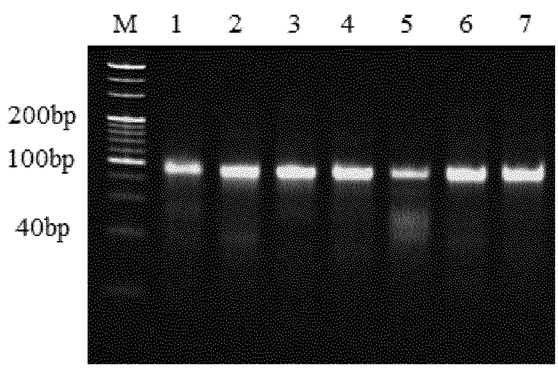
M
1
2
3
4
5
6
7
200bp
100bp
40bp

Fig. 17

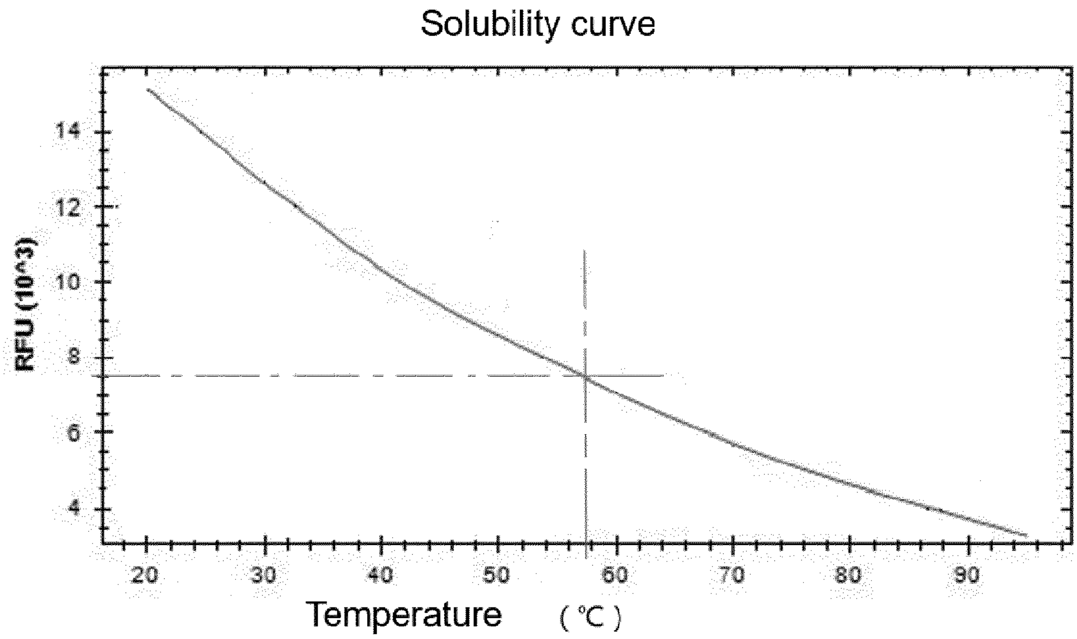
Solubility curve
RFU (10^3)
14
12
10
8
6
4
20
30
40
50
60
70
80
90
Temperature (℃)

Fig. 18

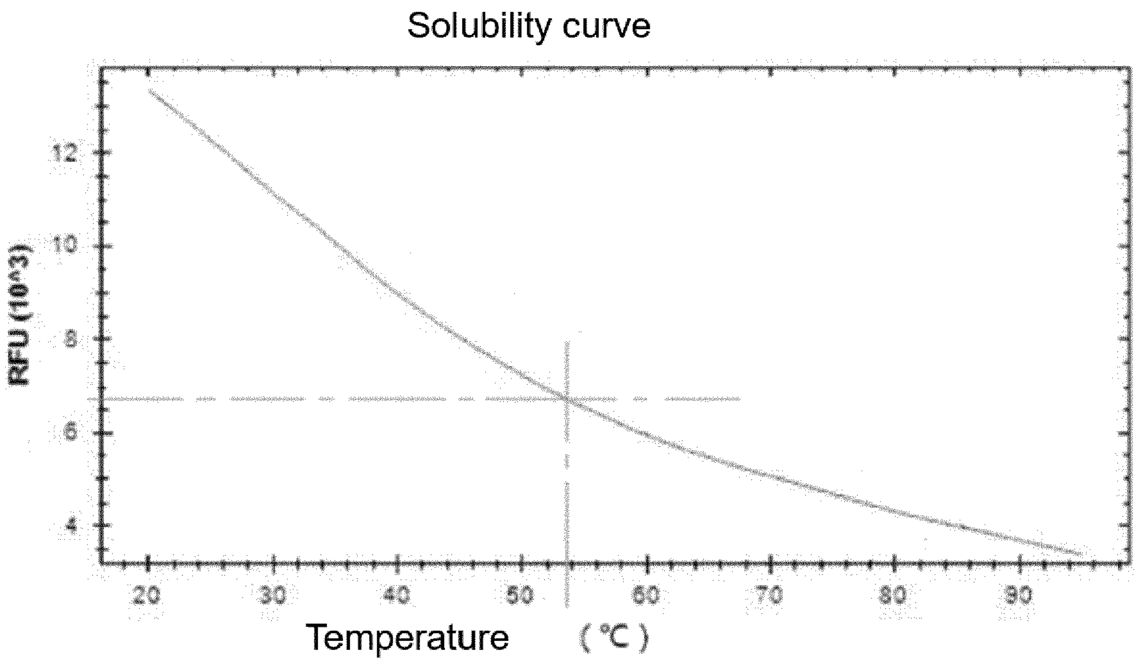
Solubility curve
RFU (10^3)
12
10
8
6
4
20
30
40
50
60
70
80
90
Temperature (℃)

Fig. 19

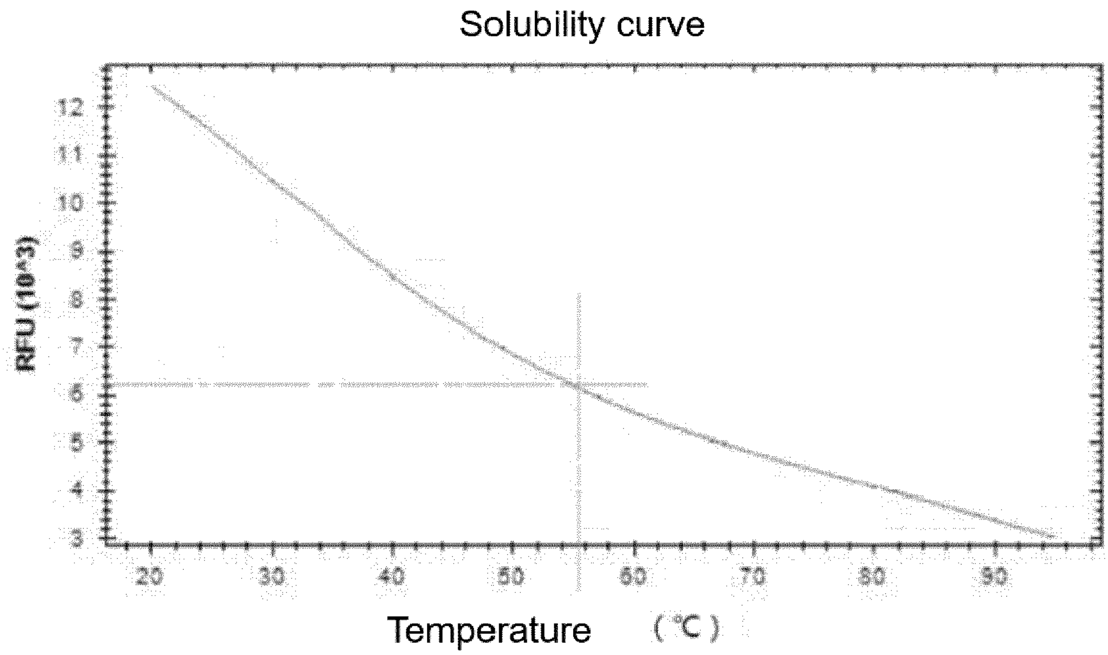
Solubility curve
RFU (10^3)
12
11
10
9
8
7
6
5
4
3
20
30
40
50
60
70
80
90
Temperature (℃)

Fig. 20

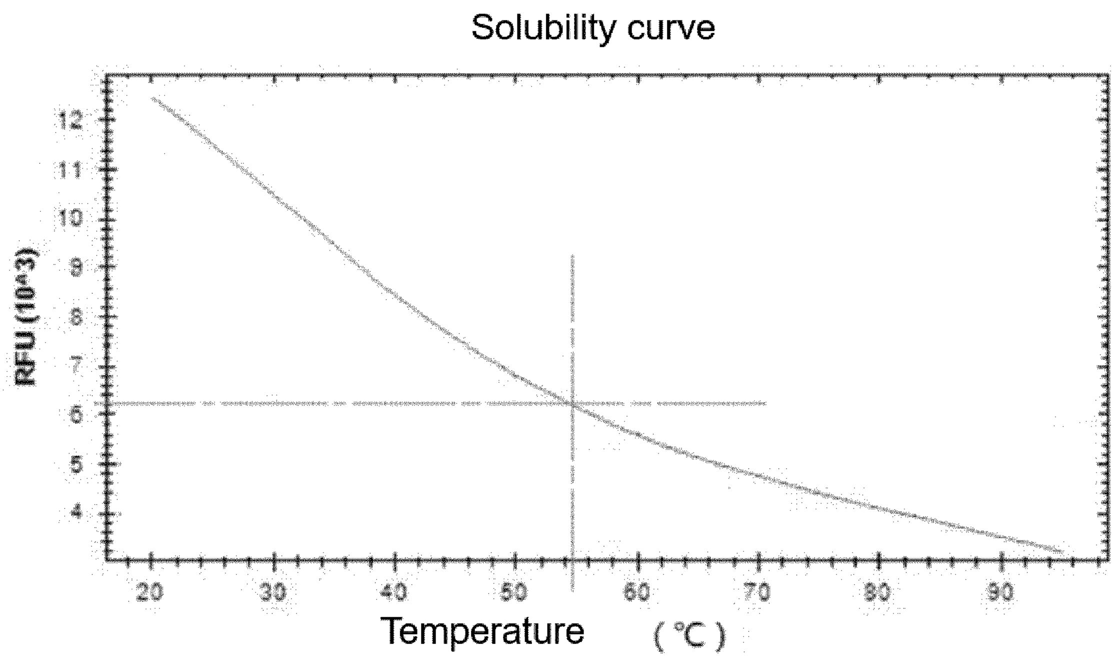
Solubility curve
RFU (10^3)
12
11
10
9
8
7
6
5
4
20
30
40
50
60
70
80
90
Temperature (℃)

Fig. 21

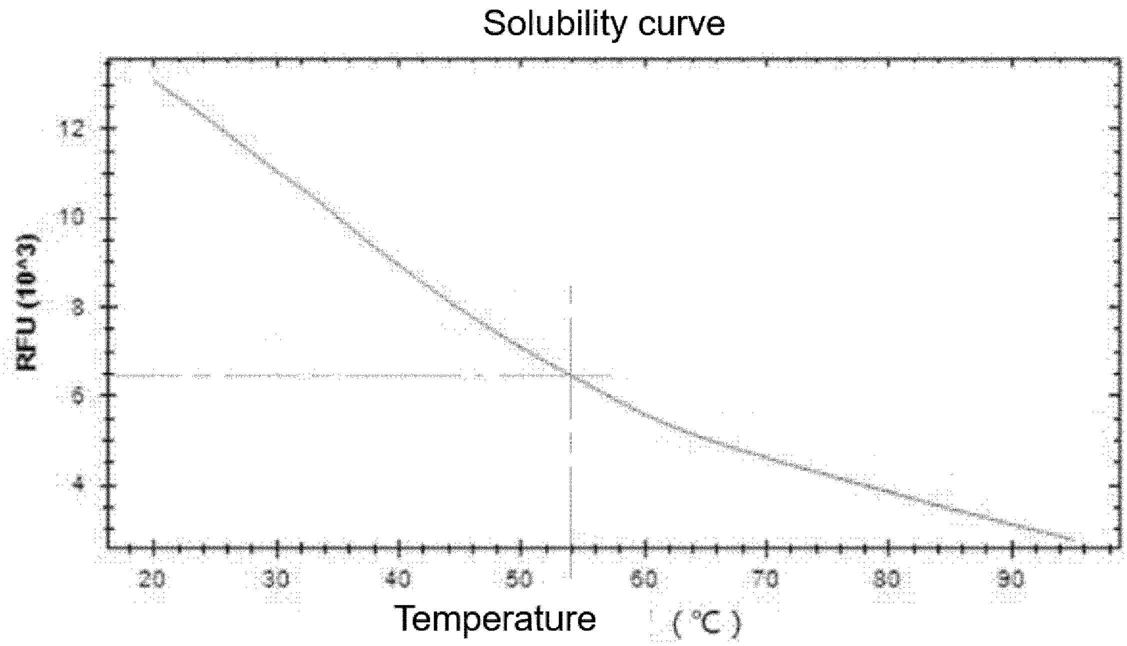
Solubility curve
RFU (10^3)
12
10
8
6
4
20
30
40
50
60
70
80
90
Temperature
( °C )

Fig. 22

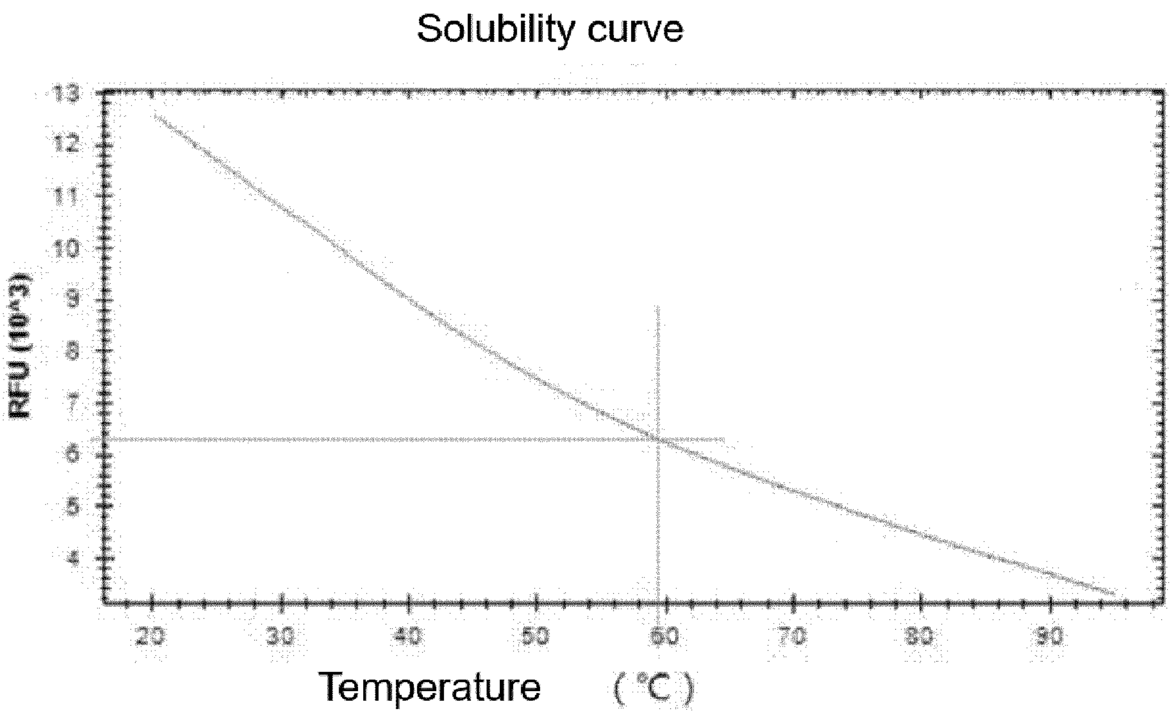
Solubility curve
RFU (10^3)
13
12
11
10
9
8
7
6
5
4
20
30
40
50
60
70
80
90
Temperature
( °C )

Fig. 23

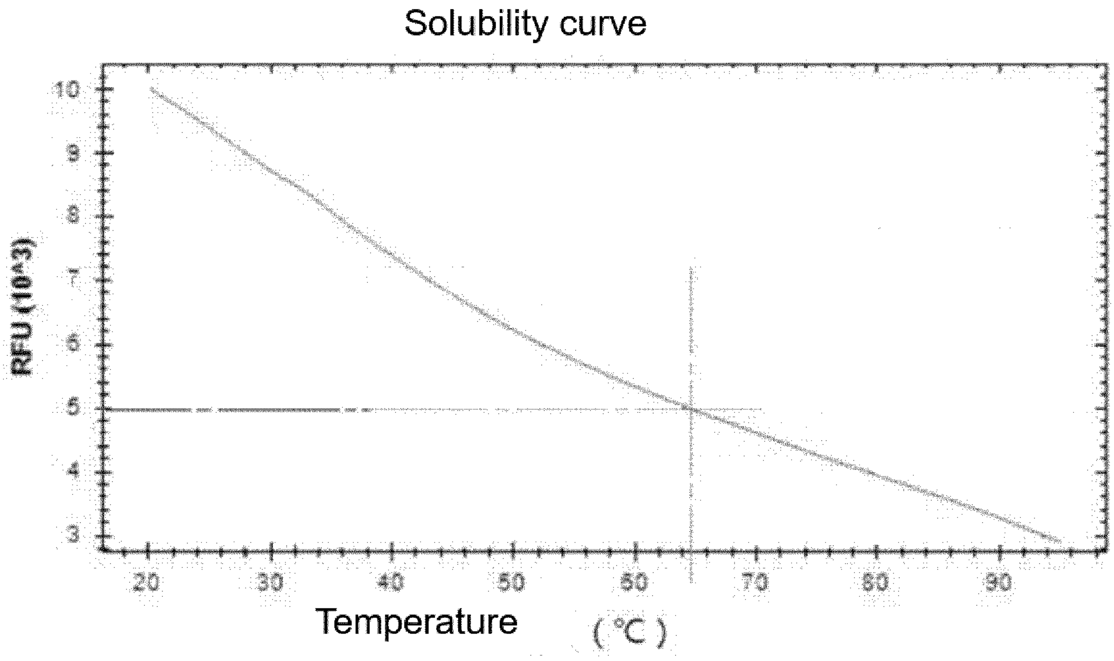
Solubility curve
RFU (10^3)
10
9
8
7
6
5
4
3
20
30
40
50
60
70
80
90
Temperature
( °C )

Fig. 24

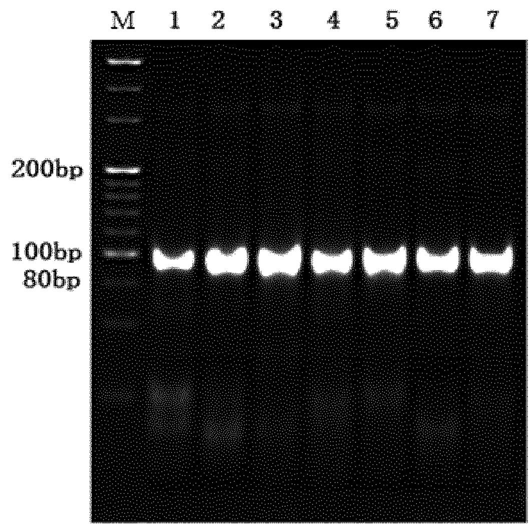
M
1
2
3
4
5
6
7
200bp
100bp
80bp

Fig. 25

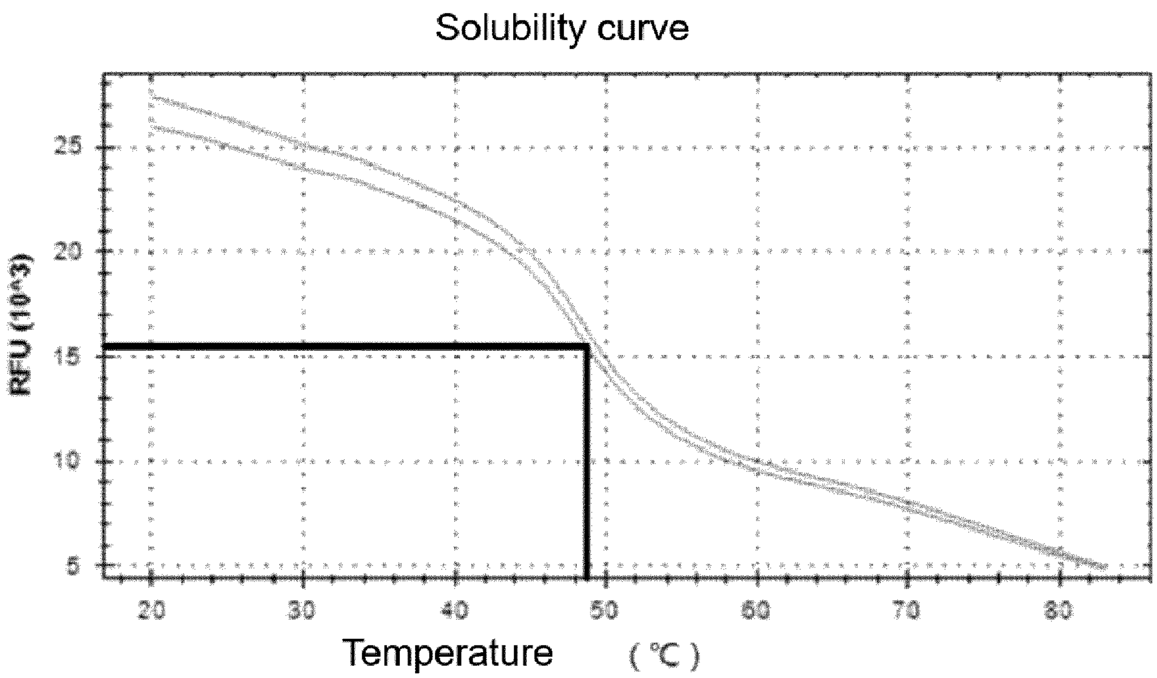
Solubility curve
RFU (10^3)
25
20
15
10
5
20
30
40
50
60
70
80
Temperature (℃)

Fig. 26

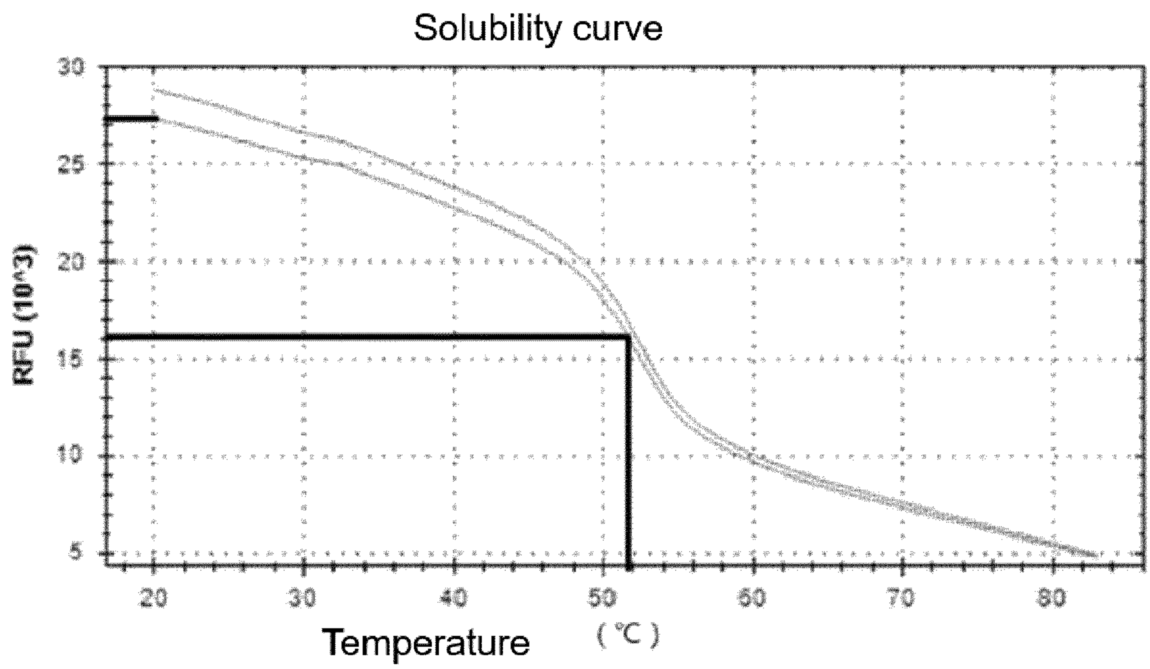
Solubility curve
RFU (10^3)
30
25
20
15
10
5
20
30
40
50
60
70
80
Temperature (℃)

Fig. 27

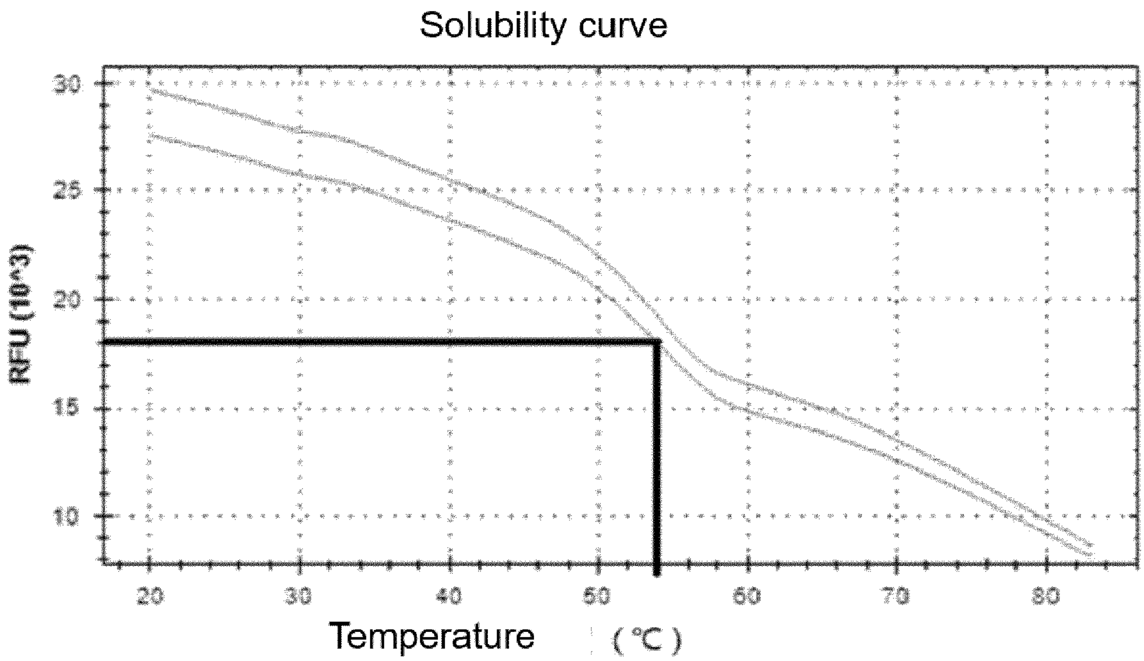
Solubility curve
RFU (10^3)
30
25
20
15
10
20
30
40
50
60
70
80
Temperature (℃)

Fig. 28

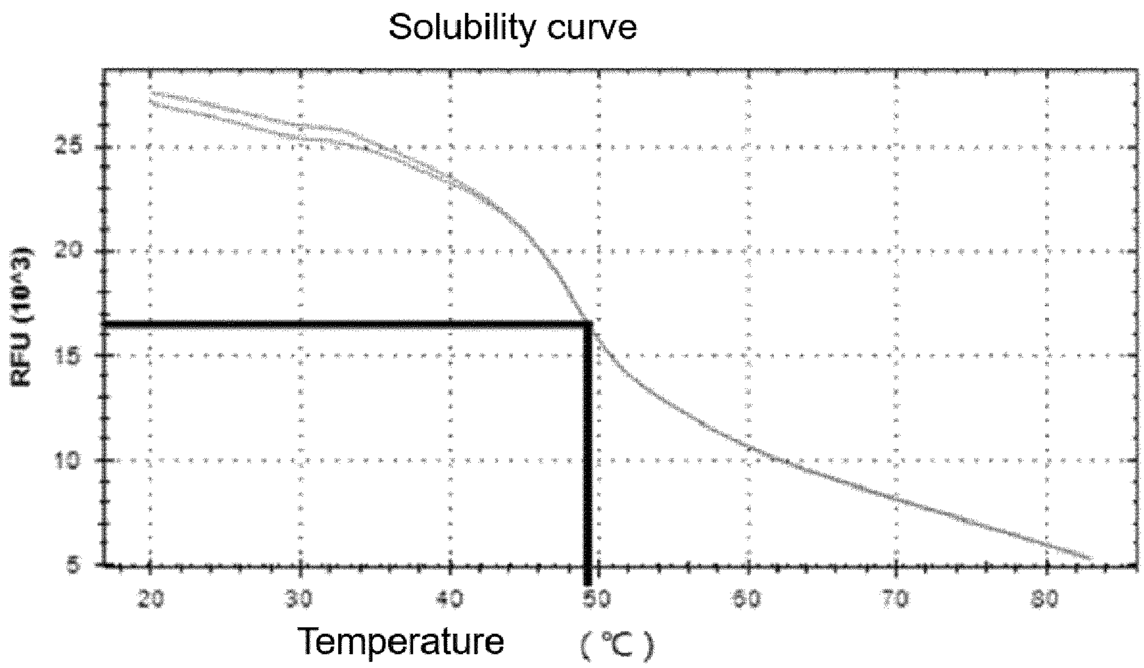
Solubility curve
RFU (10^3)
25
20
15
10
5
20
30
40
50
60
70
80
Temperature (℃)

Fig. 29

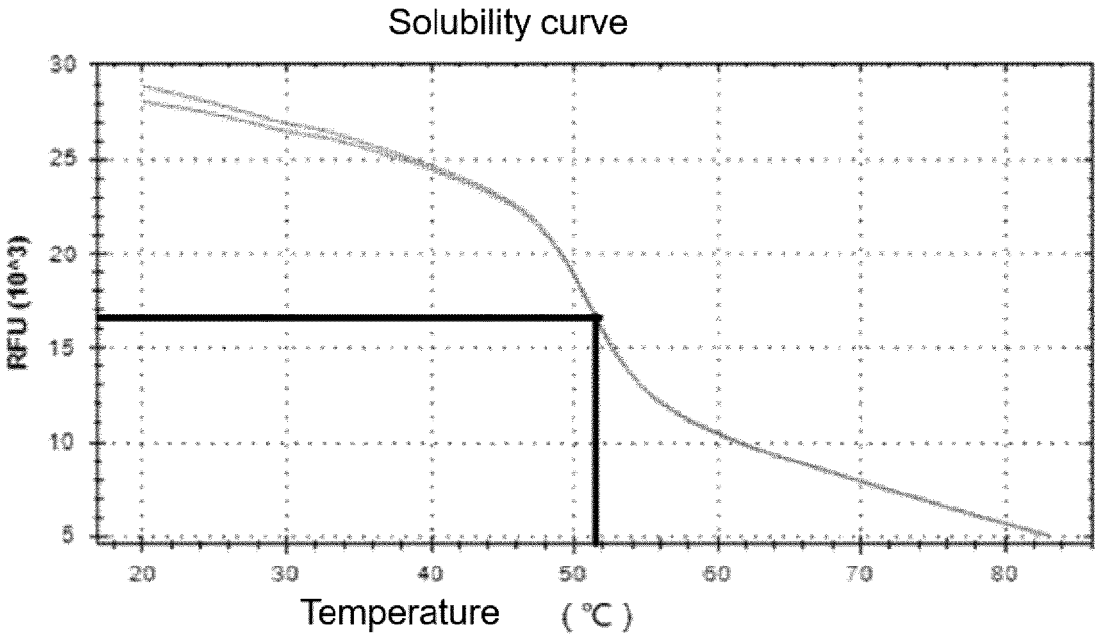
Solubility curve
RFU (10^3)
30
25
20
15
10
5
20
30
40
50
60
70
80
Temperature (℃)

Fig. 30

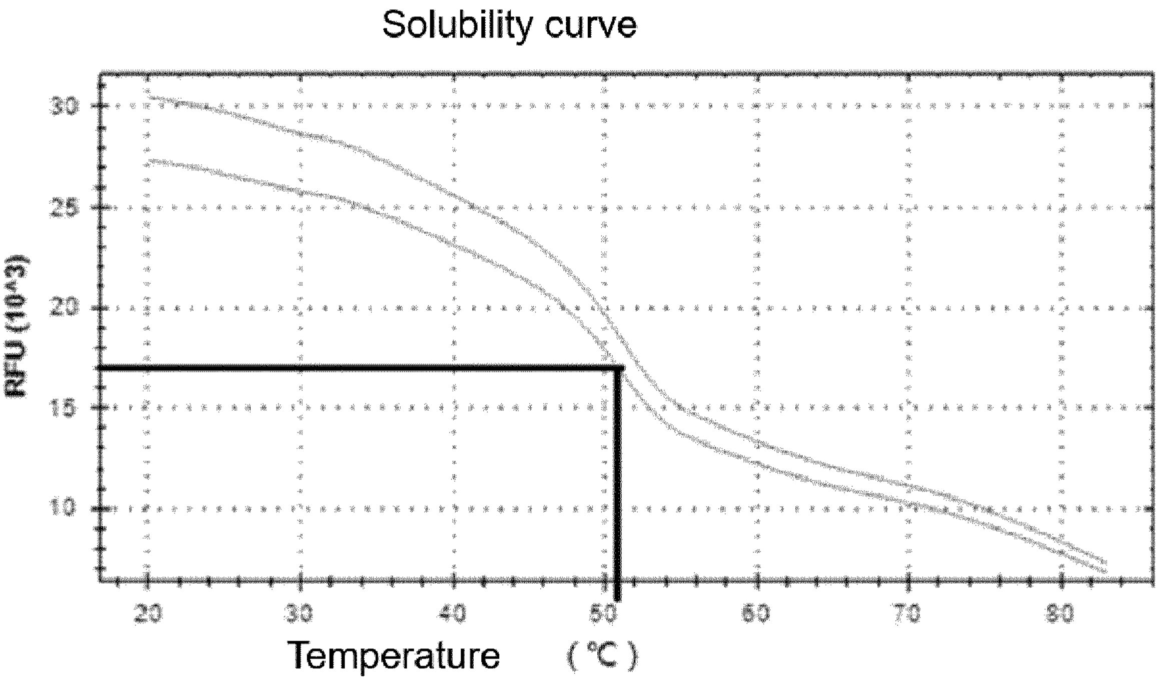
Solubility curve
RFU (10^3)
30
25
20
15
10
20
30
40
50
60
70
80
Temperature (℃)

Fig. 31

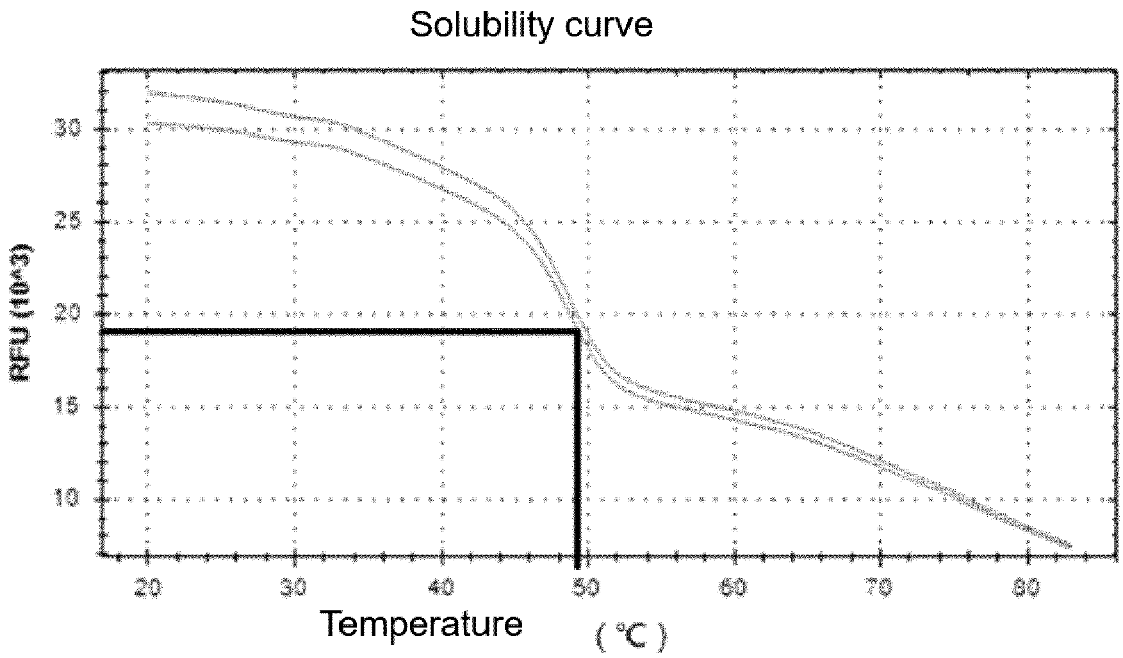
Solubility curve
RFU (10^3)
30
25
20
15
10
20
30
40
50
60
70
80
Temperature
(℃)

Fig. 32

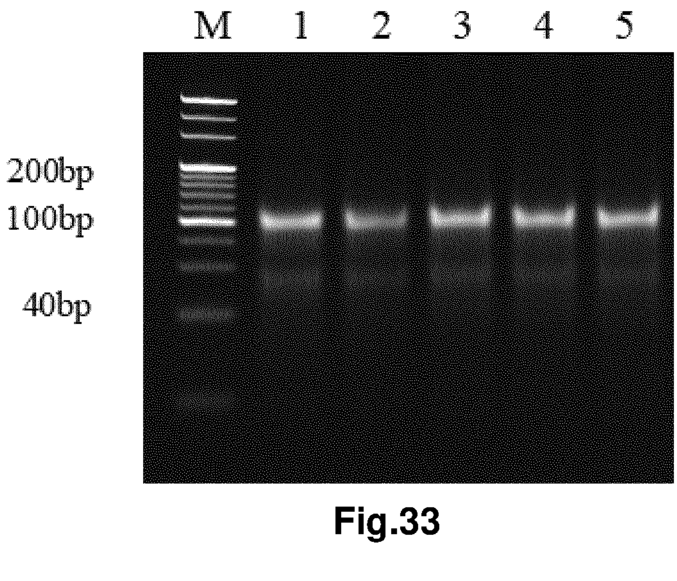
M
1
2
3
4
5
200bp
100bp
40bp

Fig.33

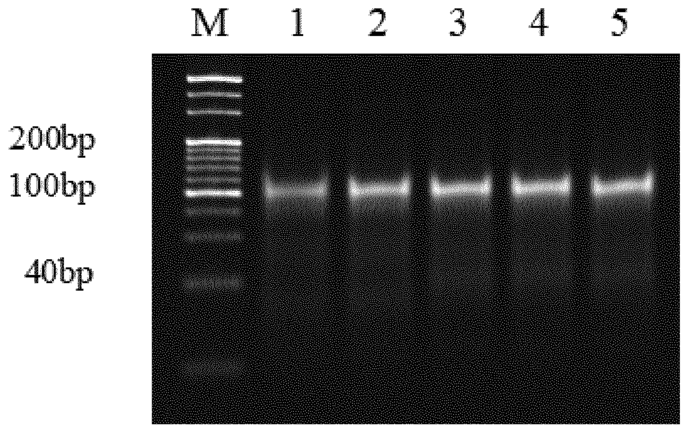


Fig.34

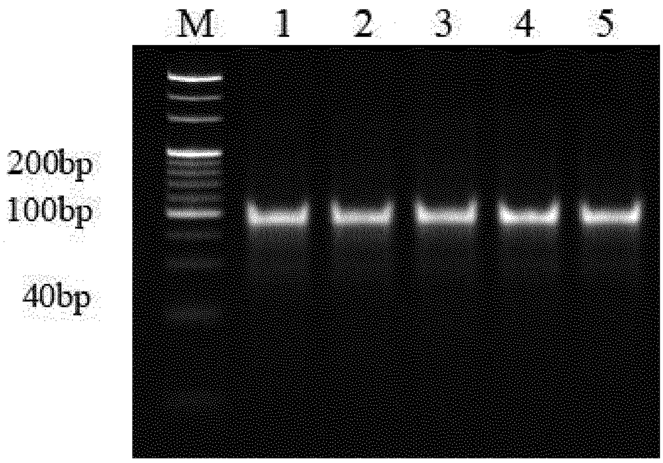


Fig.35

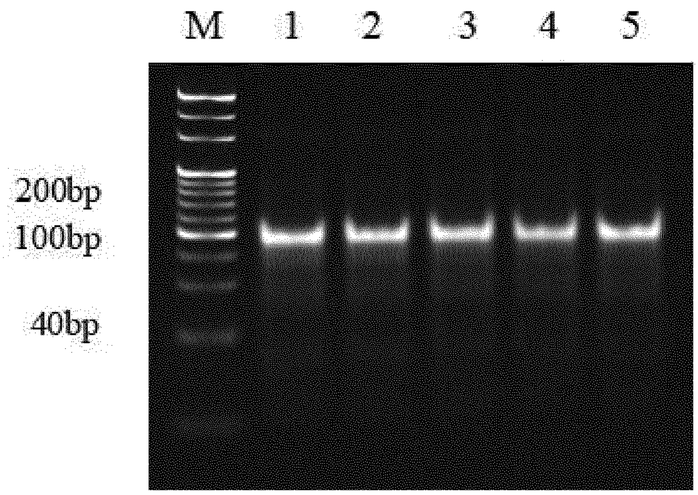


Fig.36

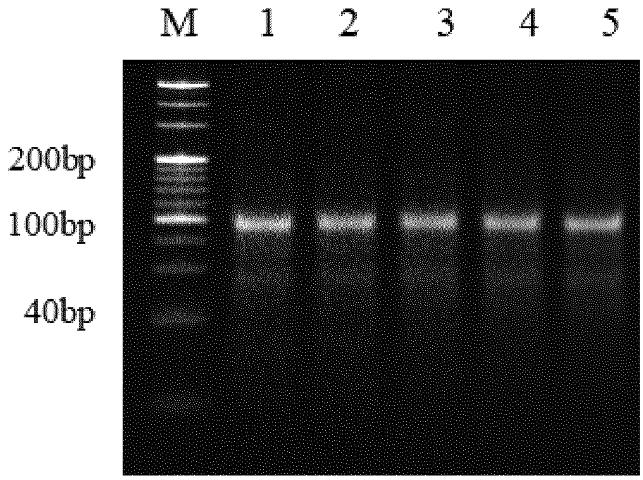


Fig.37

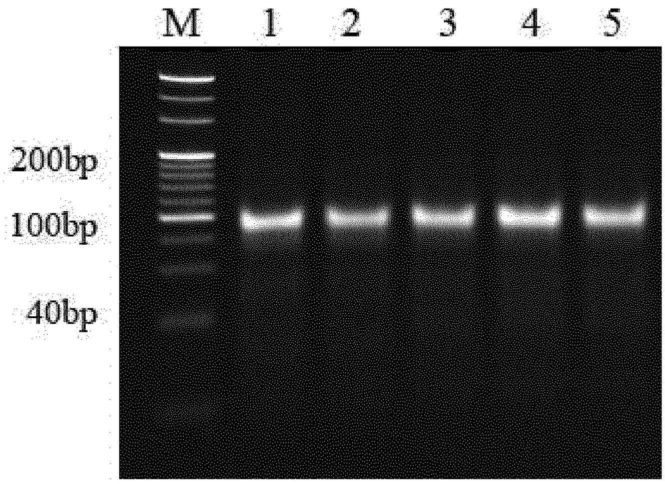


Fig.38

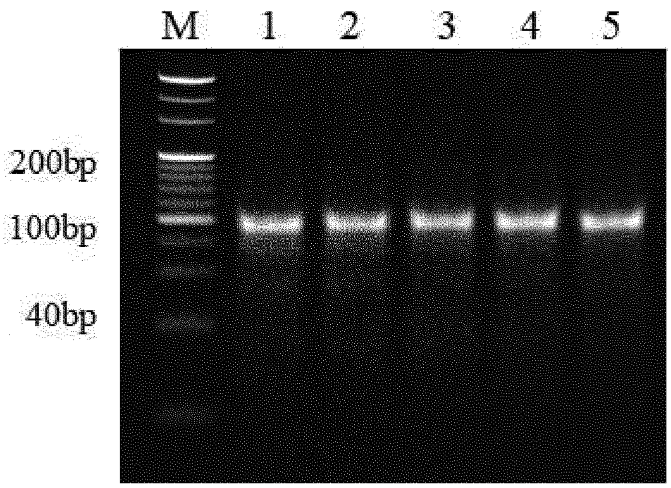


Fig.39

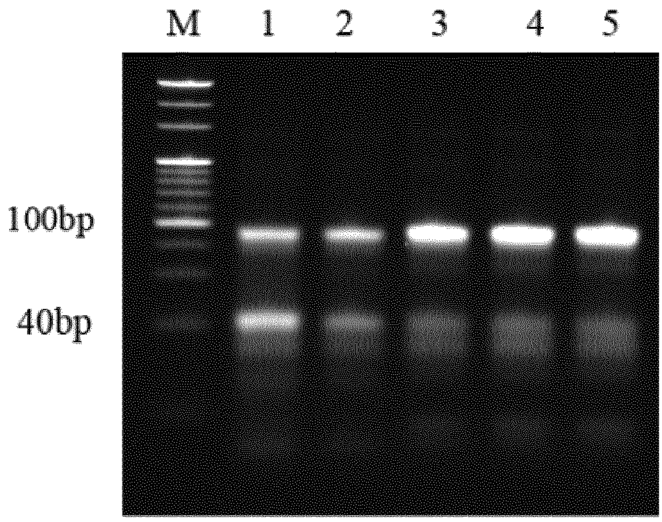


Fig.40

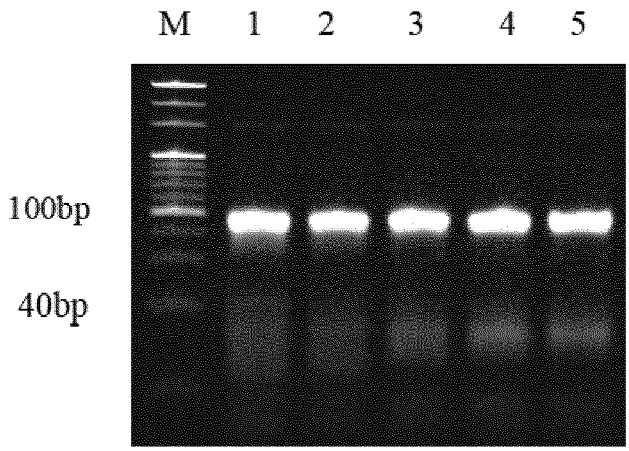


Fig.41

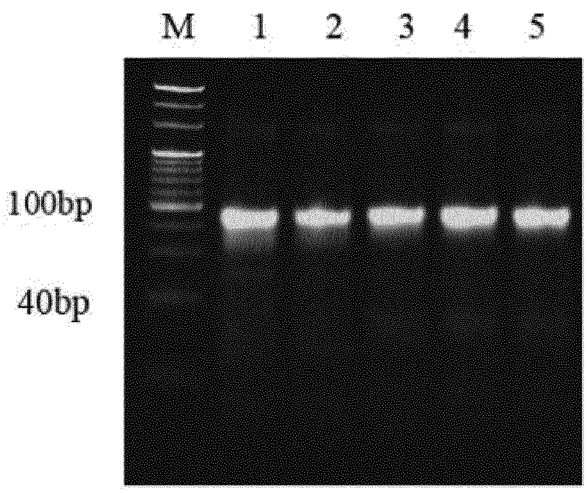


Fig.42

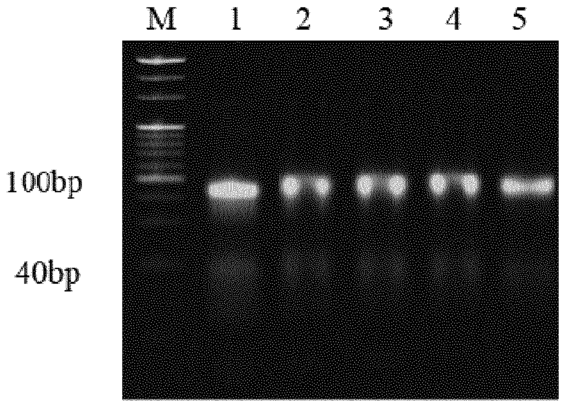


Fig.43

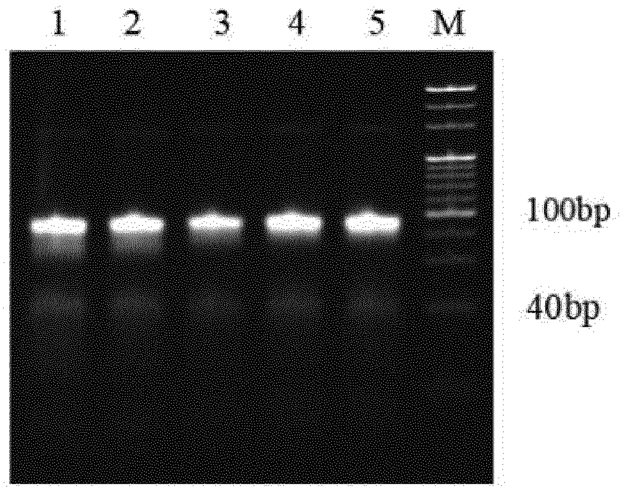


Fig.44

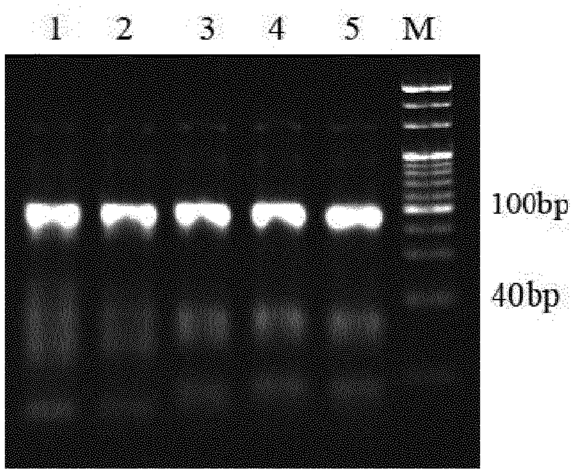


Fig.45

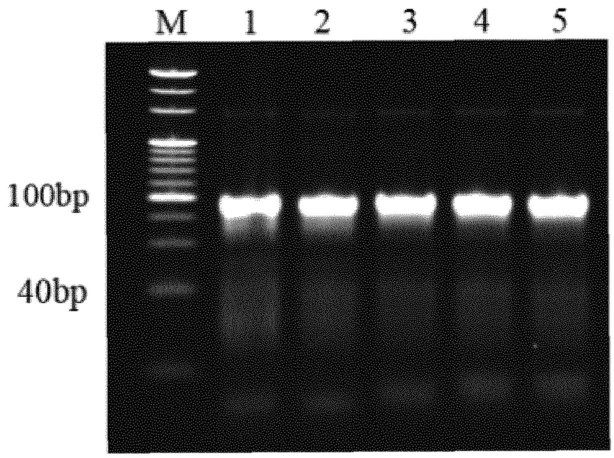


Fig.46

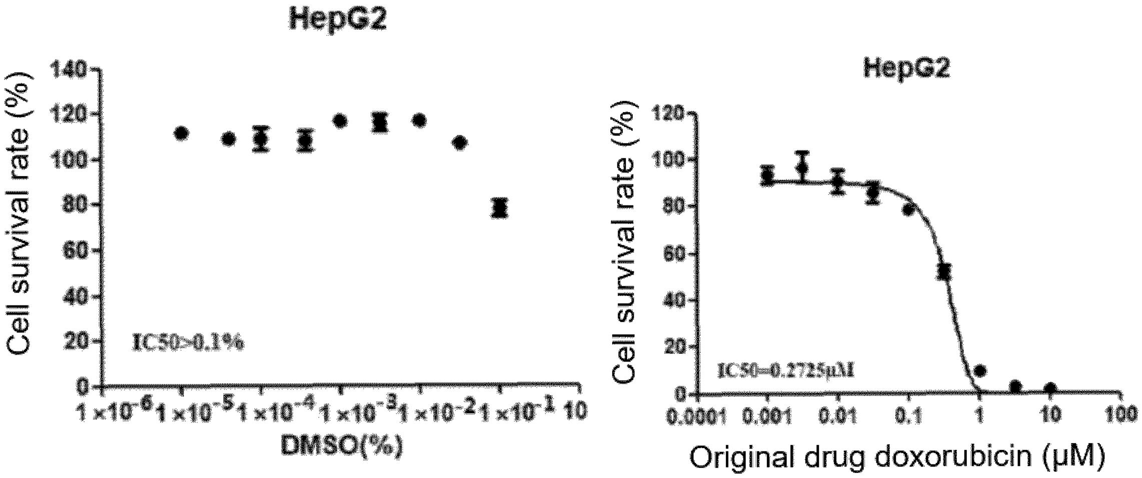


Fig. 47a

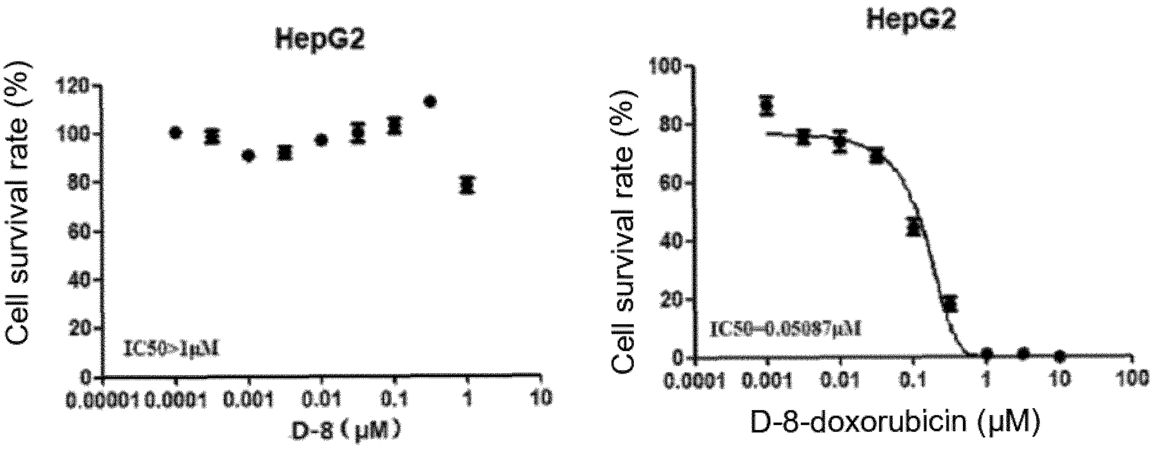


Fig. 47b

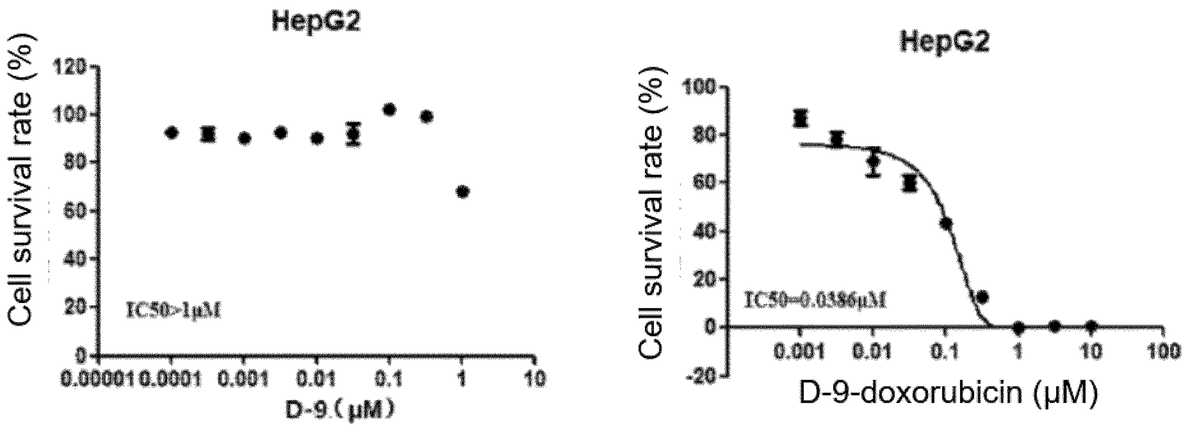


Fig. 47c

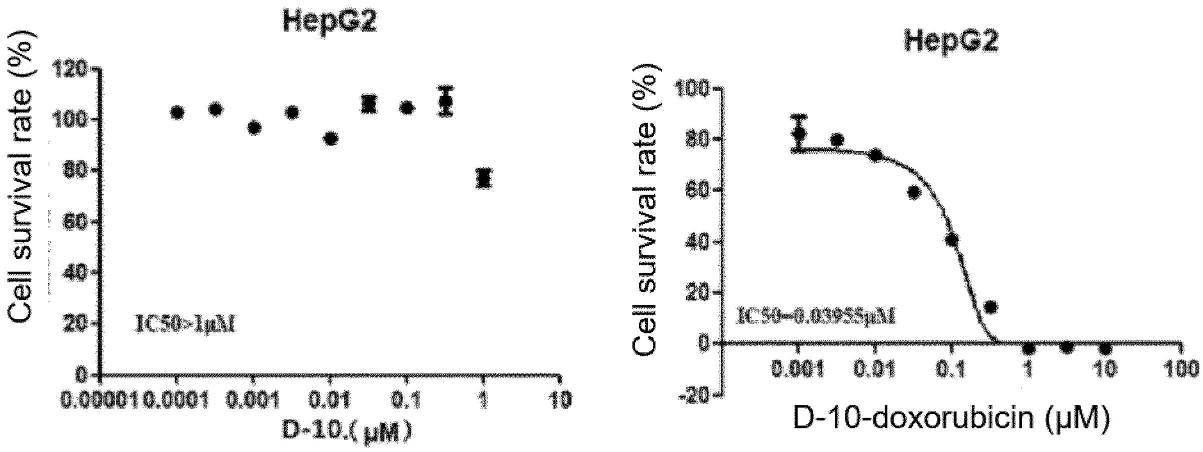


Fig. 47d

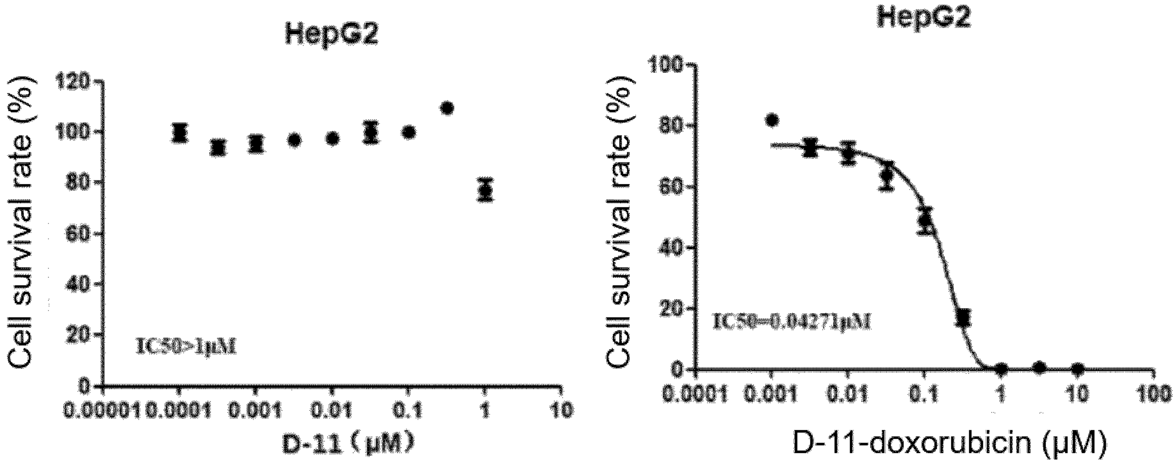


Fig. 47e

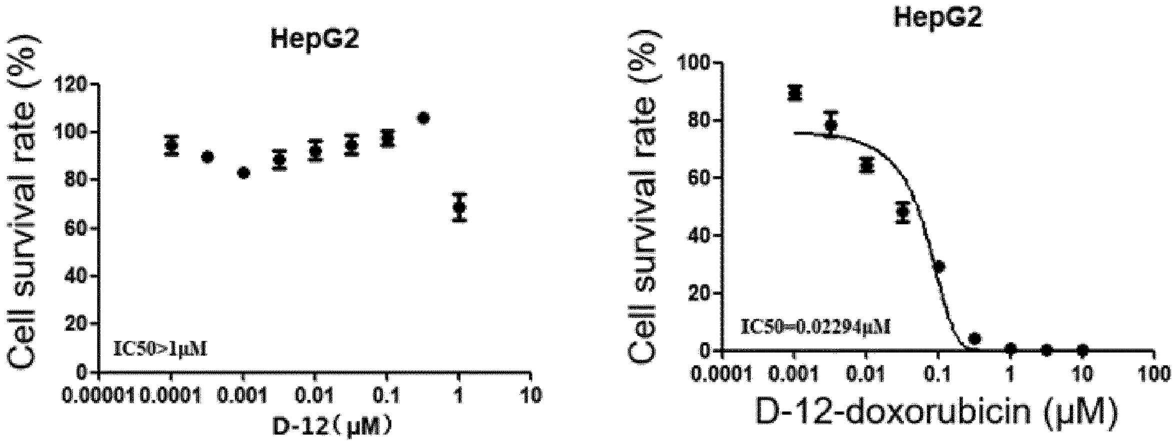


Fig. 47f

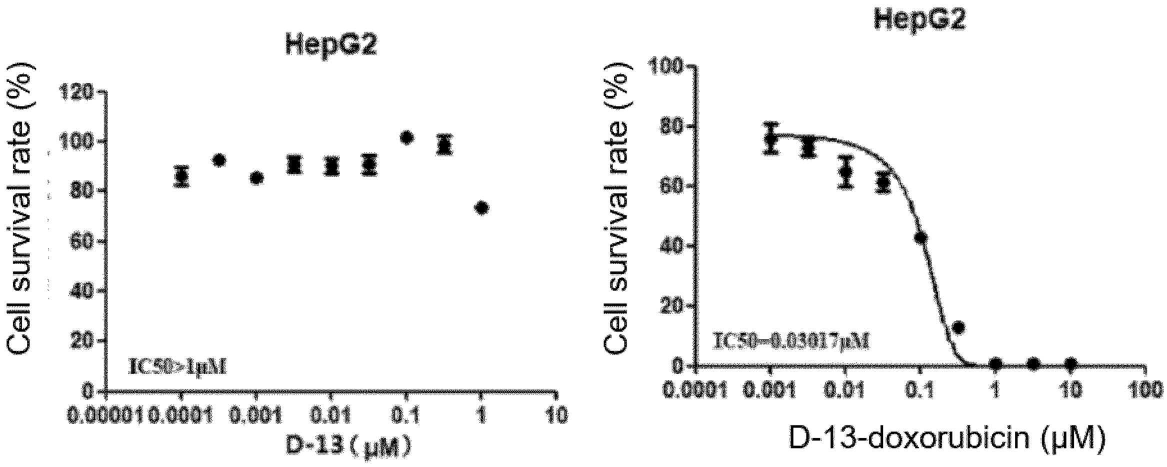


Fig. 47g

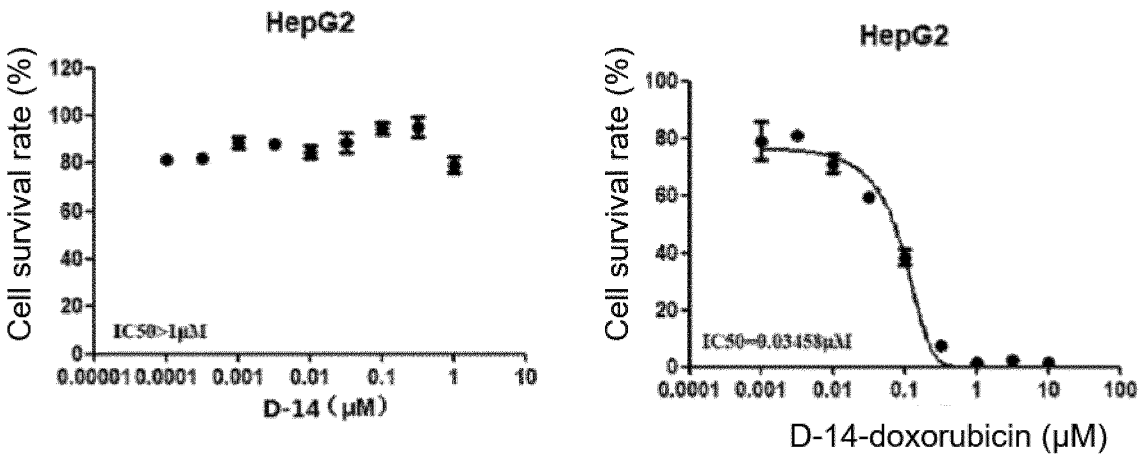


Fig. 47h

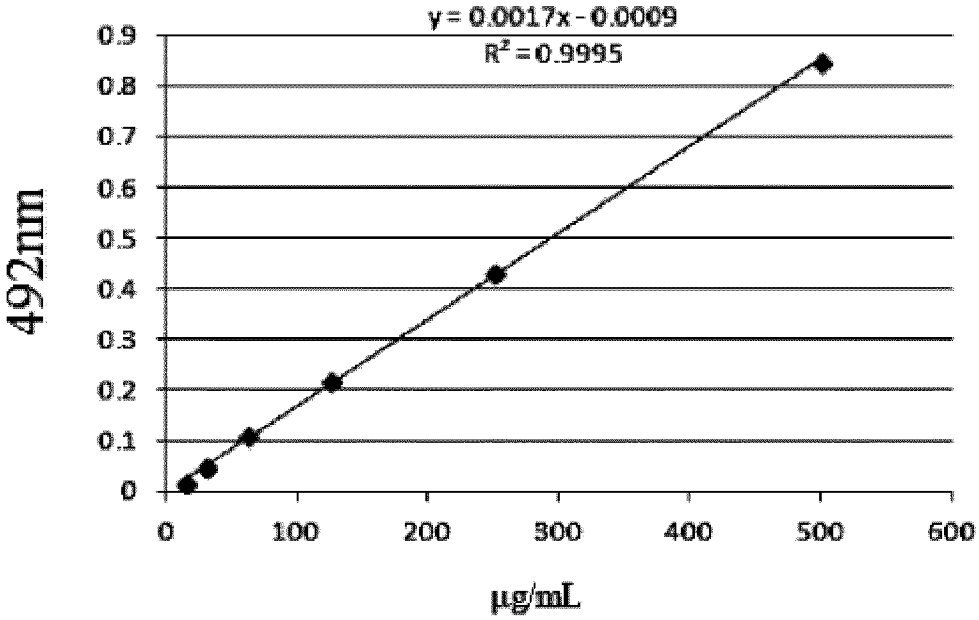
y = 0.0017x - 0.0009
$R^2$ = 0.9995
0.9
0.8
0.7
0.6
0.5
0.4
0.3
0.2
0.1
0
492nm
0
100
200
300
400
500
600
µg/mL

Fig. 48

**INTERNATIONAL SEARCH REPORT**

International application No.
**PCT/CN2019/095766**

**A. CLASSIFICATION OF SUBJECT MATTER**

A61K 47/54(2017.01)i； C12N 15/10(2006.01)i； C12N 15/11(2006.01)i； A61K 31/713(2006.01)i

According to International Patent Classification (IPC) or to both national classification and IPC

**B. FIELDS SEARCHED**

Minimum documentation searched (classification system followed by classification symbols)

A61K, C12N

Documentation searched other than minimum documentation to the extent that such documents are included in the fields searched

Electronic data base consulted during the international search (name of data base and, where practicable, search terms used)

CNABS, SIPOABS, DWPI, CNKI, 万方, WANFANG, PubMed, ISI web of knowledge, googlescholar: DNA, RNA, 核酸, 纳米, 颗粒, 载体, 自组装, Y型, T型, 三叉型, 配对, 单链, nano, self assembly, nucleotide, vehicle, shape, structure, match

**C. DOCUMENTS CONSIDERED TO BE RELEVANT**

| Category* | Citation of document, with indication, where appropriate, of the relevant passages | Relevant to claim No. |
|---|---|---|
| X | MOHRI, K. et al. "Design and development of nanosized DNA assemblies in polypod-like structures as efficient vehicles for immunostimulatory CpG motifs to immune cells" *ACS Nano,* Vol. 6, No. 7, 21 June 2012 (2012-06-21), pp. 5931-5940 | 1-37 |
| A | 俞洋 等 (YU, Yang et al.). "基于DNA 自组装过程的纳米结构研究 (Advances on Self-Assembled DNA Nanostructures)" *生物技术通报 (Biotechnology Bulletin),* Vol. 31, No. 4, 31 December 2015 (2015-12-31), pp. 120-133 | 1-37 |
| A | NISHIKAWA, M. et al. "Biodegradable CpG DNA hydrogels for sustained delivery of doxorubicin and immunostimulatory signals in tumor-bearing mice" *Biomaterials,* Vol. 32, 06 October 2010 (2010-10-06), pp. 488-494 | 1-37 |
| A | HAMADA, S. et al. "Substrate-Assisted Assembly of Interconnected Single-Duplex DNA" *Angew. Chem. Int. Ed.,* Vol. 48, 17 August 2009 (2009-08-17), pp. 6820-6823 | 1-37 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 September 2019** | **16 October 2019** |

| Name and mailing address of the ISA/CN | Authorized officer |
|---|---|
| **China National Intellectual Property Administration (ISA/CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2019/095766**

**C. DOCUMENTS CONSIDERED TO BE RELEVANT**

| Category* | Citation of document, with indication, where appropriate, of the relevant passages | Relevant to claim No. |
|---|---|---|
| A | 葛志磊 等 (GE, Zhilei et al.). "DNA 纳米自组装的研究进展及应用 (Research progress and applications of self-assembled DNA nanostructures)" *科学通报 (Science Bulletin),* Vol. 59, No. 2, 22 August 2013 (2013-08-22), pp. 146-157 | 1-37 |
| A | CN 1390253 A (MERCK PATENT GMBH) 08 January 2003 (2003-01-08) entire document | 1-37 |
| A | WO 9312244 A1 (UNIV. NEW YORK) 24 June 1993 (1993-06-24) entire document | 1-37 |
| A | US 5386020 A (UNIV. NEW YORK) 31 January 1995 (1995-01-31) entire document | 1-37 |

Form PCT/ISA/210 (second sheet) (January 2015)

**INTERNATIONAL SEARCH REPORT**
**Information on patent family members**

International application No.
**PCT/CN2019/095766**

| Patent document cited in search report | | | Publication date (day/month/year) | Patent family member(s) | | | Publication date (day/month/year) |
|---|---|---|---|---|---|---|---|
| CN | 1390253 | A | 08 January 2003 | KR | 20020059727 | A | 13 July 2002 |
| | | | | NO | 20022231 | L | 10 May 2002 |
| | | | | NO | 20022231 | A | 10 May 2002 |
| | | | | PL | 358811 | A1 | 23 August 2004 |
| | | | | HU | 0203914 | A2 | 28 March 2003 |
| | | | | MX | PA02004727 | A | 30 August 2002 |
| | | | | CZ | 20021472 | A3 | 17 July 2002 |
| | | | | CA | 2391084 | A1 | 25 May 2001 |
| | | | | AU | 782880 | B2 | 08 September 2005 |
| | | | | EP | 1228201 | A1 | 07 August 2002 |
| | | | | SK | 6042002 | A3 | 03 December 2002 |
| | | | | BR | 0015484 | A | 02 July 2002 |
| | | | | RU | 2002113755 | A | 10 January 2004 |
| | | | | WO | 0136624 | A1 | 25 May 2001 |
| | | | | JP | 2003522524 | A | 29 July 2003 |
| | | | | NO | 20022231 | D0 | 10 May 2002 |
| | | | | AU | 2000101 | A | 30 May 2001 |
| WO | 9312244 | A1 | 24 June 1993 | AU | 3236593 | A | 19 July 1993 |
| | | | | US | 5278051 | A | 11 January 1994 |
| | | | | US | 5468851 | A | 21 November 1995 |
| US | 5386020 | A | 31 January 1995 | AU | 1236292 | A | 17 August 1992 |
| | | | | WO | 9212164 | A1 | 23 July 1992 |

Form PCT/ISA/210 (patent family annex) (January 2015)